# EUROPEAN PATENT SPECIFICATION

(11) **EP 1 253 160 B1**
(45) Date of publication and mention of the grant of the patent: **23.02.2011**
(21) Application number: 02009695.4
(22) Date of filing: 29.04.2002
(51) Int. Cl.: C08G 63/06, C09D 167/04, C09C 3/10, C09B 67/00, C12P 7/62, G03G 9/087, G03G 7/00, B41M 5/00, G03G 9/093

(54) **Electrophotographic encapsulated toner particles and method for making them**
Elektrophotographische Kapseltonerteilchen und Herstellungsverfahren
Particules encapsulées de toner électrophotographiques et procédé de fabrication

(30) Priority: 27.04.2001 JP 2001131694; 10.07.2001 JP 2001208704
(43) Date of publication of application: 30.10.2002
(73) Proprietor: CANON KABUSHIKI KAISHA, Ohta-ku Tokyo 146-8501 (JP)
(72) Inventor: Honma, Tsutomu, Ohta-ku, Tokyo (JP); Yano, Tetsuya, Ohta-ku, Tokyo (JP); Nomoto, Tsuyoshi, Ohta-ku, Tokyo (JP); Kozaki, Shinya, Ohta-ku, Tokyo (JP)
(74) Representative: Weser, Wolfgang

(56) References cited:
- US-A- 5 004 664
- US-A- 6 025 028
- US-A- 6 146 665

## Description

### BACKGROUND OF THE INVENTION

### Field of the Invention

The present invention relates to an electrophotographic encapsulated toner particle comprising a particle as base material coated with a shell of polyhydroxyalkanoate, and to a method for making an encapsulated particle. More specifically, the shell of said toner particle comprises a polyhydroxyalkanoate made with a 3-hydroxyalkanoic acid monomer unit other than 3-hydroxypropionic acid unit, 3-hydroxy-n-butyric acid unit, or 3-hydroxy-n-valeric acid unit. The present invention also relates to a method for making an encapsulated particle coated with a PHA shell. Further, the present invention provides a toner and a method for making an encapsulated toner particle. Also disclosed, although outside the invention as claimed, is a laminated construct that comprises a plate or film base material coated with polyhydroxyalkanoate.

The also disclosed laminated construct can be used as a recording medium such as OHP film or inkjet recording medium.

### Related Background Art

Polymer materials are indispensable for today's life or industries. Polymer materials are utilized in various fields, for example, as hausing materials for home electrics, packing materials, buffer materials and textile materials because of cheapness, light in weight and good formability. On the other hand, various polymer functional materials such as liquid crystals and coating materials have been produced utilizing the stable properties by introducing various functional substituents into polymer chains. These functional materials can expect larger market needs in a smaller production scale due to the higher added value than the bulk polymers as structural materials. Such polymeric functional materials have been produced by organic synthetic chemistry, for example, by introducing substituents during or after synthesis process of the polymer. Most polymers to be the skeleton of functional polymers are produced from petroleum-based raw materials by organic synthetic chemistry. Such polymers include polyethylene, polyethylene terephthalate, polyesters, polystyrene, polyvinylchloride, and polyacrylamide.

As one of the constituent techniques for confer higher added value to the polymer compound, the inventors of the present invention have been focusing on a layered construct in which a base material is coated with a polymer compound. By coating a certain base material a polymer compound, a composite construct having an extremely useful functionality can be obtained. Specific applications of such a construct include, for example, a capsule toner for electrophotography having a microcapsule structure encapsulating toner components in a polymer compound, and a recording medium for inkjet recording, where a sheet of base material is coated with a polymer compound.

Generally, in the electrophotography, an electrostatic latent image is formed by various means on a photosensitive material utilizing a photoconductive substance, then the latent image is developed with a toner, and the toner image is transferred to a transfer material such as paper according to the necessity and then fixed by heat, pressure, heat and pressure, or solvent vapor to obtain a copy image. As a toner used for this purpose, a "pulverized toner" has been used. The pulverized toner is produced by melting and mixing uniformly a colorant such as dyes and pigments in a thermoplastic resin, and then pulverizing the resin mixture by means of a pulverizer and a classifier to obtain a desired particle size. Although such a toner has excellent performance, there are some problems that, for example, the selection range of the materials is limited because brittleness is required the materials due to the making step in the toner production. In order to overcome such problems, Japanese Patent Publication No. 36-10231 or the like have suggested the manufacture of "a polymerized toner" by means of suspension polymerization. In the suspension polymerization, a polymerizable monomer, a colorant, a polymerization initiator, and if necessary a crosslinking agent and a charge controlling agent all of which are uniformly dissolved or dispersed are dispersed into a continuous phase (an aqueous phase, for example) containing a dispersion stabilizer by using an agitator, and polymerization reaction is carried out to obtain a desired toner. This method has advantages that, for example, brittleness is not required for the toner material because this method does not include pulverization step and consequently soft materials can be used. However, with such a polymerized toner of a fine particle size, the colorant and the charge controlling agent tend to be exposed on the toner surface, so that the toner tends to be affected by the colorant and reduction in uniform charging is concerned. In order to solve these problems, so-called "capsule toner" of which surface is coated with a polymer layer or layers has been proposed.

For example, Japanese Patent Application Laid-Open No. 8-286416, for example, discloses a capsule toner for electrostatic development and a production method in which polymerized-particles are coated with a shell of a polar resin. In accordance with this method, a shell is formed around a core of a polymerized particle containing toner components by organic synthetic chemistry, by which the above described problem is overcome and an excellent capsule toner is obtained for electrostatic charge development with increased image durability and uniformity and stabilization of charging. In addition, Japanese Patent Application Laid-Open No. 9-292735 discloses a capsule toner for image formation in which a core made of a material of high thermal expansion and a release agent is covered with a hard resin layer. The above-described toner is a functional microcapsule designed such that the thermally expandable material within the core expands at fixation heating to break the shell and release the contained releasing agent out of the shell. Therefore, it can be expected that the above-described toner have such advantages that it can prevent offset when a film heating fixation apparatus is used, or it enables low-pressure fixation and alleviation of paper wrinkling when a roller fixing apparatus is used. Japanese Patent Application Laid-Open Nos. 5-119531, 5-249725, 6-332225, 9-43896, 10-78676, 11-7163, 2000-66444, 2000-112174, and 2000-330321 also disclose capsule constructs having a polymer shell and production methods thereof, all of which produce capsule toners by organic synthetic chemistry such as suspension polymerization, emulsion polymerization, precipitation polymerization, dispersion polymerization, soap-free emulsion polymerization, and seed polymerization.

However, these methods for making the capsule toners have some problems that the making process becomes extremely complicated and a large amount of solvents and surfactants are used in the making process.

In any of the methods mentioned above, the polymer compounds used for coating the base material are synthesized by organic synthesis, followed by addition of various functions.

Meanwhile, active studies have been done to produce polymer compounds by using biotechnology, and partly in practice. For example, known microbial polymers include polyhydroxyalkanoates (PHAs) such as poly-3-hydroxy-n-butyric acid (PHB), and copolymers of 3-hydroxy-n-butyric acid and 3-hydroxy-n-valeric acid (PHB/V); polysaccharides such as bacterial cellulose and pullulan; and polyamino acids such as poly-γ-glutamic acid and polylysine. Particularly, PHA can be processed into various products by melt-preparation etc., just like other existing plastics. In addition, because of excellent biocompatibility, application of PHA as medical soft materials is also expected.

It has been reported that many microorganisms produce PHA and accumulate it within cells. For example, microbial productions of PHB/V by *Alcaligenes eutrophus* H16 (ATCC No. 17699), *Methylobacterium* sp., *Paracoccus* sp., *Alcaligenes* sp., and *Pseudomonas* sp. have been reported (for example, Japanese Patent Application Laid-Open No. 5-74492, Japanese Patent Publication Nos. 6-15604, 7-14352, and 8-19227). Furthermore, *Comamonas acidovorans* IFO 13852 produces PHA comprised of monomer units of 3-hydroxy-n-butyric acid and 4-hydroxy-n-butyric acid (Japanese Patent Application Laid-Open No. 9-191893), and *Aeromonas caviae* produces a copolymer of 3-hydroxy-n-butyric acid and 3-hydroxyhexanoic acid (Japanese Patent Application Laid-Open Nos. 5-93049 and 7-265065).

Biosynthesis of these PHB and PHB/V is an enzymatic polymerization reaction using as a substrate (R)-3-hydroxybutyryl CoA or (R)-3-hydroxyvaleryl CoA that is synthesized from various carbon sources through various metabolic pathways within a living organism. The enzyme that catalyzes this polymerization reaction is PHB synthetase (this can be referred to as PHB polymerase or PHB synthase). CoA is an abbreviation for coenzyme A, and its chemical structure is represented by the following chemical formula.

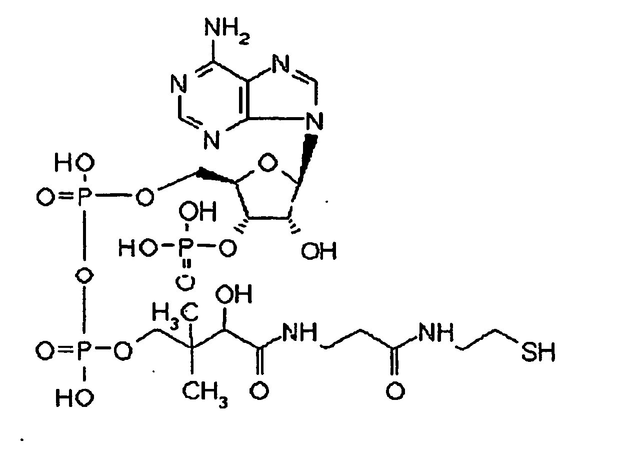

Recently, active studies on polyhydroxyalkanoate comprised of 3-hydroxyalkanoic acid units of medium-chain-length (about 3 to 12 carbon atoms) (mcl-PHA) have been conducted.

For example, Japanese Patent No. 2642937 discloses that *Pseudomonas oleovorans* ATCC 29347 can produce PHA comprised of 3-hydroxyalkanoic acid monomer units of 6 to 12 carbon atoms from non-cyclic aliphatic hydrocarbons. In addition, it has been reported, in Appl. Environ. Microbiol., 58, 746 (1992), that *Pseudomonas resinovorans* produces PHA of which monomer units are 3-hydroxy-n-butyric acid, 3-hydroxyhexanoic acid, 3-hydroxyoctanoic acid, and 3-hydroxydecanoic acid using octanoic acid as a sole carbon source, and it also produces PHA of which monomer units are 3-hydroxy-n-butyric acid, 3-hydroxyhexanoic acid, 3-hydroxyoctanoic acid, and 3-hydroxydecanoic acid using hexanoic acid as sole carbon source. Here, the 3-hydroxyalkanoic acid monomer units longer than the raw material fatty acid are considered derived from the fatty acid synthesizing pathway described below.

Int. J. Biol. Macromol., 16 (3), 119 (1994) reported that *Pseudomonas* sp. Strain 61-3 produces PHA comprised of monomer units of 3-hydroxyalkanoic acids such as 3-hydroxy-n-butyric acid, 3-hydroxyhexanoic acid, 3-hydroxyoctanoic acid, and 3-hydroxydecanoic acid, and 3-hydroxyalkenoic acids such as 3-hydroxy-5-cis-decenoic acid and 3-hydroxy-5-cis-dodecenoic acid, using sodium gluconate as a sole carbon source.

The above-described PHAs are comprised of monomer units having alkyl groups as the side chain (usual-PHAs). However, when wider application of PHA, e.g., as a functional polymer, is intended, PHA having side chains other than alkyl groups (for example, side chains having substituents such as phenyl group, unsaturated hydrocarbons, ester groups, allyl group, cyano group, halogenated hydrocarbons, and epoxides) is extremely useful (unusual-PHA).

As for biosynthesis of unusual-PHA having phenyl groups, it was reported that *Pseudomonas oleovorans* produced PHA having 3-hydroxy-5-phenylvaleric acid units from 5-phenylvaleric acid (Polymers, 24, 5256-5260 (1991), Macromol. Chem., 191, 1957-1965 (1990); Chirality, 3, 492-494 (1991)). Polymers, 29, 1762-1766 (1996) reported that *Pseudomonas oleovorans* produced PHA having 3-hydroxy-5-(4-tolyl)valeric acid units from 5-(4-tolyl)valeric acid (5-(4-methylphenyl)valeric acid). Further, Polymers, 32, 2889-2895 (1999) reported that *Pseudomonas oleovorans* produced PHA having 3-hydroxy-5-(2,4-dinitrophenyl)valeric acid units and 3-hydroxy-5-(4-nitrophenyl)valeric acid units from 5-(2,4-dinitrophenyl)valeric acid.

As for unusual-PHA having phenoxy groups, Macromol. Chem. Phys., 195, 1665-1672 (1994) reported that *Pseudomonas oleovorans* produced PHA having 3-hydroxy-5-phenoxyvaleric acid units and 3-hydroxy-9-phenoxynonanoic acid units from 11-phenoxyundecanoic acid. Also, Polymers, 29, 3432-3435 (1996) reported that Pseudomonas oleovorans produced a PHA having 3-hydroxy-4-phenoxybutyric acid units and 3-hydroxy-6-phenoxyhexanoic acid units from 6-phenoxyhexanoic acid, a PHA having 3-hydroxy-4-phenoxybutyric acid units, 3-hydroxy-6-phenoxyhexanoic acid units, and 3-hydroxy-8-phenoxyoctanoic acid units from 8-phenoxyoctanoic acid, and a PHA having 3-hydroxy-5-phenoxyvaleric acid unit and 3-hydroxy-7-phenoxyheptanoic acid units from 11-phenoxyundecanoic acid. Further, Can. J. Microbiol., 41, 32-43 (1995) reported that *Pseudomonas oleovorans* ATCC 29347 and *Pseudomonas putida* KT 2442 produced PHA having 3-hydroxy-p-cyanophenoxyhexanoic acid units and PHA having 3-hydroxy-p-nitrophenoxyhexanoic acid units from p-cyanophenoxyhexanoic acid and p-nitrophenoxyhexanoic acid respectively. Japanese Patent No. 2989175 describes a homopolymer comprised of 3-hydroxy-5-(monofluorophenoxy)valeric acid units or 3-hydroxy-5-(difluorophenoxy)valeric acid units and a copolymer containing at least 3-hydroxy-5-(monofluorophenoxy)pentanoate unit or 3-hydroxy-5-(difluorophenoxy)pentanoate unit and a method for producing such homopolymer or copolymer, reciting that such homopolymer and copolymer can provide water-repellency and stereoregularity with high melting point and good workability.

As an example of unusual-PHA having a cyclohexyl group, Polymers, 30, 1611-1615 (1997) reported that *Pseudomonas oleovorans* produced such PHA from cyclohexylbutyric acid or cyclohexylvaleric acid.

These mcl-PHA and unusual-PHA are synthesized through an enzymatic polymerization reaction using (R)-3-hydroxyacyl CoA as a substrate. Such 3-hydroxyacyl CoAs are produced through various metabolic pathways (for example, β-oxidation pathway or fatty acid synthesis pathway) in a living organism from different alkanoic acids. The enzyme that catalyzes this polymerization reaction is PHA synthetase (this can be referred to as PHA polymerase or PHA synthase). The following is the reaction route from alkanoic acid to PHA via the β-oxidation pathway and polymerization reaction by PHA synthetase.

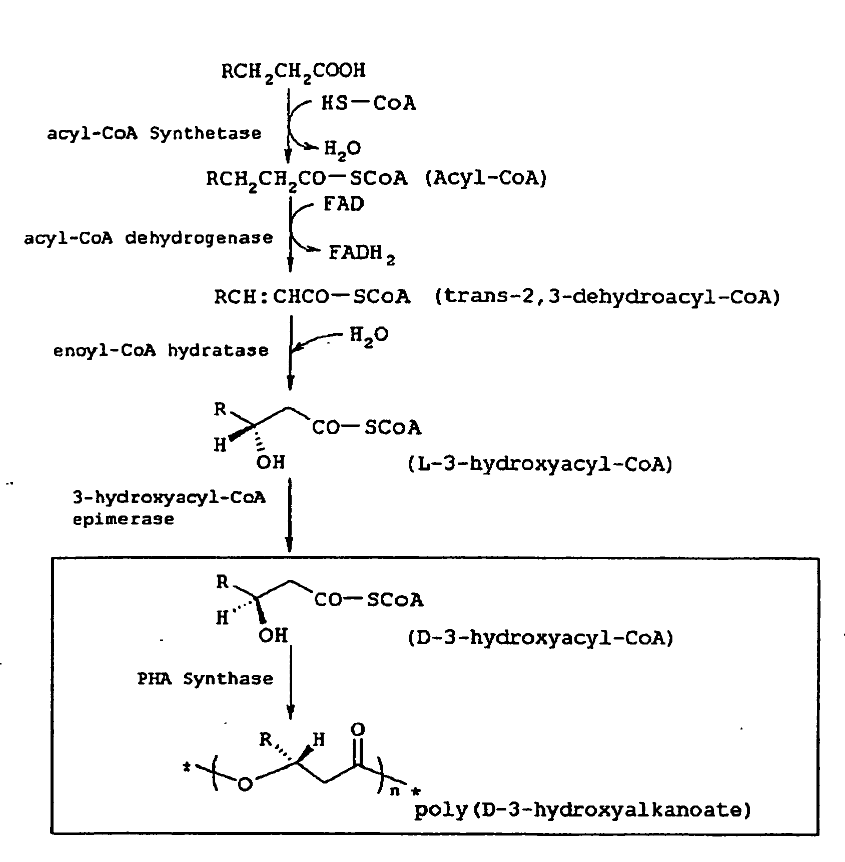

On the other hand, when the production is performed through the fatty acid synthesis pathway, it is considered that (R)-3-hydroxyacyl-ACP (ACP means acyl carrier protein) generated in this pathway is converted to (R)-3-hydroxyacyl CoA from which PHA is synthesized by PHA synthetase.

Recently, attempts have been made to synthesize PHA *in vitro* using PHB synthetase or PHA synthetase isolated from cells.

For example, Proc. Natl. Acad. Sci. USA, 92, 6279-6283 (1995) describes that PHB comprised of 3-hydroxy-n-butyric acid units has been successfully synthesized by using PHB synthetase derived from *Alcaligenes eutrophus* and 3-hydroxybutyryl CoA as a substrate. In addition, Int. J. Biol. Macromol., 25, 55-60 (1999) describes that PHA comprised of 3-hydroxy-n-butyric acid units or 3-hydroxy-n-valeric acid units has been successfully synthesized by reacting PHB synthetase derived from *Alcaligenes eutrophus* with 3-hydroxybutyryl CoA or 3-hydroxyvaleryl CoA. Further, this report mentions that, when racemic 3-hydroxybutyryl CoA was reacted with PHB synthetase, PHB comprised of only (R) 3-hydroy-n-butyric acid units was successfully synthesized due to the stereoselectivity of the enzyme. Macromol. Rapid Commun., 21, 77-84 (2000) reported in vitro PHB synthesis using PHB synthetase derived from *Alcaligenes eutrophus.*

FEMS Microbiol. Lett., 168, 319-324 (1998) describes that PHB comprised of 3-hydroxy-n-burytic acid units was successfully synthesized by reacting PHB synthetase derived from *Chromatium vinosum* with 3-hydroxybutyryl CoA.

In Appl. Mirobiol. Biotechnol., 54, 37-43 (2000), PHA comprised of 3-hydroxydecanoic acid is synthesized by reacting PHA synthetase derived from *Pseudomonas aeruginosa* with 3-hydroxydecanoyl CoA.

As described above, application of biotechnological methods to polymer synthesis may enable synthesis of new polymer compounds which can not be made by conventional organic synthesis or enable imparting new functions and constructs to polymer compounds. In addition, there are many cases where conventional multi-step reaction can be replaced with only one step reaction, so that simplification of the production process, cost reduction, and time saving are expected. Further, this enables consumption reduction of organic solvents, acids, alkalis, surfactants, etc., moderate reaction conditions, and synthesis from non-petroleum raw materials or crude raw materials, thus enables a synthesis process of lower environmental burden and of resource-recycling type. In more detail with synthesis from the crude raw materials, the substrate specificity of the enzyme used as a catalyst in biotechnological synthesis is high in general, so that it is possible to selectively promote a desired reaction even when the low purity raw material is used. Thus, use of waste materials or recycled materials can be expected.

On the other hand, as an elemental technical idea for adding values to polymer compounds, the inventors of the present invention has been focusing on a construct in which a base material is coated with a polymer compound as described above. Coating a certain base material with a polymer compound can provide composite constructs having extremely useful functionality. Conventionally, organic synthesis techniques have been used to produce such constructs, but there are certain limits to such techniques.

If such a construct can be manufactured by using a biotechnological method as described above, it is possible to utilize new polymer compounds that can not be realized by conventional organic synthesis techniques or to impart new functions and constructs to polymer compounds. It is also possible to achieve a making process being less burdening environment and of resource-recycling type at a low cost. For example, utilizing the highly strict molecular recognition ability or stereoselectivity specific to biocatalysts, it is possible to produce capsule constructs or laminated constructs coated with a new functional polymer compound or a polymer compound having extremely high chirality that could not be realized through conventional techniques of the synthetic organic chemistry, through a simple process with low environmental burden.

Therefore, the present invention provides a construct of highly functional polymer compound that can be manufactured by a biotechnological method. The present invention also provides an efficient method for making a construct of a base material coated with a polymer compound usable as a functional composite construct.

US 6,025,028 discloses polyhydroxyalkanoate coatings formed by melting PHA pellets and extruding molten PHA to an object to be coated.

### SUMMARY OF THE INVENTION

As a result of intensive investigation to achieve the above objects, the inventors have found that a construct of a base material coated with PHA can be obtained by immobilizing PHA synthetase to a surface of the base material and adding 3-hydroxyacyl CoA thereto and synthesizing desired PHA on the surface of the base material. Thus, the inventors have accomplished the present invention. Furthermore, the inventors have found that a construct having various improved properties can be obtained by chemically modifying the above-described PHA. More specifically, the inventors have found that, for example, by introducing a graft chain into PHA, one can obtain a construct of which base material is at least partly coated with PHA having properties derived from the introduced graft chains. In addition, the inventors have found that by crosslinking PHA, one can obtain a construct of which base material is coated with PHA having desired physico-chemical properties (for example, mechanical strength, chemical resistance, heat resistance, etc.). In the present invention, the chemical modification means alteration of the molecular structure of the polymeric material by a chemical reaction within the polymer or between polymer molecules or between the polymer and other chemical substance. The crosslinking means formation of a network structure by chemical or physico-chemical intramolecular or intermolecular bonding of the polymeric materials, and a crosslinking agent means a substance having a certain reactivity with the above described polymeric material to be added for crosslinking reaction.

In the light of the aforementioned prior art and objects, the present invention provides the electrophotographic encapsulated toner particle according to claim 1, the method for making an encapsulated particle according to claim 19, the toner according to claim 37, and the method for making a toner according to claim 38. The other claims relate to further developments.

In addition, the present invention relates to a capsule construct having a base material as a core and mcl-PHA or unusual-PHA as a shell, and more particularly, the present invention relates to a capsule construct in which the core contains colorants at the least, a capsule construct in which the colorants contain pigments at the least, or a capsule construct in which the core is a pigment.

### BRIEF DESCRIPTION OF THE DRAWINGS

FIG. 1 is a GC-MS analytical result of the shell of a capsule construct in Example 1;
FIG. 2 is a GC-MS analytical result of the shell of a capsule construct in Example 4;
FIG. 3 is a GC-MS analytical result of the shell of a capsule construct in Example 5; and
FIG. 4 is a GC-MS analytical result of the coating layer of a laminated construct in Example 11.

### DESCRIPTION OF THE PREFERRED EMBODIMENTS

The toner particle according to the present invention has a configuration in which a base material is coated with PHA comprised of various types of monomer units having a substituent on side chains. Such a construct is extremely useful and highly functional capsule toner for electrophotography. The present invention will be described in detail.

### <PHA>

PHA which is available for the present invention is not particularly limited as long as the PHA can be synthesized by a PHA synthetase participating with mcl-PHA synthesis, that is, various mcl-PHAs and unusual-PHAs are included in PHA. As described above, PHA synthetase is an enzyme that catalyzes the final step in the *in vivo* PHA synthesis, so that any PHA known to be synthesized in a living organism is synthesized by the catalytic action of PHA synthetase. Therefore, it is possible to make a construct of which base material is coated with any kind of PHA known to be synthesized *in vivo,* by reacting 3-hydroxyacyl CoA corresponding to the desired PHA with the enzyme immobilized on the base material.

Such PHA may contain monomer units represented by the following chemical formulae [1] to [10].

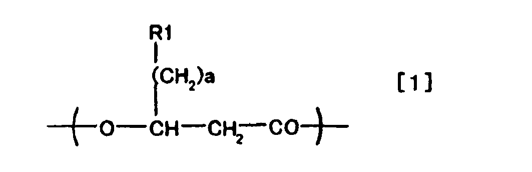

wherein R1 and a are selected from the group of combinations consisting of:
(1) R1 is a hydrogen atom (H) and a is any of integers from 3 to 10;
(2) R1 is a halogen atom and a is any of integers from 1 to 10;
(3) R1 is a chromophore and a is any of integers from 1 to 10;
(4) R1 is a carboxyl group or a salt thereof and a is any of integers from 1 to 10; and
(5) R1 is 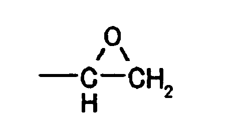
and a is any of integers from 1 to 7.

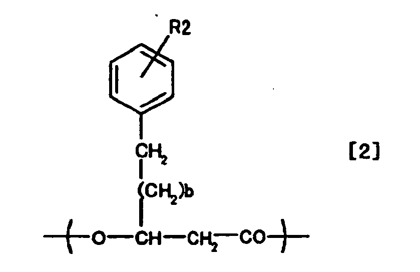

wherein b represents any of integers from 0 to 7 and R2 represents any one selected from the group consisting of hydrogen atom (H), halogen atoms, -CN, -NO₂, -CF₃, -C₂F₅, and -C₃F₇.

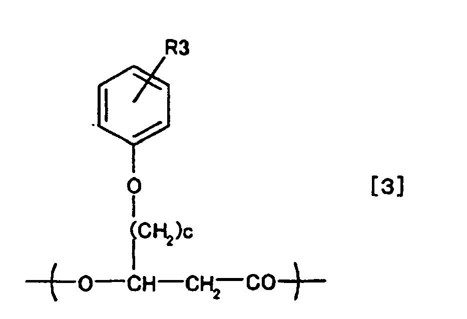

wherein c represents any of integers from 1 to 8 and R3 represents any one selected from the group consisting of hydrogen atom (H), halogen atoms, -CN, -NO₂, -CF₃, -C₂F₅, and -C₃F₇.

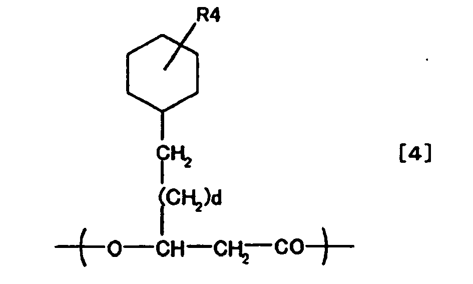

wherein d represents any of integers from 0 to 7 and R4 represents any one selected from the group consisting of hydrogen atom (H), halogen atoms, -CN, -NO₂, -CF₃, -C₂F₅, and -C₃F₇.

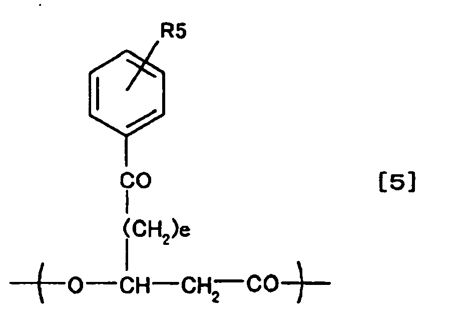

wherein e represents any of integers from 1 to 8 and R5 represents any one selected from the group consisting of hydrogen atom (H), halogen atoms, -CN, -NO₂, -CF₃, -C₂F₅, -C₃F₇, -CH₃, -C₂H₅, and -C₃H₇.

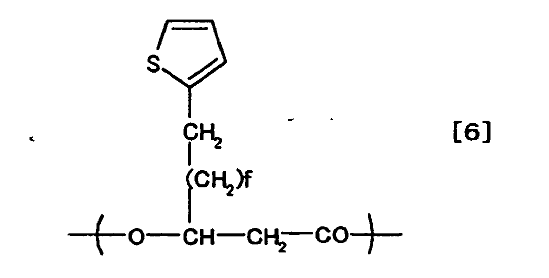

wherein f represents any of integers from 0 to 7.

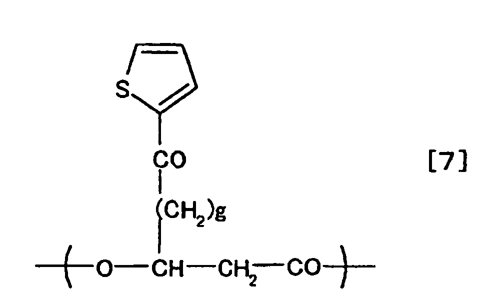

wherein g represents any of integers from 1 to 8.

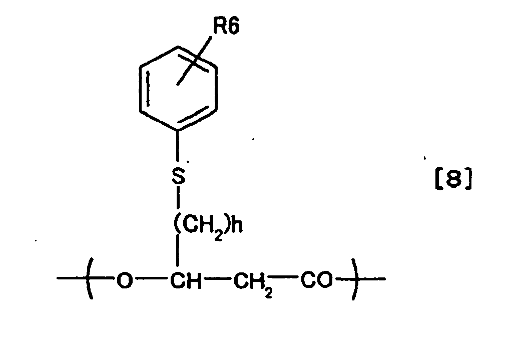

wherein h represents any of integers from 1 to 7 and R6 represents any one selected from the group consisting of hydrogen atom (H), halogen atoms, -CN, **-**NO₂**,** -COOR', -SO₂R⁻, -CH₃, -C₂H₅, -C₃H₇, -CH(CH₃)₂, and -C(CH₃)₃, wherein R' is selected from the group consisting of hydrogen atom (H), Na, K, -CH₃, and -C₂H₅, and R" is selected from the group consisting of -OH, -ONa, -OK, halogen atoms, -OCH₃, and -OC₂H₅.

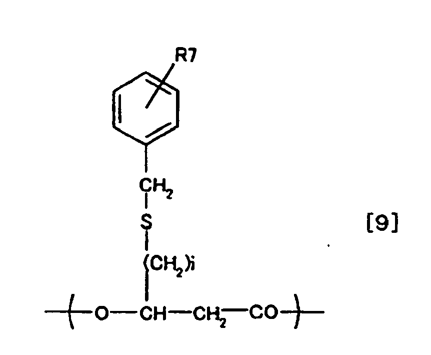

wherein i represents any of integers from 1 to 7 and R7 represents any one selected from the group consisting of hydrogen atom (H), halogen atoms, -CN, -NO₂, -COOR', -SO₂R", in which R' is selected from the group consisting of hydrogen atom (H), Na, K, -CH₃, and -C₂H₅, and R" is selected from the group consisting of -OH, -ONa, -OK, halogen atoms, -OCH₃, and -OC₂H₅.

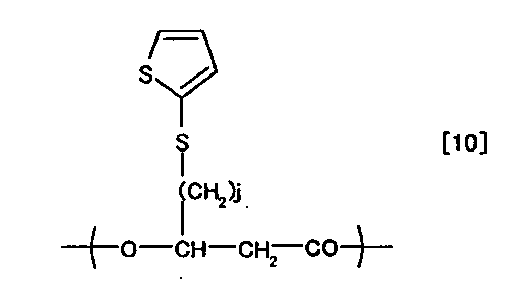

wherein j represents any of integers from 1 to 9.

In the above formulas 1 to 10, halogen atoms can be fluorine, chlorine, or bromine. The chromophores are not particularly limited as long as the 3-hydroxyacyl CoA having the chromophore is catalyzed by PHA synthetase, but it is preferable that a methylene chain of 1 to 5 carbon atoms is present between the chromophore and the carboxyl group to which CoA is bonded, in view of steric hindrance at the time of polymer synthesis. When the light absorption wavelength of the chromophore is within a visible range, a colored construct can be obtained and when the light absorption wavelength is outside the visible range, the construct may be used as various electronic materials. Such chromophores include nitroso, nitro, azo, diarylmethane, triarylmethane, xanthene, acridine, quinoline, methine, thiazole, indamine, indophenol, lactone, aminoketone, hydroxyketone, stilbene, azine, oxazine, thiazine, anthraquinone, phthalocyanine, and indigoid.

As PHA which is used for the present invention, random copolymers or block copolymers which include a plurality of the above described monomer units can be used. Therefore, it becomes possible to control physical properties of PHA and add some functions to the PHA by utilizing properties of each monomer unit or functional groups included therein, and also possible to manifest new functions obtained by utilizing interaction between functional groups.

Further, it is possible to change a monomer unit composition of the PHA in a radial direction in the case where a shape of the construct is like a particle and in a vertical direction in the case where a shape of the construct is like a plate, by changing the composition with time through alteration of kinds and concentrations of 3-hydroxyacyl CoA which is a base materials.

Consequently, in the case of a capsule toner for example, the toner becomes possible to simultaneously possess a plurality of functions such as an excellent blocking resistance at the time of preserving the toner and an excellent low temperature fixing property at the time of the fixing, by forming PHA having a high glass transition temperature as a surface layer of the toner and by forming PHA having a low glass transition temperature as an inner layer of the toner.

In addition, if it requires that a coating construct is formed by PHA which has a less affinity for a base material for example, it becomes possible to form a PHA coating which is strongly bound to the base material by firstly coating the base material with PHA having a high affinity for the base material and then changing a monomer unit composition of the PHA having a high affinity for the base material in its radial direction or in its vertical direction to obtain a desired monomer unit composition of the PHA, for example, a multilayered construct or a gradient construct.

PHA comprising only 3-hydroxypropionic acid unit, 3-hydroxy-n-butyric acid unit, 3-hydroxy-n-valeric acid unit, or 4-hydroxy-n-butyric acid unit is not applicable to mcl-PHA or unusual-PHA, but PHA in which these monomer units are mixed in the previously illustrated monomer units is available for the present invention. In addition, a chemical modification can be performed after synthesizing PHA or during synthesizing PHA, as required. As for the molecular weight of PHA, the number average molecular weight of PHA is about 1,000 to 10 mullion, and preferably about 3,000 to a million when the above described construct is used as a capsule toner for electrophotography.

In the present invention, the PHA synthesized by PHA synthetase and used for the construct of the present invention is generally isotactic polymer comprised of R bodies alone.

### <3-hydroxyacyl CoA>

Specifically, 3-hydroxyacyl CoA usable as the substrate of PHA synthetase in the present invention is represented by the following chemical formulae [11] to [20].

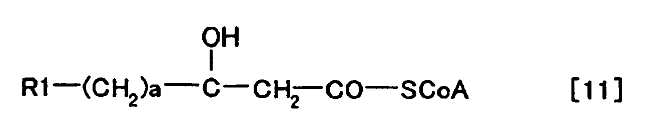

wherein -SCoA represents coenzyme A bonded to alkanoic acid, and R1 and a are as defined for the above-described chemical formula [1].

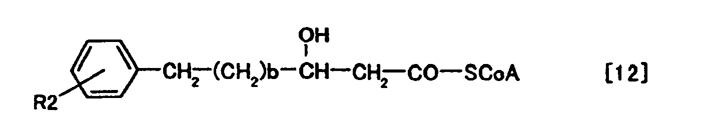

wherein -SCoA represents coenzyme A bonded to alkanoic acid, R2 and b are as defined for the above-described chemical formula [2],

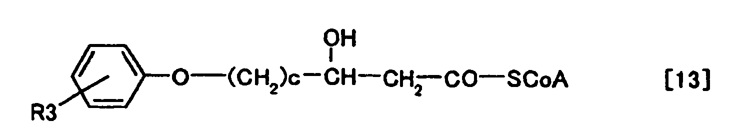

wherein -SCoA represents coenzyme A bonded to alkanoic acid, R3 and c are as defined for the above-described chemical formula [3].

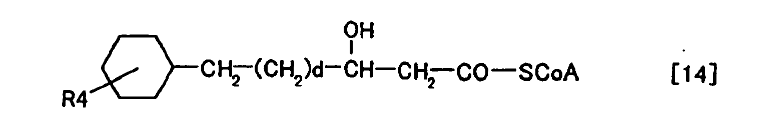

wherein -SCoA represents coenzyme A bonded to alkanoic acid, R4 and d are as defined for the above-described chemical formula [4].

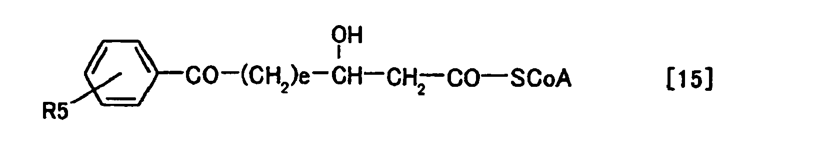

wherein -SCoA represents coenzyme A bonded to alkanoic acid, R5 and e are as defined for the above-described chemical formula [5].

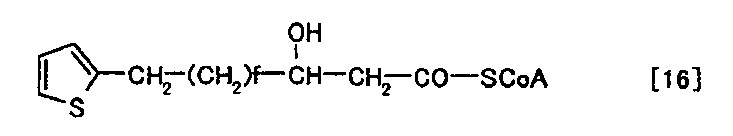

wherein -SCoA represents coenzyme A bonded to alkanoic acid, and f is as defined for the above-described chemical formula [6].

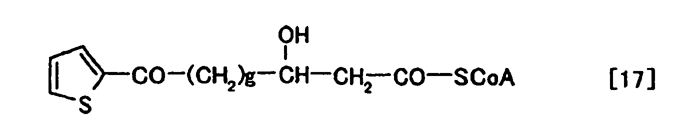

wherein, -SCoA represents coenzyme A bonded to alkanoic acid, and g represents any of integers from 1 to 8 as defined for the above-described chemical formula [7].

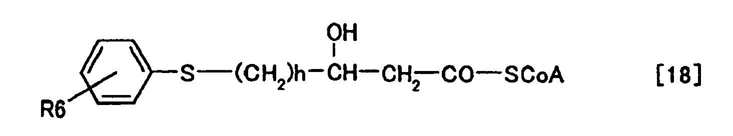

wherein, -SCoA represents coenzyme A bonded to alkanoic acid, R6 and h are as defined for the above-described chemical formula [8].

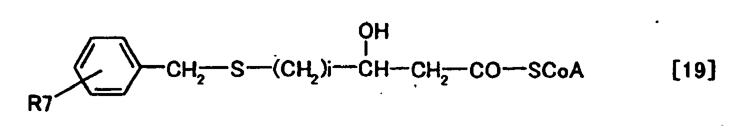

wherein, -SCoA represents coenzyme A bonded to alkanoic acid, R7 and i are as defined for the above-described chemical formula [9].

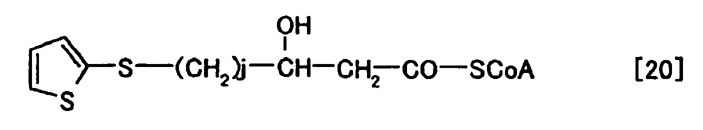

wherein, -SCoA represents coenzyme A bonded to alkanoic acid, and j represents any of integers from 1 to 9 as defined for the above-described chemical formula [10].

These 3-hydroxyacyl CoAs can be synthesized by a suitable method selected from, for example, *in vitro* synthesis use an enzyme, *in vivo* synthesis using living organisms such as microorganisms and plants, and chemical synthesis. Enzymatic synthesis, especially, is commonly used to synthesize these substrates. For example, it is known a method to use a commercially available acyl CoA synthetase (acyl CoA ligase, E. C. 6. 2. 1. 3) to catalyze the following reaction: 3-hydroxyalkanoic acid + CoA → 3-hydroxyacyl CoA (Eur. J. Biochem., 250, 432-439 (1997), Appl. Microbiol. Biotechnol., 54, 37-43 (2000) etc.). The synthesis process using enzyme or organism may be a batch process or a continuous process using immobilized enzyme or cells.

### <PHA synthetase and production microorganisms>

The PHA synthetase used in the present invention can be produced by using a microorganism selected from the microorganisms known to produce PHA synthetase, or by using a transformant to which the PHA synthetase gene of such a microorganism has been introduced.

For example, mcl-PHA- or unusual-PHA-producing microorganisms can be used as the PHA synthetase-producing microorganism. Such microorganisms include, in addition to the above described *Pseudomonas oleovorans, Pseudomonas resinovorans, Pseudomonas* sp. strain 61-3, *Pseudomonas putida* KT 2442, and *Pseudomonas aeruginosa,* strains of *Pseudomonas* sp. such as *Pseudomonas putida* P91, *Pseudomonas cichorii* H45, *Pseudomonas cichorii* YN2, and *Pseudomonas jessenii* P161 all of which were isolated by the inventors, strains belonging to *Burkholderia* sp. such as *Burkholderia* sp. OK3, FERM P-17370 described in Japanese Patent Application Laid-Open No. 2001-78753 and *Burkholderia* sp. OK4, FERM P-17371 described in Japanese Patent Application Laid-Open No. 2001-69968. In addition to the above-described microorganisms, it is possible to use microorganisms of genus Aeromonas and *Comamonas* that can produce mcl-PHA and unusual-PHA.

Strains P91, H45, YN2 and P161 have been deposited (accession number: FERM BP-7373, FERM BP-7374, FERM BP-7375, and FERM BP-7376 respectively) in International Patent Organism Depositary of Institute of Advanced Industrial Science and Technology (former National Institute of Bioscience and Human Technology, Agency of Industrial Science and Technology), 1-3, Higashi 1-chome, Tsukuba-shi, Ibaraki-ken, 305-0046, Japan, under the Budapest Treaty on the International Recognition of the Deposit of Microorganism for the Purpose of Patent Procedure.

Microbiological properties of the above described P91, H45, YN2 and P161 are as follows. As for the strain P161, the base sequence of 16S rRNA is as SEQ ID NO: 1.

### Pseudomonas putida P91

### (1) Morphology

Form and size of the cell: rod, 0.6 µm × 1.5 µm
Polymorphism of the cell: -
Mobility: +
Spore formation: -
Gram stain: negative
Colony shape: circular, smooth edge, low convex, smooth surface, lustrous, cream color

### (2) Physiological properties

Catalase: positive
Oxidase: positive
O/F test: oxidizing type
Reduction of nitrate: negative
Production of indole: negative
Acidification of glucose: negative
Arginine dihydrolase: positive
Urease: negative
Esculin hydrolysis: negative
Gelatin hydrolysis: negative β-galactosidase: negative
Fluorescent dye production on King's B agar: positive

### (3) Substrate assimilation

Glucose: positive ,
L-arabinose: negative
D-mannose: negative
D-mannitol: negative
N-acetyl-D-glucosamine: negative
Maltose: negative
Potassium gluconate: positive
n-capric acid: positive
Adipic acid: negative
dl-malic acid: positive
Sodium citrate: positive
Phenyl acetate: positive

### Pseudomonas cichorii H45

### (1) Morphology

Form and size of the cell: rod, 0.8 µm × 1.0 to 1.2 µm
Polymorphism of the cell: -
Mobility: +
Spore formation: -
Gram stain: negative
Colony shape: circular, smooth edge, low convex, smooth surface, lustrous, cream color

### (2) Physiological properties

Catalase: positive
Oxidase: positive
O/F test: oxidizing type
Reduction of nitrate: negative
Production of indole: negative
Acidification of glucose: negative
Arginine dihydrolase: negative
Urease: negative
Esculin hydrolysis: negative
Gelatin hydrolysis: negative
β-galactosidase: negative
Fluorescent dye production on King's B agar: positive
Growth in 4% NaCl: negative
Accumulation of poly-β-hydroxybutyric acid: negative

### (3) Substrate assimilation

Glucose: positive
L-arabinose: negative
D-mannose: positive
D-mannitol: positive
N-acetyl-D-glucosamine: positive
Maltose: negative
Potassium gluconate: positive
n-capric acid: positive
Adipic acid: negative
dl-malic acid: positive
Sodium citrate: positive
Phenyl acetate: positive

### Pseudomonas cichorii YN2

### (1) Morphology

Form and size of the cell: rod, 0.8 µm × 1.5 to 2.0 µm
Polymorphism of the cell: -
Mobility: +
Spore formation: -
Gram stain: negative
Colony shape: circular, smooth edge, low convex, smooth surface, lustrous, translucent

### (2) Physiological properties

Catalase: positive
Oxidase: positive
O/F test: oxidizing type
Reduction of nitrate: negative
Production of indole: positive
Acidification of glucose: negative
Arginine dihydrolase: negative
Gelatin hydrolysis: negative
β-galactosidase: negative
Fluorescent dye production on King's B agar: positive
Growth in 4% NaCl: positive (weakly growth)
Accumulation of poly-β-hydroxybutyric acid: negative
Hydrolysis of Tween 80: positive

### (3) Substrate assimilation

Glucose: positive
L-arabinose: positive
D-mannose: negative
D-mannitol: negative
N-acetyl-D-glucosamine: negative
Maltose: negative
Potassium gluconate: positive
n-capric acid: positive
Adipic acid: negative
dl-malic acid: positive
Sodium citrate: positive
Phenyl acetate: positive

### Pseudomonas jessenii P161

### (1) Morphology

Form and size of the cell: spherical: φ 0.6 µm, rod: 0.6 µm × 1.5 to 2.0 µm
Polymorphism of the cell: + (elongation) Mobility: +
Spore formation: -
Gram stain: negative
Colony shape: circular, smooth edge, low convex, smooth surface, pale yellow

### (2) Physiological properties

Catalase: positive
Oxidase: positive
O/F test: oxidizing type
Reduction of nitrate: positive
Production of indole: negative
Arginine dihydrolase: positive
Urease: negative
Esculin hydrolysis: negative
Gelatin hydrolysis: negative
β-galactosidase: negative
Fluorescent dye production on King's B agar: positive

### (3) Substrate assimilation

Glucose: positive
L-arabinose: positive
D-mannose: positive
D-mannitol: positive
N-acetyl-D-glucosamine: positive
Maltose: negative
Potassium gluconate: positive
n-capric acid: positive
Adipic acid: negative
dl-malic acid: positive
Sodium citrate: positive
Phenyl acetate: positive

For routine culture of the PHA synthetase-producing microorganisms, for example, to prepare cell stocks, to obtain sufficient cells for enzyme production or to maintain active state of the cells, one can select suitable culture medium containing ingredients necessary for the growth of microorganisms. Any culture medium can be used as long as it does not interfere microbial growth or vitality, including common natural media such as nutrient broth and yeast extracts, and synthetic media supplemented with nutrients.

Any culture method, such as liquid culture and solid culture, can be used as long as the subject microorganism can proliferate. It may be batch culture, fed-batch culture, semi-continuous culture, or continuous culture. Liquid batch culture includes a method of shaking a culture flask to supply oxygen, and a method using a jar fermenter that supplies oxygen by aeration. A plurality of these processes may be combined as a multistage method.

To produce PHA synthetase by using the above described PHA-producing microorganism, first the microorganism is grown on an inorganic medium containing alkanoic acid such as octanoic acid and nonanoic acid, and then the cells in the late logarithmic to early stationary growth phase are harvested by centrifugation or the like to extract the enzyme from the cells. When cells are cultured as above, mcl-PHA is synthesized in the cells from added alkanoic acid. In this case, it has been considered that PHA synthetase exists in a bound form to the fine particles of PHA synthesized in the cell. However, the inventors have found that substantial enzyme activity is present in the supernatant when the cultured cells were disrupted and centrifuged. Presumably, a certain amount of free PHA synthetase is present because this enzyme is actively synthesized during this relatively early growth phase of the late logarithmic to early stationary phase.

Any inorganic culture medium can be used for the above described culture process as long as the medium contains ingredients such as phosphorus source (phosphate etc.), and nitrogen source (ammonium salt, nitrate etc.) to support microbial growth. Therefore, MSB medium, E medium (J. Biol. Chem., 218, 97-106 (1956)), or M9 medium can be used as the inorganic salt medium, for example. Composition of M9 medium which is used in Examples is as follows.

| | |
|---|---|
| Na₂HPO₄: | 6.2 g |
| KH₂PO₄: | 3.0 g |
| NaCl: | 0.5 g |
| NH₄Cl: | 1.0 g |
| (per liter, pH 7.0) | |

In addition, it is preferable to add the stock solution of trace ingredients of the following composition to about 0.3% (v/v), for good proliferation and production of PHA synthetase.

### Stock solution of trace ingredients

| | |
|---|---|
| Nitrilotriacetic acid: | 1.5 g |
| MgSO₄: | 3.0 g |
| MnSO₄: | 0.5 g |
| NaCl: | 1.0 g |
| FeSO₄ : | 0.1 g |
| CaCl₂: | 0.1 g |
| CoCl₂: | 0.1 g |
| ZnSO₄: | 0.1 g |
| CuSO₄: | 0.1 g |
| AlK(SO₄)₂: | 0.1 g |
| H₃BO₃: | 0.1 g |
| Na₂MoO₄: | 0.1 g |
| NiCl₂: | 0.1 g |
| (per liter) | |

Culture temperature is chosen to be favorable for proliferation of the above strains, so that, for example, in the range of 14 to 40°C, preferably about 20 to 35°C.

It is also possible to produce PHA synthetase by using a transformant to which the PHA synthetase gene of the above PHA producing strain has been introduced. Cloning of the PHA synthetase gene, construction of expression vectors and transformants can be done according to the conventional methods.
To culture a transformant obtained using a bacterial host such as *Escherichia coli,* natural media such as LB medium or synthetic media such as M9 medium can be used. Cells are cultured with aeration for 8 to 27 hours at 25 to 37°C. After culture, cells are collected to recover PHA synthetase accumulated in the cells. Antibiotics such as kanamycin, ampicillin, tetracycline, chloramphenicol, and streptomycin may be added to the culture as required. In addition, if the an inducible promoter is used in the expression vector, expression may be promoted by adding a corresponding inducer to the culture medium when the transformant is cultured. Such an inducer may be isopropyl-β-D-thiogalactopyranoside (IPTG), tetracycline, or indoleacrylic acid (IAA).

The PHA synthetase may be a crude enzyme such as a cell lysate or protein components precipitated with ammonium sulfate, or a purified enzyme purified by various methods. The enzyme preparation may be added with a stabilizer or activator such as metallic salts, glycerin, dithiothreitol, EDTA, and bovine serum albumin (BSA) as required.

PHA synthetase may be isolated and purified by any method as long as the enzyme activity is maintained. For example, the enzyme can be purified as follows: a crude enzyme or ammonium sulfate precipitate thereof, prepared by disrupting microbial cells by using a French press or ultrasonic homogenizer, lysozyme, or various surfactants and by centrifuging the cell lysate, is purified by affinity chromatography, cation or anion exchange chromatography, gel filtration, or a certain combination thereof. Recombinant proteins, those expressed as a fusion protein having a tag such as histidine residue at N-terminus or C terminus, can be purified easily by binding through this tag to the affinity resin. The protein of interest may be isolated from the fusion protein by treating with protease such as thrombin and blood coagulation factor Xa, by lowering pH, or by adding high concentration imidazole as a binding competitive agent. Alternatively, when pTYB1 (made by New England Biolabs Inc.) was used as an expression vector, and the tag contains inteins, the bonding may be broken under reduced conditions by using dithiothreitol. In addition to the histidine tag, glutathione S-transferase (GST), chitin binding domain (CBD), maltose binding protein (MBP), and thioredoxin are known to allow affinity purification of fusion proteins. The GST fusion protein can be purified by a GST affinity resin.

Enzyme activity of PHA synthetase can be measured various known methods. For example, the following method that measures CoA released from 3-hydroxyacyl CoA during PHA polymerization reaction catalyzed by PHA synthetase utilizing color development with 5,5'-dithiobis-(2-nitrobenzoic acid):
Reagent 1: a 3.0 mg/ml solution of bovine serum albumin (Sigma) dissolved in 0.1 M Tris-HCl buffer (pH 8.0), Reagent 2: a 3.0 mM solution of 3-hydroxyoctanoyl CoA in 0.1 M Tris-HCl buffer (pH 8.0); Reagent 3: a 10 mg/ml solution of trichloroacetic acid in 0.1 M Tris-HCl buffer (pH 8.0), Reagent 4: a 2.0 mM solution of 5,5'-dithiobis-(2-nitrobenzoic acid) in 0.1 M Tris-HCl buffer (pH 8.0).

- First reaction (PHA synthesizing reaction): 100 µl of Reagent 1 is added to and mixed with 100 µl of the sample (enzyme) solution, then the mixture is preincubated for one minute at 30°C, to which 100 µl of Reagent 2 is added and mixed. The resultant mixture is incubated for 1 to 30 minutes at 30°C and the reaction is stopped by adding Reagent 3.
- Second reaction (color development of free CoA): The resulting first reaction solution is centrifuged (15,000 × g, for 10 minutes). To 500 µl of the supernatant, 500 µl of Reagent 4 is added and incubated for 10 minutes at 30°C. Then the absorbance at 412 nm is measured.
- Calculation of enzyme activity: The amount of enzyme that releases 1 µmol of CoA within one minute is defined as one unit (U).

Generally, PHA synthesized by the above described enzyme is an isotactic polymer made with R bodies alone.

### <Base material>

The base material used in the present invention can be appropriately selected from ordinary polymer compounds and inorganic solid materials such as resins, glass, and metals as long as PHA synthetase can be immobilized to it. The type and structure of the base material can be chosen appropriately according to the method for immobilizing PHA synthetase to the base material or application form of the resulting construct.

For example, the following substances can be used as a base material (core) of a capsule construct according to the present invention but are not limited thereto as a matter of course: resin particulates produced by polymerization of polymerizable monomers selected from the group consisting of styrenic polymerizable monomers such as styrene, α-methylstyrene, β-methylstyrene, o-methylstyrene, m-methylstyrene, p-methylstyrene, 2,4-dimethylstyrene, p-n-butylstyrene, p-tert-butylstyrene, p-n-hexylstyrene, p-n-octylstyrene, p-n-nonylstyrene, p-n-decylstyrene, p-n-dodecylstyrene, p-methoxystyrene, and p-phenylstyrene, acrylic polymerizable monomers such as methyl acrylate, ethyl acrylate, n-propyl acrylate, iso-propyl acrylate, n-butyl acrylate, iso-butyl acrylate, tert-butyl acrylate, n-amyl acrylate, n-hexyl acrylate, 2-ethylhexyl acrylate, n-octyl acrylate, n-nonyl acrylate, cyclohexylacrylate, benzyl acrylate, dimethylphosphate ethylacrylate, diethylphosphate ethylacrylate, dibutylphosphate ethylacrylate, and 2-benzoyloxy ethylacrylate, methacrylic polymerizable monomers such as methyl methacrylate, ethyl methacrylate, n-propyl methacrylate, iso-propyl methacrylate, n-butyl methacrylate, iso-butyl methacrylate, tert-butyl methacrylate, n-amyl methacrylate, n-hexyl methacrylate, 2-ethylhexyl methacrylate, n-octyl methacrylate, n-nonyl methacrylate, diethylphosphate ethylmethacrylate, and dibutylphosphate ethylmethacrylate, polymerizable vinyl monomers including methylene aliphatic monocarboxylic esters, vinylesters such as vinyl acetate, vinyl propionate, vinyl benzoate, vinyl butyrate, vinyl benzoate, and vinyl formate, vinyl ethers such as vinyl methyl ether, vinyl ethyl ether, and vinyl isobutyl ether, vinyl ketones such as vinyl methyl ketone, vinyl hexyl ketone, and vinyl isopropyl ketone; resin particulates produced by adding various additives such as polar group polymers and colorants to the above described monomers; particulates including paraffin wax, polyolefin wax, Fisher-Tropsch wax, amide wax, higher fatty acids, ester wax and derivatives thereof or graft and block compounds thereof; clay minerals such as kaolinite, bentonite, talc, and mica; metal oxides such as alumina and titanium oxide; insoluble inorganic salts such as silica gel, hydroxyapatite, and calcium phosphate gel; black pigments such as carbon black, copper oxide, manganese dioxide, aniline black, activated carbon, nonmagnetic ferrite, and magnetite; yellow pigments such as chrome yellow, zinc yellow, yellow iron oxide, cadmium yellow, mineral fast yellow, nickel titanium yellow, navels yellow, naphtol yellow S, Hansa yellow G, Hansa Yellow 10G, benzidine yellow G, benzidine yellow GR, quinoline yellow lake, permanent yellow NCG, and tartrazine lake; orange pigments such as red chrome yellow, molybdenum orange, permanent orange GTR, pyrazolone orange, vulkan orange, benzidine orange G, indanthrene brilliant orange RK, and indanthrene brilliant orange GK; red pigments such as red iron oxide, cadmium red lead, mercury sulfide, cadmium, permanent red 4R, lithol red, pyrazolone red, watching red, calcium salt, lake red C, lake red D, brilliant carmine 6B, brilliant carmine 3B, eosin lake, rhodamine lake B, and alizarin lake; blue pigments such as iron blue, cobalt blue, alkali blue lake, victoria blue lake, copper phthalocyanine blue, metal-free phthalocyanine blue, partly chlorinated phthalocyanine blue compound, fast sky blue, and indanthrene blue BC; purple pigments such as manganese purple, fast violet B, and methyl violet lake; green pigments such as chromium oxide, chrome green, pigment green B, malachite green lake, and final yellow green G; white pigments such as zinc oxide, titanium oxide, and zinc sulfide; extender pigments such as barytes, barium carbonate, clay, white carbon, talc, and alumina white. Although a shape of the core can appropriately varies depending on its intended use, it is preferable that a particle whose particle size is within a range of 0.1 µm to 1.0 mm is used for example. In addition, when the construct is used as a capsule toner for electrophotography, a particle may be selected so that its particle size becomes within a range of 3.0 µm to 10 µm.

### <Making of encapsulated particle>

The method for making a encapsulated particle, subsequently also designated as "construct", according to the present invention comprises a step of immobilizing a PHA synthetase onto a base material and a step of reacting the immobilized PHA synthetase with 3-hydroxyacyl CoA to synthesize PHA.

As a method for immobilizing the PHA synthetase onto the base material, one can choose and use any conventional method for immobilizing enzymes as long as the method would not affect the activity of the synthetase and is applicable to the intended base material. For example, covalent bonding, ionic adsorption, a hydrophobic adsorption, physical adsorption, affinity adsorption, crosslinking, lattice inclusion can be utilized, In particular, immobilization using ionic adsorption or hydrophobic adsorption is simple for use.

An enzyme protein such as PHA synthetase is a polypeptide made with a plurality of amino acids bonded together. The enzyme protein exhibits properties as an ionic adsorbate due to constituent amino acids having free ionic groups such as lysine, histidine, arginine, aspartic acid, glutamic acid. The enzyme protein also exhibits properties as a hydrophobic adsorbate due to the constituent amino acids having free hydrophobic groups such as alanine, valine, leucine, isoleucine, methionine, tryptophan, phenylalanine, and proline and because protein is organic polymer. Therefore, the PHA synthetase can be adsorbed by any solid surfaces having ionic and/or hydrophobic properties in various degrees.

To immobilize PHA synthetase using mainly ion adsorption, one can use a base material having ionic functional groups on the surface. Such a base material includes clay minerals such as kaolinite, bentonite, talc, and mica; metal oxides such as alumina and titanium oxide; and insoluble inorganic salts such as silica gel, hydroxyapatite, and calcium phosphate gel. In addition, inorganic pigment made with the above-described substance as the principal constituent, a polymer having ionic functional groups such as ion exchange resins, chitosan, and polyamino polystyrene can be used as an ion adsorptive base material.

To immobilize PHA synthetase by mainly hydrophobic adsorption, one can use a base (core) material having a nonpolar surface, including many polymers not having ionic functional groups or having hydrophobic functional groups on the surface, such as styrenic polymers, acrylic polymers, methacrylic polymers, vinyl esters, and vinyl polymers. Also, organic pigments such as azo pigments having a plurality of aromatic rings, phthalocyanine pigments and anthraquinone pigments having condensed polycyclic structure, and carbon black etc. show hydrophobic adsorptivities.

To immobilize PHA synthetase by ionic or hydrophobic adsorption, the above described synthetase and the core material are mixed in a certain reaction solution. In this case, it is desirable to shake the reaction vessel or stir the reaction solution with a suitable strength so that the enzyme may be uniformly adsorbed to the core surface.

Polarity of surface charge, charge amount, and hydrophobicity of the base (core) material and the PHA synthetase will vary depending on pH, a salt concentration, and a temperature of the reaction solution. Thus, it is desirable to adjust the solution in accordance with the properties of the core within an enzymatically acceptable range. For example, if adsorption is ionic nature, lower salt concentration will increase the charge involved in adsorption of the PHA synthetase to the base (core) material. pH change may increase the amount of opposite charges of the core or base material and the PHA synthetase. If the adsorption is basically hydrophobic nature, higher salt concentration will increase hydrophobicity of the base material (core) and PHA synthetase. In addition, it is possible to determine solution conditions suitable for adsorption by investigating the charge condition and hydrophobicity of the base material (core) and PHA synthetase performing electrophoresis or measuring wetting angle. Further, the conditions can be determined by direct measurement of adsorption between base material (core) and PHA synthetase. The measurement of the adsorption amount is carried out, for example, by adding a PHA synthetase solution of a known concentration to a dispersion of the core to allow adsorption in the resultant mixture, and then measuring the concentration of free PHA synthetase in the solution to determine the adsorbed enzyme amount by subtraction.

If it is difficult to immobilize the enzyme to the base material by the ion adsorption method or hydrophobic adsorption method, one may use a covalent bonding method knowing complicated operations and possibility of enzyme inactivation. Some methods for such immobilization will be listed below by way of example: the enzyme is bonded by diazo-coupling to a solid material having diazo group converted from aromatic amino group; a peptide bond is formed between the enzyme and a base material having a carboxyl group or amino group; alkylation using halogen group of the base material and amino group etc. of the enzyme; a polysaccharide base material activated by cyanogen bromide is reacted with an amino group of the enzyme; crosslinking between amino group of the base material and amino group of the enzyme; a base material having a carboxyl group or amino group is reacted with the enzyme in the presence of a compound having aldehyde or ketone group and an isocyanide compound; and exchange reaction between disulfide group of the base material and the thiol group of the enzyme.

Alternatively, the enzyme may be attached to the base material through affinity adsorption. Affinity adsorption is biological adsorption between a biopolymer and a substance having specific affinity to the biopolymer called ligand, e.g., an enzyme and its substrate, an antibody and its antigen, a receptor and its signaling molecule such as acetylcholine, and mRNA and tRNA for example. Generally, to immobilize an enzyme by affinity adsorption, a substrate, a reaction product, a competitive inhibitor, a coenzyme, an allosteric effecter, etc. of the enzyme is bonded to a solid as a ligand, then the enzyme is applied to this solid. In the present invention, if PHA synthetase is immobilized using 3-hydroxyacyl CoA as a ligand, the active site of the PHA synthetase for synthesizing PHA is blocked by the ligand. Thus, to maintain PHA synthesizing activity, the PHA synthetase may be fused to another biopolymer, and then a ligand of the fused biopolymer is employed for affinity adsorption. PHA synthetase may be fused to the biopolymer by genetic engineering procedure, or the biopolymer may be chemically bonded to the PHA synthetase. Although any biopolymers can be used as long as its ligand is readily available and is easily bonded to the base material (core), the biopolymer is preferably a protein when the fusion product is expressed by genetic recombination. For example, a PHA synthetase fused to glutathione S-transferase (GST) is produced by using an *Escherichia coli* transformant in which the PHA synthetase gene is linked to the GST gene for expression, and the fusion protein can adsorb to Sepharose to which glutathione, a ligand of GST, has been bonded.

Alternatively, PHA synthetase can be immobilized to a surface of the base material through binding between the base material and a peptide portion having a binding ability to the base material and being fused to the PHA synthetase.

The amino acid sequence having a binding ability to the base material can be determined by screening a random peptide library for example. Specifically, it is preferably used a phage display peptide library constructed to link random synthetic genes to a gene encoding a surface protein gene of M13 phage (for example, gene III) to be display at the N-terminus of the coat protein. Screening process for the amino acid sequence that binds to the base material is as follows. The phage display peptide library is added to the base material or to at least one constituent of the base material to bring the phages into contact with the base material, then the bound phages are separated from the not bound phages. Phages bound to the base material are eluted with an acid etc. and is neutralized with a buffer solution, then the phages are infected to *Escherichia coli* for phage proliferation. After repeating the screening process more than once, clones having binding ability to the base material are enriched. Then, to isolate single clones, colonies of *E*. *coli* infected with such clones are formed on a culture plate, and each colony is cultured in a liquid culture medium, and the phages in the culture medium is purified by polyethylene glycol precipitation etc. Consequently, the peptide structure is known by analyzing the base sequence.

Such an amino acid sequence having binding capacity for the base material, obtained as above, is utilized fusing this amino acid sequence to the PHA synthetase by ordinary genetic engineering process. Such a binding peptide can be linked to the N-terminus or C-terminus of the PHA synthetase for expression. In this case, an appropriate spacer sequence can be inserted for expression. Such a spacer sequence has preferably about 3 to 400 amino acids, and the spacer sequence may include any amino acids. Most preferably, the spacer sequence will not prevent the PHA synthetase activity or the binding to the base materials.

The immobilized enzyme prepared as above may be used as it is, or may be lyophilized before use.

The base material is a core of the capsule construct, and the amount of PHA synthetase to be immobilized to the base material may be within a range of 10 to 1,000 units (U) per gram of base material, and preferably 50 to 500 units (U) per gram of base material, where the amount of enzyme that releases 1 µmol of CoA within one minute is defined as one unit (U).

When the above described immobilized enzyme is reacted in an aqueous reaction solution containing 3-hydroxyacyl CoA, it synthesizes PHA on the base material to form a construct in which the base material is coated with the PHA on the surface. The aqueous reaction solution should be prepared as a reaction system suitable for PHA synthetase activity. For example, pH is adjusted with a buffer solution to from 5.5 to 9.0 and preferably from 7.0 to 8.5. However, a possibility of setting the solution conditions out of the above described range is not eliminated depending on an optimum pH of the PHA synthetase to be used and on the pH stability thereof. Any buffer solution can be used according to the desired pH range as long as it does not affect the PHA synthetase activity. For example, one can use buffer solutions commonly used for biochemical reactions such as acetate buffer, sodium phosphate buffer, potassium phosphate buffer, 3-(N-morpholino)propanesulfonic acid (MOPS) buffer, N-tris(hydroxymethyl)methyl-3-aminopropanesulfonic acid (TAPS) buffer, Tris-HCl buffer, glycine buffer, and 2-(cyclohexylamino)ethanesulfonic acid (CHES) buffer. The concentration of the buffer solution is not particularly limited to a certain range as long as the PHA synthetase to be used can exhibit its activity, but generally it is from 5.0 mM to 1.0 M, preferably from 0.1 to 0.2 M. The reaction temperature is appropriately set according to characteristics of the PHA synthetase to be used, but generally it may be within a range of 4°C to 50°C and preferably within a range of 20°C to 40°C, but not limited thereto according to the optimum temperature and heat resistance of the PHA synthetase to be used. The reaction time depends on stability of the PHA synthetase to be used, but generally, it may be within a range of one minute to 24 hours and preferably within a range of 30 minutes to 3 hours. The concentration of 3-hydroxyacyl CoA in the reaction solution is appropriately set within a certain range in which the PHA synthetase to be used can be exhibit it activity, but the concentration may be generally set within a range of 0.1 mM to 1.0 M and preferably within a range of 0.2 mM to 0.2 M. When the 3-hydroxyacyl CoA concentration in the reaction solution is high, it is preferable to use a buffer solution of a higher concentration since pH of the reaction system tends to be lowered.

In the above described process, it is possible to change the monomer unit composition of the coating PHA in a radial direction with a particulate construct by changing the composition of 3-hydroxyacyl CoA (e.g., species and/or concentration) in the aqueous reaction solution with time.

one example of such a construct of which monomer unit composition is varied is such a construct that the base material is coated with one PHA layer of which composition is continuously changed to form a composition gradient in a radial direction. Such a construct may be produced, for example, by adding a different 3-hydroxyacyl CoA composition to the reaction solution during PHA synthesis.

Another example is such a construct that the base material is coated with a plurality of PHA layers of which compositions are different from one another. Such a construct may be produced, for example, by carrying out the reaction with one 3-hydroxyacyl CoA composition and then collecting the construct under making by centrifugation etc. from the reaction solution and transferring it to another reaction solution containing different 3-hydroxyacyl CoA composition for reaction.

The construct obtained by the above described reaction is subjected to a washing process as required. The method for washing is not particularly limited as long as the construct would not be affected undesirably in view of production purpose of the construct. The construct is a capsule construct comprised of a core of the base material and a shell of PHA, and unwanted constituents contained in the reaction solution may be removed by centrifuging the reaction solution and removing the supernatant therefrom, for example. Further washing nay be carried out by adding to the pellet a washing agent such as water, a buffer solution, and methanol that would not dissolve the PHA, and by repeating centrifugation. Also, other operations such as filtration may be used instead of centrifugation. Further, the construct can be subjected to a drying process as required. Furthermore, the construct may be subjected to various secondary processing or chemical modifications before use.

For example, a construct provided with more useful functions and characteristics can be obtained by performing chemical modification on the PHA coating.

For example, introducing a graft chain to the PHA, one can obtain a construct in which at least part of the base material is coated with the PHA having various characteristics owing to the graft chain. In addition, it is possible to control mechanical strength, chemical resistance, heat resistance, etc. of the construct by cross-linking the PHA.

Although the method of chemical modification is not particularly limited as long as an object of obtaining the desired function and construct is achieved, it is preferably used a method to synthesize PHA having reactive functional groups on the side group and to utilize the reactivity of the functional group for chemical modification.

Although a kind of the above described reactive functional groups is not particularly limited as long as an object of obtaining the desired function and construct is achieved, the reactive functional groups include the above described epoxy group for example. In PHA having the epoxy groups on the side chains, chemical conversion can be performed as in the case of a common polymer having epoxy groups. Specifically, it is possible to convert epoxy to hydroxyl group or to introduce sulfone group. In addition, it is possible to add a compound having thiol or amine. For example, a compound having a reactive functional group at an end thereof, specifically a compound having at an end thereof an amino group having high reactivity with epoxy can be added and reacted to form a graft polymer.

The compounds having an amino group at the end include amino modified polymers such as polyvinyl amine, polyethylene imine, and amino modified polysiloxane (amino modified silicone oil). Among these, commercially available modified silicone oil may be used as the amino modified polysiloxane. Such amino modified polysiloxane can be also synthesized by a method described in J. Amer. Chem. Soc., 78, 2278 (1956). Improvement in properties such as the heat resistance is expected due to the addition of graft chains to the polymer.

Another example of chemical conversion of polymers having epoxy groups is the crosslinking reaction using diamine compounds such as hexamethylenediamine, succinic anhydride, 2-ethyl-4-methylimidazole, or electron beam irradiation. For example, the reaction of PHA having epoxy group on the side chain with hexamethylenediamine proceeds as shown below to form a crosslinked polymer.

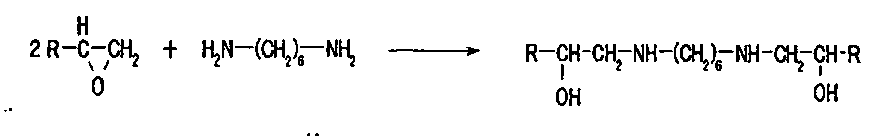

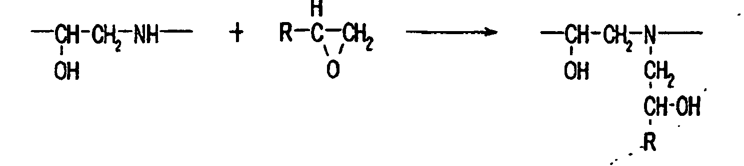

To confirm that the base material is coated with PHA in the obtained construct, one can use a combination of composition analysis by gas chromatography etc. and morphological observation by electron microscopy etc.; or a method for determining the structure from the mass spectra of constituent layers by means of time-of-flight secondary ion mass spectroscopy (TOF-SIMS) and ion sputtering technology. It is also possible to use a method newly developed by the inventors as a more simple and direct confirmation method in which Nile blue A staining is combined with a fluorescence microscopic observation. As a result of intensive study on the method for simply determining the *in vitro* PHA synthesis by PHA synthetase, the inventors have found that the Nile blue A that fluoresces specifically when it binds to PHA and is applicable to calling *in vivo* microbial production of PHA (Appl. Environ. Microbiol., 44, 238-241 (1982) can also be used for calling the *in vitro* PHA synthesis if an appropriate method and conditions are determined. That is, in this method, a Nile blue A solution of a certain concentration is filtered and mixed with a reaction solution containing PHA, and when the sample is irradiated with an excitation light of a certain wavelength and observed under a fluorescence microscope, fluorescence is observed only from the synthesized PHA, allowing simple calling of *in vitro* PHA synthesis. PHA coating on the surface of the base material can be directly observed and evaluated by applying this method to the manufacture of the construct of the present invention, as long as the used base material itself does not fluoresce under the above-described conditions.

The composition distribution of PHA coating the base material along inside-to-outside or vertical direction can be evaluated by combining the ion sputtering technology with TOF-SIMS.

### <Use of construct>

One of features of the present invention is that it enables production of constructs of which production has been difficult by conventional organic synthetic chemistry. Therefore, it is possible to obtain a construct having excellent properties that could not have been conferred to the capsule constructs or laminated constructs manufactured by conventional organic synthetic chemistry. For example, it enables utilization of new polymer compounds or it can provide new functions or construct, which have been difficult by the conventional organic synthetic chemistry. More specifically, by utilizing highly strict molecule-recognition ability or stereo selectivity specific to a biological catalyst derived from a living organism, it is possible to produce a new functional polymer compound not obtainable by the conventional synthetic chemistry, or a capsule coated with a polymer compound having extremely high chirality through a significantly simple process.

An exemplary application of the above described constructs is a highly functionalized capsule toner for electrophotography. As described above, the capsule toner for electrophotography has some problems that the production process is significantly complicated and a large amount of solvents and surfactants are used in the production process. In accordance with a method of the present invention, such problems are solved and the capsule toner can be produced by a simple method. In addition, the thickness of the shell and the monomer unit composition, for example, can be relatively easily controlled. Japanese Patent Application Laid-Open No. 8-286416 teaches that inclusion of polar polymers such as polyester in the shell of a capsule toner improves image durability and provides charging uniformity and stability. Such advantages are expected for the shell of PHA of a capsule toner produced by the method of the present invention. Further, in the method of the present invention, PHA having various functional groups can be used as a shell. Thus, it is possible to control physical properties of the toner surface by these functional groups or to confer new functionalities to the toner. This method uses little or no organic solvents or surfactants except for the production process of the core and the reaction conditions are extremely mild, so that it is possible to reduce the environmental loads in making to a great extent.

According to the present invention, the construct, usage thereof or the method for making thereof is not limited to those described above.

### Examples

Now the present invention will be described in more detail with reference to Examples. Although each of the examples described below is the best mode of the present invention, the technical scope of the present invention is not limited to these examples. "%" or "part" herein is based on the weight unless otherwise specified.

### Reference Example 1 Construction of transformant having PHA synthetase production capacity

Strain YN2 was cultured in 100 ml of LB medium (1% polypeptone (NIPPON SEIYAKU CO., LTD.), 0.5% yeast extract (Difco), 0.5% sodium chloride, pH 7.4) overnight at 30°C, then the chromosomal DNA was isolated by the method of Marmar et al. The obtained DNA was completely digested by a restriction enzyme HindIII. A vector pUC18 was also cut by HindIII. After terminal dephosphorylation (Molecular Cloning, 1, 572, (1989); Cold Spring Harbor Laboratory Press), complete HindIII digestion fragments of the chromosomal DNA were ligated to the HindIII cleavage site of the vector (a cloning site) using a DNA ligation kit Ver. II (TAKARA SHUZO CO., LTD.). With these plasmid vectors containing chromosomal DNA fragments, *Escherichia coli* HB 101 was transformed to construct a DNA library of strain YN2. Next, to select DNA fragments covering the PHA synthetase gene of strain YN2, a probe for colony hybridization was prepared by synthesizing an oligonucleotide comprised of the base sequences of SEQ ID NO: 2 and SEQ ID NO: 3 (Amersham Pharmacia Biotech). Then PCR was carried out by using the chromosomal DNA as a template. The PCR-amplified DNA fragments were used as a probe. Labeling of the probe was conducted by employing a commercially available labeling enzyme Alk Phos Direct (Amersham Pharmacia Biotech). Using the obtained labeled probe for colony hybridization, a clone carrying the recombinant plasmid containing the PHA synthetase gene was selected from the chromosomal DNA library of YN2. The plasmid was recovered from the selected clone by the alkali process to give DNA fragment including the PHA synthetase gene. This gene fragment was inserted into a vector pBBR122 having a wide-host-replication range (Mo Bi Tec) not belonging to any of IncP, IncQ, or IncW incompatibility group. When *Pseudomonas cichorii* YN2ml (a PHA synthesis negative strain) was transformed with this recombinant plasmid by electroporation, the PHA synthesizing capacity of the YN2ml strain was recovered to show complementarity. Consequently, it was confirmed that the selected gene fragment contains PHA synthetase gene region which can be translated into PHA synthetase within *Pseudomonas cichorii* YN2ml.

The base sequence of this DNA fragment was determined by the Sanger method. As a result, it was shown that there are base sequences represented by SEQ ID NO: 4 and SEQ ID NO: 5 each encoding a polypeptide in the determined base sequence. With respect to these PHA synthetase genes, PCR was carried out by using the chromosomal DNA as a template to produce the complete PHA synthetase gene. More specifically, an upstream primer (SEQ ID NO: 6) and a downstream primer (SEQ ID NO: 7) corresponding to the PHA synthetase gene of SEQ ID NO:4, and an upstream primer (SEQ ID NO: 8) and a downstream primer (SEQ ID NO: 9) corresponding to the PHA synthetase gene of SEQ ID NO: 5 were synthesized respectively (Amersham Pharmacia Biotech).

Using these primers, PCR was carried out for each of the base sequences shown by SEQ ID NO: 4 and SEQ ID NO: 5, then a full length of PHA synthetase gene was amplified (an LA-PCR kit; TAKARA SHUZO CO., LTD.). Next, the obtained PCR amplified fragment and an expression vector pTrc99A were digested by the restriction enzyme HindIII and dephosphorylated (Molecular Cloning, vol.1, p.572, (1989); Cold Spring Harbor Laboratory Press), then the DNA fragment including a full length PHA synthetase gene excluding unnecessary base sequences at both terminuses was linked to a restriction site of.the expression vector pTrc99A by using a DNA ligation kit Ver. II (TAKARA SHUZO CO., LTD.).

An *E. coli* strain (*Escherichia coli* HB101: TAKARA SHUZO) was transformed with each of the obtained recombinant plasmids by the calcium chloride method. The obtained recombinants were cultured and the recombinant plasmids were amplified, then the recombinant plasmids were respectively recovered. The recombinant plasmid having a DNA of SEQ ID NO: 4 was designated as pYN2-C1, and the recombinant plasmid having a DNA of SEQ ID NO: 5 was designated as pYN2-C2. An *E. coli* strain (*Escherichia coli* HB101fB fadB deletion strain) was transformed with pYN2-C1 and pYN2-C2 respectively by the calcium chloride method to obtain recombinant *E. coli* strains having respective recombinant plasmids, i.e., a pYN2-C1 recombinant strain and a pYN2-C2 recombinant strain.

### Reference Example 2 Production of PHA synthetase (1)

For the pYN2-C1. an upstream primer (SEQ ID NO: 10) and a downstream primer (SEQ ID NO: 11) were designed and synthesized respectively (Amersham Pharmacia Biotech). PCR was carried out with LA-PCR kit (TAKARA SHUZO CO., LTD.) using these primers and template pYN2-C1 to synthesize a full length PHA synthetase gene having a BamHI restriction site upstream and a XhoI restriction site downstream.

Similarly, for pYN2-C2, an upstream primer (SEQ ID NO: 12) and a downstream primer (SEQ ID NO: 13) were designed and synthesized respectively (Amersham Pharmacia Biotech). PCR was carried out with an LA-PCR kit (TAKARA SHUZO CO., LTD.) using these primers and the template pYN2-C2 to amplify the full length PHA synthetase gene having a BamHI restriction site upstream and a XhoI restriction site downstream.

Respective purified PCR amplification products were digested by BamHI and XhoI, then inserted into the corresponding restriction sites of plasmid pGEX-6P-1 (Amersham Pharmacia Biotech). An *E. coli* strain JM109 was transformed with these vectors, and consequently a strain expressing PHA synthetase was obtained. For confirmation, the plasmid DNA was prepared by Miniprep (Wizard Minipreps DNA Purification Systems, PROMEGA) in a large amount and digested by BamHI and XhoI, and the resulting DNA fragment was identified. The PHS synthetase was expressed and purified as follows: The obtained strain was pre-cultured in 10 ml of LB-Amp medium overnight, and then an 0.1 ml culture was transferred to 10 ml of LB-Amp medium and cultured at 37°C, 170 rpm for 3 hours with shaking. Then, IPTG was added to the culture to a concentration of 1 mM, then the culture was continued for 4 to 12 hours at 37°C.

The *E. coli* cells induced with IPTG were collected (8,000×g, 2 min., 4°C) and resuspended in a 1/10 volume of phosphate buffer physiological saline (PBS; 8 g NaCl, 1.44 g Na₂HPO₄, 0.24 g KH₂PO₄, 0.2 g, KC1, 1,000 ml purified water) at 4°C. The cells were disrupted by freeze and thawing and sonication, and subjected to centrifugation (8,000×g, 10 min., 4°C) to remove solid impurities. Confirming that the aimed recombinant protein was present in the supernatant by SDS-PAGE, the induced and expressed GST fusion protein was purified by using Glutathione Sepharose 4B (Amersham Pharmacia Biotech). The Glutathione Sepharose was previously treated to avoid nonspecific adsorption, that is, the Glutathione Sepharose was washed with an equivalent amount of PBS for three times (8,000×g, 1 min., 4°C), and then an equivalent amount of 4% bovine serum albumin PBS was added thereto at 4°C for one hour. After that, the Sepharose was washed with an equivalent amount of PBS twice, and re-suspended in an 1/2 amount of PBS. The pre-treated 40 µl of Glutathione Sepharose was added to 1 ml of the above cell free extract, and gently stirred at 4°C to adsorb fusion protein GST-YN2-C1 and GST-YN2-C2 onto Glutathione Sepharose respectively. After centrifugation (8,000×g, 1 min., 4°C) to collect the Glutathione Sepharose, it was washed with 400 µl of PBS for three times. After the washing, 10 mM of glutathione was added thereto and stirred for one hour at 4°C to elute the adsorbed fusion protein. After centrifugation (8,000×g, 2 min., 4°C), the supernatant was recovered and dialyzed against PBS to purify the GST fusion protein. Single band was recognized by SDS-PAGE.

Then 500 µg of each GST fusion protein was digested by PreScission protease (Amersham Pharmacia Biotech, 5U), the protease and the GST were removed therefrom by passing through Glutathione Sepharose. The flow-through fraction was further loaded to Sephadex G200 column equilibrated with PBS, then expression proteins YN2-C1 and YN2-C2 were obtained as final purified products. By SDS-PAGE, single bands (60.8 kDa and 61.5 kDa, respectively) were confirmed.

The above described enzymes were concentrated with a bioliquid concentrating agent (Mizubutorikun AB-1100, Atto Corp.) to obtain 10U/ml of purified enzyme solutions.

The enzyme activity was measured by the above-described method. The protein concentration of the sample was determined by using a micro BCA protein assay reagent kit (Pierce Chemical Co.). The results are shown in Table 1.

**Table 1**

| | Specific activity |
|---|---|
| pYN2-C1 | 4.1 U/mg protein |
| pYN2-C2 | 3.6 U/mg protein |

### Reference Example 3 Production of PHA synthetase (2)

Each of *E coli* strains P91, H45, YN2, and P161 was inoculated in 200 ml of an M9 medium containing 0.5% of yeast extract (Difco) and 0.1% of octanoic acid, and incubated at 30°C, with shaking at 125 strokes/min. After 24 hours, the cells were harvested by centrifugation (10,000×g, 4°C, for 10 min.), then the cells were re-suspended in 200 ml of 0.1M Tris-HC1 buffer (pH 8.0) and further subjected to centrifugation for washing. The cells were re-suspended in 2.0 ml of 0.1M Tris-HC1 buffer (pH 8.0) and disrupted by an ultrasonic homogenizer and then centrifuged (12, 000×g, 4°C, for 10 min.) to collect the supernatant to obtain the crude enzyme.

Each crude enzyme activity was measured by the above described method, and the result is shown in Table 2.

**Table 2**

| | Activity |
|---|---|
| P91 | 0.1 U/ml |
| H45 | 0.2 U/ml |
| YN2 | 0.4 U/ml |
| P161 | 0.2 U/ml |

### Example 1 Preparation of capsule construct (1)

One part of alumina particles (particle size: 0,12 µm to 135 µm) and 39 parts of PBS were added to 10 parts of a PHA synthetase solution (10U/ml) derived from a pYN2-C1 recombinant strain, and the mixture was reacted with mild shaking for 30 minutes at 30°C to adsorb PHA synthetase onto the surface of the alumina particles. Then the mixture was centrifuged at 10,000×g, 4°C, for 10 min, and the precipitate was suspended in a PBS solution and centrifuged again (10,000×g, 4°C, for 10 min.) to obtain the immobilized enzyme.

The immobilized enzyme was suspended in 48 parts of a 0.1M phosphate buffer solution (pH 7.0), to which one part of (R)-3-hydroxyoctanoyl CoA (prepared according to the method described in Eur. J. Biochem., 250, 432-439 (1997)) and 0.1 part of bovine serum albumin (Sigma) were added, and incubated with mild shaking for 2 hours at 30°C.

A 10 µl aliquot of the above reaction solution was put on a slide glass, to which 10 µl of a 1% solution of Nile blue A in water was added. These solutions were mixed on the slide glass, covered with a cover glass, and observed under a fluorescence microscope (330 to 380 nm excitation filter, 420 nm long pass absorption filter, Nikon Corp.). As a result, fluorescence from the surface of the alumina particles was observed to confirm that the alumina particles were coated with PHA on the surface.

As a control, one part of alumina particles was added to 49 parts of a 0.1M phosphate buffer (pH 7.0), and incubated with mild shaking for 2.5 hours at 30°C. Observation under a fluorescence microscope after Nile blue A staining showed the surface of the alumina particle did not fluoresce at all.

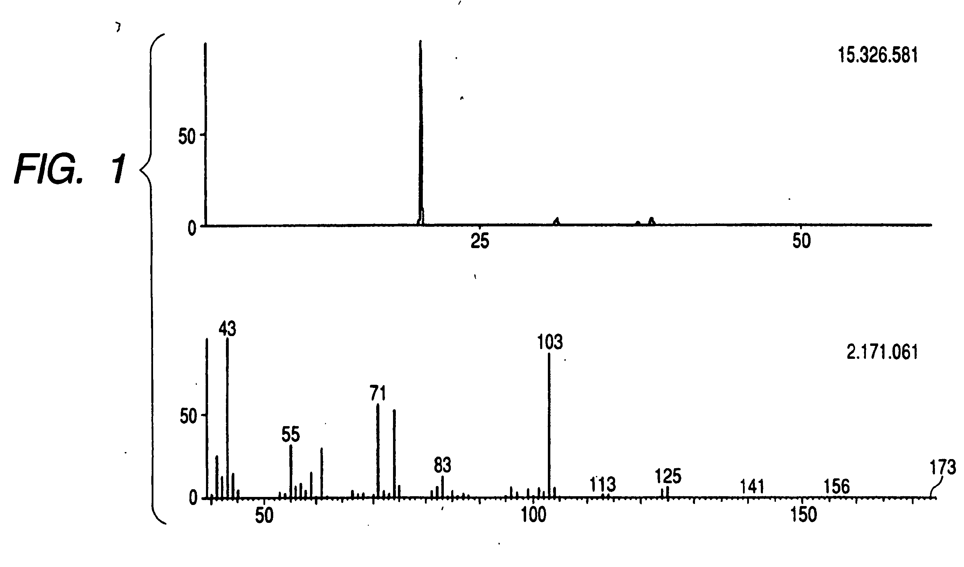

Further, part of the above coated particles was collected by centrifugation (10,000×g, 4°C, for 10 min.), dried in vacuum, suspended in chloroform, and stirred for 20 hours at 60°C to extract the PHA coating. The extract was filtered through a membrane filter of 0.45 µm pore size and concentrated under a reduced pressure by using a rotary evaporator. Then the extract was subjected to methanolysis by a conventional method and analyzed by gas chromatography-mass spectroscopy (GC-MS, Shimadzu QP-5050, an EI method) to identify the methyl-esterified PHA monomer unit. As a result, it was confirmed that the PHA was made from 3-hydroxyoctanoic acid monomer unit as shown in FIG. 1.

In addition, the molecular weight of the above PHA was determined by gel permeation chromatography (GPC; TOSOH HLC-8020, column; Polymer Laboratories Ltd. PL gel MIXED-C (5 µm), solvent; chloroform, column temperature; 40°C, polystyrene base conversion). As a result, Mn=21,000, Mw=40,000.

### Example 2 Preparation of capsule construct (2)

One part of alumina particles (particle size: 0.12 µm to 135 µm) and 39 parts of PBS were added to 10 parts of a PHA synthetase solution (10U/ml) derived from a pYN2-C2 recombinant strain, and the mixture was reacted with mild shaking for 30 minutes at 30°C to adsorb PHA synthetase onto the surface of the alumina particles. Then the mixture was centrifuged at 10,000×g, 4°C, for 10 min, and the precipitate was suspended in a PBS solution and centrifuged again (10,000×g, 4°C, for 10 min.) to obtain the immobilized enzyme.

The immobilized enzyme was suspended in 48 parts of a 0.1M phosphate buffer solution (pH 7.0), to which one part of (R)-3-hydroxyoctanoyl CoA (prepared according to the method described in Eur. J. Biochem., 250, 432-439 (1997)) and 0.1 part of bovine serum albumin (Sigma) were added, and incubated with mild shaking for 2 hours at 30°C.

A 10 µl aliquot of the above reaction solution was put on a slide glass, to which 10 µl of a 1% solution of Nile blue A in water was added. These solutions were mixed on the slide glass, covered with a cover glass, and observed under a fluorescence microscope (330 to 380 nm excitation filter, 420 nm long pass absorption filter, Nikon Corp.). As a result, fluorescence from the surface of the alumina particles was observed to confirm that the alumina particles were coated with PHA on the surface.

Further, part of the above coated particles was collected by centrifugation (10,000×g, 4°C, for 10 min.), dried in vacuum, suspended in chloroform, and stirred for 20 hours at 60°C to extract the PHA coating. The extract was filtered through a membrane filter of 0.45 µm pore size and concentrated under a reduced pressure by using a rotary evaporator. Then the extract was subjected to methanolysis by a conventional method and analyzed by gas chromatography-mass spectroscopy (GC-MS, Shimadzu QP-5050, an EI method) to identify the methyl-esterified PHA monomer unit. As a result, as in Example 1, it was confirmed that the PHA was made from 3-hydroxyoctanoic acid monomer unit.

### Example 3 Preparation of capsule construct (3)

One part of alumina particles (particle size: 0.12 µm to 135 µm) was added to 99 parts of crude PHA synthetase preparations derived from strains YN2, H45, P91 and P161 respectively, and each mixture was reacted with mild shaking for 30 minutes at 30°C to adsorb PHA synthetase onto the surface of the alumina particles. Then each mixture was centrifuged at 10,000×g, 4°C, for 10 min, and the precipitate was suspended in a PBS solution and centrifuged again (10,000×g, 4°C, for 10 min.) to obtain the immobilized enzyme.

Each immobilized enzyme was suspended in 48 parts of a 0.1M phosphate buffer solution (pH 7.0), to which one part of (R)-3-hydroxyoctanoyl CoA (prepared according to the method described in Eur. J. Biochem., 250, 432-439 (1997)) and 0.1 part of bovine serum albumin (Sigma) were added, and incubated with mild shaking for 2 hours at 30°C.

A 10 µl aliquot of each above reaction solution was put on a slide glass, to which 10 µl of a 1% solution of Nile blue A in water was added. These solutions were mixed on the slide glass, covered with a cover glass, and observed under a fluorescence microscope (330 to 380 nm excitation filter, 420 nm long pass absorption filter, Nikon Corp.). As a result, fluorescence from the surface of the alumina particles was observed to confirm that the alumina particles were coated with PHA on the surface with each immobilized enzyme reaction.

Further, part of the above coated particles was collected by centrifugation (10,000×g, 4°C, for 10 min.), dried in vacuum, suspended in chloroform, and stirred for 20 hours at 60°C to extract the PHA coating. The extract was filtered through a membrane filter of 0.45 µm pore size and concentrated under a reduced pressure by using a rotary evaporator. Then the extract was subjected to methanolysis by a conventional method and analyzed by gas chromatography-mass spectroscopy (GC-MS, Shimadzu QP-5050, an EI method) to identify the methyl-esterified PHA monomer unit. As a result, as in Example 1, it was confirmed that the PHA was made from 3-hydroxyoctanoic acid monomer unit.

### Example 4 Preparation of capsule construct (4)

One part of alumina particles (particle size: 0.12 µm to 135 µm) and 39 parts of PBS were added to 10 parts of a PHA synthetase solution (10U/ml) derived from a pYN2-C1 recombinant strain, and the mixture was reacted with mild shaking for 30 minutes at 30°C to adsorb PHA synthetase onto the surface of the alumina particles. Then the mixture was centrifuged at 10,000×g, 4°C, for 10 min, and the precipitate was suspended in a PBS solution and centrifuged again (10,000×g, 4°C, for 10 min.) to obtain the immobilized enzyme.

The immobilized enzyme was suspended in 48 parts of a 0.1M phosphate buffer solution (pH 7.0), to which one part of (R)-3-hydroxy-5-phenylvaleryl CoA and 0.1 part of bovine serum albumin (Sigma) were added, and incubated with mild shaking for 2 hours at 30°C. The (R)-3-hydroxy-6-phenylvaleryl CoA was prepare from 3-hydroxy-5-phenylvaleric acid according to the method described in Eur. J. Biochem., 250, 432-439 (1997), where first 3-hydroxy-5-phenylvaleriate was prepared by the Reformatsky reaction, and then hydrolyzed to give 3-hydroxy-5-phenylvaleric acid.

A 10 µl aliquot of the above reaction solution was put on a slide glass, to which 10 µl of a 1% solution of Nile blue A in water was added. These solutions were mixed on the slide glass, covered with a cover glass, and observed under a fluorescence microscope (330 to 380 nm excitation filter, 420 nm long pass absorption filter, Nikon Corp.). As a result, fluorescence from the surface of the alumina particles was observed to confirm that the alumina particles were coated with PHA on the surface.

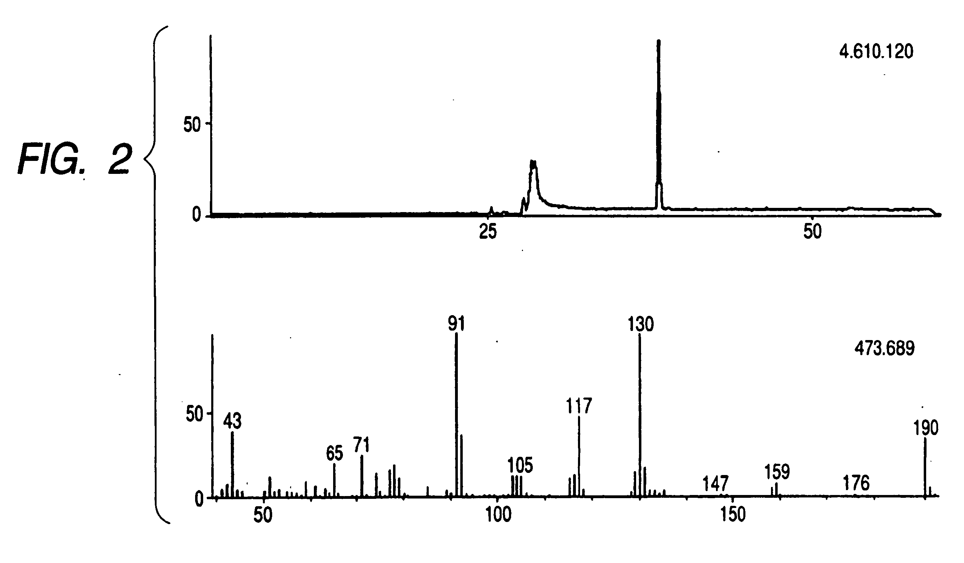

Further, part of the above coated particles was collected by centrifugation (10,000×g, 4°C, for 10 min.), dried in vacuum, suspended in chloroform, and stirred for 20 hours at 60°C to extract the PHA coating. The extract was filtered through a membrane filter of 0.45 µm pore size and concentrated under a reduced pressure by using a rotary evaporator. Then the extract was subjected to methanolysis by a conventional method and analyzed by gas chromatography-mass spectroscopy (GC-MS, Shimadzu QP-5050, an EI method) to identify the methyl-esterified PHA monomer unit. As a result, it was confirmed that the PHA was made from 3-hydroxy-5-phenylvaleric acid monomer unit as shown in FIG. 2.

In addition, the molecular weight of the above PHA was determined by gel permeation chromatography (GPC; TOSOH HLC-8020, column; Polymer Laboratories Ltd. PL gel MIXED-C (5 µm), solvent; chloroform, column temperature; 40°C, polystyrene base conversion) to give a result of Mn=16,000, and Mw=36,000.

### Example 5 Preparation of capsule construct (5)

One part of alumina particles (particle size: 0.12 µm to 135 µm) and 39 parts of PBS were added to 10 parts of a PHA synthetase solution (10U/ml) derived from a pYN2-C1 recombinant strain, and the mixture was reacted with mild shaking for 30 minutes at 30°C to adsorb PHA synthetase onto the surface of the alumina particles. Then the mixture was centrifuged at 10,000×g, 4°C, for 10 min, and the precipitate was suspended in a PBS solution and centrifuged again (10,000×g, 4°C, for 10 min.) to obtain the immobilized enzyme.

The immobilized enzyme was suspended in 48 parts of a 0.1M phosphate buffer solution (pH 7.0), to which one part of (R)-3-hydroxy-5-(4-fluorophenyl)valeryl CoA and 0.1 part of bovine serum albumin (Sigma) were added, and incubated with mild shaking for 2 hours at 30°C. The (R)-3-hydroxy-6-phenylvaleryl CoA was prepared from 3-hydroxy-5-(4-fluorophenyl)valeric acid according to the method described in Eur. J. Biochem., 250, 432-439 (1997), where first 3-hydroxy-5-(4-fluorophenyl)valeriate was prepared by the Reformatsky reaction, and then hydrolyzed to give 3-hydroxy-5-(4-fluorophenyl)valeric acid.

A 10 µl aliquot of the above reaction solution was put on a slide glass, to which 10 µl of a 1% solution of Nile blue A in water was added. These solutions were mixed on the slide glass, covered with a cover glass, and observed under a fluorescence microscope (330 to 380 nm excitation filter, 420 nm long pass absorption filter, Nikon Corp.). As a result, fluorescence from the surface of the alumina particles was observed to confirm that the alumina particles were coated with PHA on the surface.

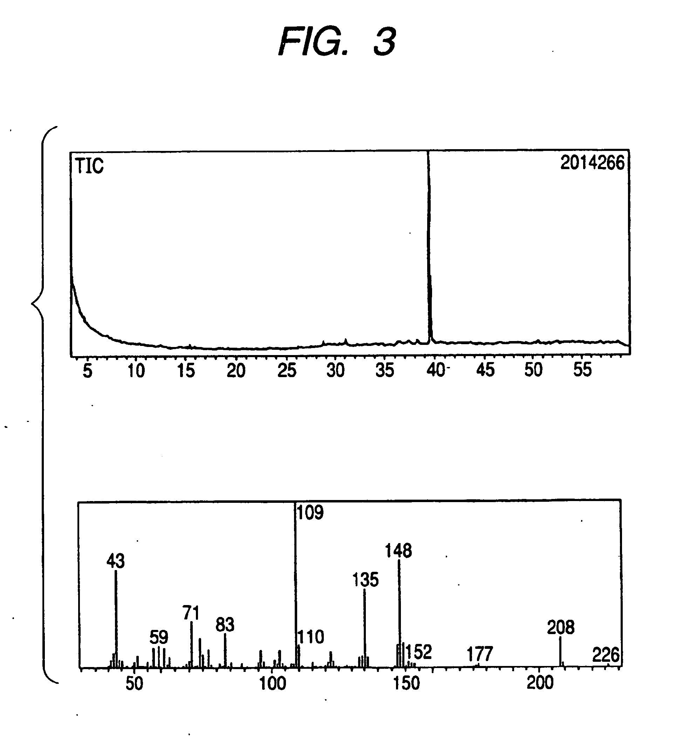

Further, part of the above coated particles was collected by centrifugation (10,000×g, 4°C, for 10 min.), dried in vacuum, suspended in chloroform, and stirred for 20 hours at 60°C to extract the PHA coating. The extract was filtered through a membrane filter of 0.45 µm pore size and concentrated under a reduced pressure by using a rotary evaporator. Then the extract was subjected to methanolysis by a conventional method and analyzed by gas chromatography-mass spectroscopy (GC-MS, Shimadzu QP-5050, an EI method) to identify the methyl-esterified PHA monomer unit. As a result, it was confirmed that the PHA was made from 3-hydroxy-5-(4-fluorophenyl)valeric acid monomer unit as shown in FIG. 3.

### Example 6 Preparation of capsule construct (6)

To 10 parts of a PHA synthetase solution (10U/ml) derived from a pYN2-C1 recombinant strain, one part of alumina particles of which particle size was adjusted by precipitation to an volume average particle size of 1.45 µm and 39 parts of PBS were added and the mixture was reacted with mild shaking for 30 minutes at 30°C to adsorb PHA synthetase onto the surface of the alumina particles. Then the mixture was centrifuged at 10,000×g, 4°C, for 10 min, and the precipitate was suspended in a PBS solution and centrifuged again (10,000×g, 4°C, for 10 min.) to obtain the immobilized enzyme.

The immobilized enzyme was suspended in 48 parts of a 0.1M phosphate buffer solution (pH 7.0), to which one part of (R)-3-hydroxyoctanoyl CoA (prepared according to the method described in Eur. J. Biochem., 250, 432-439 (1997)) and 0.1 part of bovine serum albumin (Sigma) were added, and incubated with mild shaking for 2 hours at 30°C.

A 10 µl aliquot of the above reaction solution was put on a slide glass, to which 10 µl of a 1% solution of Nile blue A in water was added. These solutions were mixed on the slide glass, covered with a cover glass, and observed under a fluorescence microscope (330 to 380 nm excitation filter, 420 nm long pass absorption filter, Nikon Corp.). As a result, fluorescence from the surface of the alumina particles was observed to confirm that the alumina particles were coated with PHA on the surface.

Further, part of the above coated particles was collected by centrifugation (10,000×g, 4°C, for 10 min.) After drying treatment, the mass of the polymer formed on the particle surface was determined by using a time-flight secondary ion mass spectroscope (TOF-SIMS IV, CAMEMA). The obtained mass spectrum showed that the surface of the construct was made of polyhydroxyoctanoate homopolymer. Furthermore, mass spectrum was taken by using TOF-SIMS scraping the construct surface little by little by ion spattering. In this case also, only polyhydroxyoctanoate homopolymer was detected showing that in the capsule construct a hydrophilic inorganic particle is directly coated with hydrophobic polyhydroxyoctanoate homopolymer.

In addition, the molecular weight of the above PHA was determined by gel permeation chromatography (GPC; TOSOH HLC-8020, column; Polymer Laboratories Ltd. PL gel MIXED-C (5 µm), solvent; chloroform, column temperature; 40°C, polystyrene base conversion) to give a result of Mn=23,000, and Mw=42,000.

### Example 7 Preparation of a capsule construct 7

To 10 parts of a PHA synthetase solution (10U/ml) derived from a pYN2-C1 recombinant strain, one part of alumina particles of which particle size was adjusted by precipitation to an volume average particle size of 1.45 µm and 39 parts of PBS were added and the mixture was reacted with mild shaking for 30 minutes at 30°C to adsorb PHA synthetase onto the surface of the alumina particles. Then the mixture was centrifuged at 10,000×g, 4°C, for 10 min, and the precipitate was suspended in a PBS solution and centrifuged again (10,000×g, 4°C, for 10 min.) to obtain the immobilized enzyme.

The immobilized enzyme was suspended in 48 parts of a 0.1M phosphate buffer solution (pH 7.0), to which one part of (R)-3-hydroxypimelyl CoA prepared according to the method described in J. Bacteriol., 182, 2753-2760 (2000) and 0.1 part of bovine serum albumin (Sigma) were added, and incubated with mild shaking for 2 hours at 30°C. Then, to this reaction solution, a 0.1M phosphate buffer solution (pH 7.0) containing one part of (R)-3-hydroxyoctanoyl CoA prepared according to the method described in Eur. J. Biochem., 250, 432-439 (1997)) and 0.1 part of bovine serum albumin was added by using a micro tube pump (TOKYO RIKAKIKAI Co., MP-3N) at a rate of one part per minute, with mild shaking at 30°C. After one and a half hours of incubation, the produced particles were collected by centrifugation (10,000×g, 4°C, for 10 min.). After the supernatant was removed, the particles were suspended in 25 parts of a 0.1M phosphate buffer (pH 7.0) including one part of (R)-3-hydroxyoctanoyl CoA (prepared by a method described in Eur. J. Biochem., 250, 432-439 (1997)) and 0.1 part of bovine serum albumin (Sigma) and incubated with mild shaking for 20 minutes at 30°C.

After reaction, a 10 µl aliquot of the above reaction solution was put on a slide glass, to which 10 µl of a 1% solution of Nile blue A in water was added. These solutions were mixed on the slide glass, covered with a cover glass, and observed under a fluorescence microscope (330 to 380 nm excitation filter, 420 nm long pass absorption filter, Nikon Corp.). As a result, fluorescence from the surface of the alumina particles was observed to confirm that the alumina particles were coated with PHA on the surface.

Further, the capsule constructs were collected by centrifugation (10,000×g, 4°C, for 10 min.) After drying treatment, the mass of the polymer formed on the particle surface was determined by using a time-flight secondary ion mass spectroscope (TOF-SIMS IV, CAMEMA). The obtained mass spectrum showed that the surface of the construct was made of polyhydroxyoctanoate homopolymer. Furthermore, spectrum was taken by using TOF-SIMS scraping the construct surface little by little by ion spattering. First appeared a copolymer of 3-hydroxyactanoic acid and 3-hydroxypimelic acid at a molar ratio of 21:1, and this ratio gradually changed as the scraping proceeded inward the particle, with decreasing 3-hydroxyoctanoic acid and increasing 3-hydroxypimelic acid, and finally a homopolymer of polyhydroxypimelate appeared. This result shows that the capsule construct of this example was constructed in such a manner that a hydrophilic particulate base material was coated with polyhydroxypimelate having hydrophilic functional groups, and the surface of this polyhydroxypimelate was coated with a copolymer of 3-hydroxypimelic acid having a hydrophilic functional group and 3-hydroxyoctanoic acid having a hydrophobic functional group with a gradually increasing ratio of 3-hydroxyoctanoic acid toward the surface, and the outmost surface was coated with polyhydroxyoctanoate homopolymer.

In addition, the molecular weight of the above PHA was determined by gel permeation chromatography (GPC; TOSOH HLC-8020, column; Polymer Laboratories Ltd. PL gel MIXED-C (5 µm), solvent; chloroform, column temperature; 40°C, polystyrene base conversion) to give a result of Mn=21,000, and Mw=40,000.

### Example 8 Evaluation of Example 6 and Example 7

The respective capsule constructs of Example 6 and Example 7, and alumina particles of which particle size had been adjusted uniform by precipitation (the volume average particle size: 1.45 µm) as a comparative example were evaluated. Each of them was added one part to 10 parts of purified water. Each suspension was stored for 30 days at room temperature, with or without vigorous shaking of 5 minutes by using a vortex mixer before storage, and the volume average particle size was measured before and after the storage. The measurement was performed by a laser Doppler type of particle size distribution measuring instrument (UPA-150; a product of Nikkiso). Table 3 shows the result without shaking, and Table 4 shows the result with shaking.

**Table 3**

| | Example 6 | Example 7 | Comparative Example |
|---|---|---|---|
| Volume average particle size (before storage, | 1.58 | 1.68 | 1.45 |
| Volume average particle size (after storage, µm) | 1.60 | 1.71 | 3.42 |
| | | | (µm) |

**Table 4**

| | Example 6 | Example 7 | Comparative Example |
|---|---|---|---|
| Volume average particle size (before storage) | 1.47 | 1.65 | 1.41 |
| Volume average particle size (after storage) | 2.05 | 1.71 | 3.56 |
| | | | (µm) |

As a result, when the shaking was not performed, the volume average particle size of capsule constructs from Example 6 and Example 7 did not change before and after storage, showing the excellent storage stability of these constructs. On the other hand, the volume average particle size of particles of Comparative Example increased after storage.

When shaking was performed before storage, the volume average particle size of the capsule construct of Example 7 almost did not change before and after storage, but the volume average particle size of the capsule construct of Example 6 increased slightly after storage. The volume average particle size of the particles of Comparative Example showed the same change as with the above case without shaking.

The capsule constructs of Example 6 and Example 7 after storage with shaking were observed under a light microscope. As a result, it was observed that the capsule constructs of Example 7 were in a good dispersion condition, but those of Example 6 were agglomerated and the PHA coating of some capsule constructs had come off.

From the above described results, it was found that a hydrophobic PHA capsule encapsulating an inorganic base material stably can be produced by coating the inorganic base material with a PHA having a hydrophilic functional group having high affinity to the inorganic base material, and then coating the particle surface with PHA copolymer made with a hydrophilic PHA monomer unit and a hydrophobic PHA monomer unit increasing the percentage of the hydrophobic PHA monomer unit toward the surface.

### Example 9 Preparation of capsule construct (8)

To 10 parts of a PHA synthetase solution (10U/ml) derived from a pYN2-C1 recombinant strain, one part of alumina particles of which particle size was adjusted by precipitation to an volume average particle size of 1.45 µm and 39 parts of PBS were added and the mixture was reacted with mild shaking for 30 minutes at 30°C to adsorb PHA synthetase onto the surface of the alumina particles. Then the mixture was centrifuged at 10,000×g, 4°C, for 10 min, and the precipitate was suspended in a PBS solution and centrifuged again (10,000×g, 4°C, for 10 min.) to obtain the immobilized enzyme.

The immobilized enzyme was suspended in 48 parts of a 0.1M phosphate buffer solution (pH 7.0), to which 0.8 parts of (R,S)-3-hydroxy-5-phenoxyvaleryl CoA, 0.2 parts of (R,S)-3-hydroxy-7,8-epoxyoctanoyl CoA and 0.1 part of bovine serum albumin (Sigma) were added, and incubated with mild shaking for 2 hours at 30°C to obtain Sample 1. Here, (R,S)-3-hydroxy-5-phenoxyvaleryl CoA was prepared from 3-hydroxy-5-phenylvaleric acid according to the method described in Eur. J. Biochem., 250, 432-439 (1997), and 3-hydroxy-5-phenylvalerylate was prepared by first preparing 3-hydroxy-5-phenylvaleriate from 3-phenoxypropanal and ethyl bromoacetate by the Reformatsky reaction, and they hydrolyzing it. (R,S)-3-hydroxy-7,8-epoxyoctanoyl CoA was prepared by the method described in Eur. J. Biochem., 250, 432-439 (1997) from 3-hydroxy-7,8-epoxyoctanoic acid synthesized by epoxidizing the unsaturated part of 3-hydroxy-7-octenoic acid synthesized by the method described in Int. J. Biol. Macromol., 12, 85-91 (1990) with 3-chlorobenzoic acid.

Separately, Sample 2 was obtained by the above described method except that 3-hydroxyoctanoyl CoA was used in the place of (R,S)-3-hydroxy-7,8-epoxyoctanoyl CoA.

A 10 µl aliquot of the above sample was put on a slide glass, to which 10 µl of a 1% solution of Nile blue A in water was added. These solutions were mixed on the slide glass, covered with a cover glass, and observed under a fluorescence microscope (330 to 380 nm excitation filter, 420 nm long pass absorption filter, Nikon Corp.). As a result, fluorescence from the surface of the alumina particles was observed to confirm that the alumina particles were coated with PHA on the surface.

As a control, one part of alumina particles was added to 49 parts of a 0.1M phosphate buffer (pH 7.0), and incubated with mild shaking for 2.5 hours at 30°C. Observation under a fluorescence microscope after Nile blue A staining showed the surface of the alumina particle did not fluoresce at all.

Further, part of the above coated particles was collected by centrifugation (10,000×g, 4°C, for 10 min.), dried in vacuum, suspended in chloroform, and stirred for 20 hours at 60°C to extract the PHA coating. The extract was subjected to 1H-NMR analysis (equipment: FT-NMR (Bruker DPX400); measured nuclide: 1H; solvent: CDCl₃ including TMS). The calculated unit percentage of respective units are shown in Table 5

**Table 5**

| Composition of shell PHA of capsule construct (1H-NMR, %) | | |
|---|---|---|
| Monomer unit | Sample 1 | Sample 2 |
| 3-hydroxy-5-phenoxyvaleric acid | 83% | 77% |
| 3-hydroxy-7,8-epoxyoctanoic acid | 17% | - |
| 3-hydroxyoctanoic acid | - | 23% |

The capsule construct of the above-described Sample 1 was washed three times by repeating centrifugation (10,000×g, 4°C, for 10 min.) and suspending the capsule construct in the equivalent volume of purified water. Then to 50 parts of the resulting suspension, was dissolved 0.5 part of hexamethylenediamine as a crosslinking agent. After confirming that the crosslinking agent was dissolved in the suspension, water was removed by lyophilization to give Sample 3. Then Sample 3 was reacted for 12 hours at 70°C to give Sample 4.

The above described Sample 3 and Sample 4 were suspended in chloroform respectively and stirred for 20 hours at 60°C to extract PHA coating, then chloroform was removed by vacuum drying. The extracts were analyzed by using a differential scanning calorimeter (DSC; Perkin Elmer, Pyris 1, temperature rise: 10°C/min.). As a result, Sample 3 showed prominent exothermal peak at about 90°C indicating that epoxy groups in the polymer were reacting with the hexamethylenediamine and the crosslinking between polymers were proceeding during heating. On the other hand, Sample 4 did not show marked heat flow indicating that the crosslinking reaction had been completed.

Further, the infrared absorption was measured with Samples 3 and 4 (FT-IR; Perkin Elmer, 1720X). As a result, peaks indicating amine (at about 3340 cm⁻¹) and epoxy (at about 822 cm⁻¹) were observed with Sample 3, but not with Sample 4.

From the above described results, it was shown that the crosslinked polymer were obtained by reacting PHA having epoxy on the side group with hexamethylenediamine.

On the other hand, when Sample 2 was treated in the same manner, clear evidence to show crosslinking between polymers was not obtained.

### Example 10 Preparation of capsule construct (9)

To 10 parts of a PHA synthetase solution (10U/ml) derived from a pYN2-C1 recombinant strain, one part of alumina particles of which.particle size was adjusted by precipitation to an volume average particle size of 1.45 µm and 39 parts of PBS were added and the mixture was reacted with mild shaking for 30 minutes at 30°C to adsorb PHA synthetase onto the surface of the alumina particles. Then the mixture was centrifuged at 10,000xg, 4°C, for 10 min, and the precipitate was suspended in a PBS solution and centrifuged again (10,000×g, 4°C, for 10 min.) to obtain the immobilized enzyme.

The immobilized enzyme was suspended in 48 parts of a 0.1M phosphate buffer solution (pH 7.0), to which 0.8 parts of (R,S)-3-hydroxy-5-phenoxyvaleryl CoA, 0.2 parts of (R,S)-3-hydroxy-7,8-epoxyoctanoyl CoA and 0.1 part of bovine serum albumin (Sigma) were added, and incubated with mild shaking for 2 hours at 30°C to obtain Sample 5. Here, (R,S)-3-hydroxy-5-phenoxyvaleryl CoA was prepared from 3-hydroxy-5-phenylvaleric acid according to the method described in Eur. J. Biochem., 250, 432-439 (1997), and 3-hydroxy-5-phenylvalerylate was prepared by first preparing 3-hydroxy-5-phenylvaleriate from 3-phenoxypropanal and ethyl bromoacetate by the Reformatsky reaction, and they hydrolyzing it. (R,S)-3-hydroxy-7,8-epoxyoctanoyl CoA was prepared by the method described in Eur. J. Biochem., 250, 432-439 (1997) from 3-hydroxy-7,8-epoxyoctanoic acid synthesized by epoxidizing the unsaturated part of 3-hydroxy-7-octenoic acid synthesized by the method described in Int. J. Biol. Macromol., 12, 85-91 (1990) with 3-chlorobenzoic acid.

A 10 µl aliquot of the above Sample 5 was put on a slide glass, to which 10 µl of a 1% solution of Nile blue A in water was added. These solutions were mixed on the slide glass, covered with a cover glass, and observed under a fluorescence microscope (330 to 380 nm excitation filter, 420 nm long pass absorption filter, Nikon Corp.). As a result, fluorescence from the surface of the alumina particles was observed to confirm that the alumina particles were coated with PHA on the surface.

As a control, one part of alumina particles was added to 49 parts of a 0.1M phosphate buffer (pH 7.0), and incubated with mild shaking for 2.5 hours at 30°C. Observation under a fluorescence microscope after Nile blue A staining showed the surface of the alumina particle did not fluoresce at all.

Further, part of the above coated particles was collected by centrifugation (10,000xg, 4°C, for 10 min.), dried in vacuum, suspended in chloroform, and stirred for 20 hours at 60°C to extract the PHA coating. The extract was subjected to 1H-NMR analysis (equipment: FT-NMR (Bruker DPX400); measured nuclide: 1H; solvent: ²H chloroform including TMS). The calculated unit percentages were: 3-hydroxy-5-phenoxyvaleric acid 83% and 3-hydroxy-7,8-epoxyoctanoic acid 17%.

The capsule construct of the above-described Sample 5 was washed three times by repeating centrifugation (10,000xg, 4°C, for 10 min.) and suspending the capsule construct in the equivalent volume of purified water. Then the resulting suspension was lyophilized to remove water, and then 10 parts of terminal amino modified polysiloxane (modified silicone oil TSF4700, GE Toshiba Silicones) was added to the capsule constructs to react for two hours at 70°C. The reaction product was washed three times by suspending in methanol and subjecting to centrifugation (10,000xg, 4°C, for 20 min.) and dried. Thus, PHA having a graft chain of polysiloxane was obtained.

### Example 11 Capsule toner production

First, a polymer particulate was produced as a core by the method described below. After 450 parts of a 0.1M solution of Na₃PO₄ in water were added to 710 parts of ion exchange water and heated to 60°C, this mixture was stirred at 12,000 rpm by a TK homomixer (TOKUSHU KIKA KOGYO CO., LTD.). Then, 68 parts of a 1.0M solution of CaCl₂ in water were gradually added to the mixture to obtain an aqueous medium containing Ca₃(PO₄)₂. Next, 165 parts of styrene monomer, 35 parts of n-butyl acrylate, 12 parts of copper phthalocyanine pigment, 10 parts of unsaturated polyester (fumaric acid-propylene oxide modified bisphenol A), and 60 parts of ester wax. 10 parts of a polymerization initiator, 2,2'-azobis(2,4-dimethylvaleronitrile), were dissolved in the above mixture to prepare a polymeric monomer composition and heated to 60°C, then uniformly dissolved and dispersed in the mixture at 12,000 rpm by using a TK homomixer (TOKUSHU KIKA KOGYO CO., LTD). The above described polymeric monomer composition was added to the above describe aqueous medium and stirred for 10 minutes at 10,000 rpm by the TK homomixer under nitrogen atmosphere at 60°C in order to perform granulation. After the granulation, the temperature was raised to 80°C while stirring with paddle stirring blades, then the reaction was performed for 10 hours. After the polymerization reaction was completed, the suspension was cooled down, and 3.6 parts of Na₂CO₃ were added to the suspension to make pH to 11. Then, a solution in which 0.3 part of potassium persulfate as a polymerization initiator was added to and dissolved in a polymeric monomer system comprised of 82 parts of styrene monomer, 12 parts of n-butyl acrylate, and 0.05 part of unsaturated polyester (fumaric acid-propylene oxide modified bisphenol A) was dropped into the above described suspension. The temperature of the solution was increased to 80°C, then the reaction was allowed to be conducted for further 6 hours. Then, the suspension was cooled to room temperature, and hydrochloric acid was added thereto for dissolving and removing calcium phosphate. Then, a filtration process and a drying process were conducted to obtain a core component particulate of a toner.

10 parts of the above described particulate and 390 parts of PBS were added to 100 parts of a PHA synthetase solution (10U/ml) derived from pYN2-Cl recombinant strain, and moderately shaken for 30 minutes at 30°C to allow adsorption of the PHA synthetase to the surface of the above described particulate. This was subjected to centrifugation (10,000xg, 4°C, for 10 min.), and the precipitate was suspended in a PBS solution and again subjected to centrifugation (10,000xg, 4°C, for 10 min.) to obtain an immobilized enzyme.

Then 10 parts of the above described immobilized enzyme was suspended in 480 parts of a 0.1M phosphate buffer (pH 7.0), then 10 parts of (R)-3-hydroxy-5-(4-fluorophenyl)valeryl CoA (prepared by a method described in Eur. J. Biochem., 250, 432-439 (1997) after obtaining 3-hydroxy-5-(4-fluorophenyl)valeric acid by hydrolyzing 3-hydroxyphenyl valeric acid ester obtained by a Reformatsky reaction) and one part of bovine serum albumin (Sigma) were added to the suspension, and this suspension was moderately shaken for two hours at 30°C.

After the reaction was completed, the suspension was subjected to centrifugation (10,000xg, 4°C, for 10 min.), and the precipitate was suspended in 1000 parts of a 0.1M phosphate buffer (pH 7.0) and again subjected to centrifugation. This operation was repeated three times to recover the precipitate. After filtration and drying, a capsule construct was obtained. Then 0.12 parts of hydrophobic titanium oxide fine powder was added to 10 parts of the obtained capsule construct to obtain a capsule toner A having titanium oxide fine powder on the surface of the capsule construct.

Meanwhile, 0.12 parts of hydrophobic titanium oxide fine powder was added to 10 parts of the above described core component particulate to obtain, as a control, toner B having titanium oxide fine powder on the surface of the toner.

Six parts of each capsule toner were mixed with 144 parts of a ferrite carrier coated with acrylic resin to obtain a two-component developer.

With these developers, an image was copied by using a commercially available copying machine NP6000 (Canon) in an environment where the temperature was 23°C and the humidity was 60%RH, to estimate running durability, toner scattering, fogging, etc. As a result, with the developer using capsule toner A, image defects such as decrease in the image density, toner scattering, fog, etc., were not observed even after 100,000 copies were produced as shown in Table 6. In addition, when the triboelectricity was measured as an electrostatic property, the initial value was -33 mC/kg and the value after the durability test was -31 mC/kg, indicating stable electrostatic properties. Further, no problem was observed with fixing. On the other hand, with the developer using the capsule toner B, image defects such as lowering of image density, toner scattering, fog, etc. were observed when only 200 sheets of copies were made so that images of high precision and fidelity were not obtained. In addition, triboelectricity varied from initial -24 mC/kg to -18 mC/kg after durability test, indicating unstable chargeability. Further, as a result of the fixing test, it was inferior in offset resistance at high temperature.

**Table 6**

| | Production property | Image density | Image quality | Electrostatic property | Fixing property |
|---|---|---|---|---|---|
| Capsule toner A | A | A | A | A | A |
| Capsule toner B | A | B | B | A | B |

| | | | | | |
|---|---|---|---|---|---|
| A: good, B: somewhat inferior, C: no good | | | | | |

### Example 12 Production of capsule toner (2)

Ten parts of core particles prepared in the same manner as in Example 11 and 390 parts of PBS were added to 100 parts of a PHA synthetase solution (10U/ml) derived from pYN2-C1 recombinant strain, and moderately shaken for 30 minutes at 30°C to allow adsorption of the PHA synthetase to the surface of the particles. The reaction was subjected to centrifugation (10.000xg, 4°C, for 10 min.), then the precipitate was suspended in a PBS solution and again subjected to centrifugation (10,000xg, 4°C, for 10 min.) for obtaining an immobilized enzyme.

Ten parts of the above described immobilized enzyme was suspended in 480 parts of a 0.1M phosphate buffer (pH 7.0), then 10 parts of (R)-3-hydroxy-5-(4-fluorophenyl)valeryl CoA (prepared by a method described in Eur. J. Biochem., 250, 432-439 (1997) after obtaining 3-hydroxy-5-(4-fluorophenyl)valeric acid by hydrolyzing 3-hydroxyphenylvaleric acid ester obtained by a Reformatsky reaction) and one part of bovine serum albumin (Sigma) were added to the suspension and moderately shaken for one and a half hours at 30°C. After the reaction was completed, centrifugation was performed for 10 minutes at 10,000xg to recover the pellet.

Then, the pellet was suspended in 480 parts of a 0.1M phosphate buffer (pH 7.0), and 5 parts of (R)-3-hydroxy-5-phenoxyvaleryl CoA (prepared by a method described in Eur. J. Biochem., 250, 432-439 (1997) after obtaining 3-hydroxy-5-phenoxyvaleric acid by hydrolyzing 3-hydroxy-5-phenoxylvaleric acid ester obtained by a Reformatsky reaction using zinc, in which 3-phenoxypropanal synthesized by a method described in J. Org. Chem., 55, 1490-1492 (1990) and ethyl bromoacetate were used as raw materials) and one part of bovine serum albumin (Sigma) were added to the suspension before moderately shaking the suspension for 30 minutes at 30°C.

After the reaction was completed, centrifugation was performed for 10 minutes at 10,000xg, then the precipitate was suspended in 1000 parts of a 0.1M phosphate buffer (pH 7.0) and again subjected to centrifugation. This operation was repeated three times to recover the precipitate. From this precipitate, a capsule construct was obtained through a filtration process and a drying process.

Weight of the polymer formed on the surface of the capsule construct was measured by a time-of-flight secondary ion mass spectroscope (TOF-SIMS IV, CAMECA). From the obtained mass spectrum, it was confirmed that the surface of the capsule construct was made of poly-3-hydroxy-5-phenoxyvaleric acid. In addition, mass spectra were further taken by the TOF-SIMS as described above while scraping the surface of the capsule construct little by little by ion sputtering, and consequently, it was confirmed that the polymer of the capsule construct was made with poly-3-hydroxy-5-(4-fluorophenyl)valeric acid from a certain point. As a result of the above measurement, it was found that the capsule construct of this example was an intended capsule construct in which poly-3-hydroxy-5-(4-fluorophenyl)valeric acid having a low glass transition temperature was coated with poly-3-hydroxy-5-phenoxyvaleric acid having a relatively high glass transition temperature.

0.12 part of hydrophobic titanium oxide fine powder was added to 10 parts of the capsule construct to obtain a capsule toner C having titanium oxide fine powder on a surface of the capsule construct.

144 parts of ferrite carrier coated with an acrylic resin were mixed with 6 parts of the capsule toner C to obtain a two-component developer.

Using the above described developer, an image was copied by a commercially available copying machine NP6000 (Canon Inc.) as described in Example 7 in an environment where the temperature was 23°C and the humidity was 60%RH, then the running durability, toner scattering, fog, etc. were evaluated. According to the evaluation, this toner exhibited the same good performance as with the capsule toner A of Example 7.

In addition, to evaluate fixing properties of the capsule toners A and C at a low temperature, a fixing test was performed by an external fixing device having the same arrangement as the NP6000. In this fixing test, a strip of 2 cm x 10 cm carrying an unfixed image on it was passed between rollers along a longitudinal direction of the strip while monitoring the temperature of the upper roller of the external fixing devise, then fixing property was determined by checking whether the offset was seen or not at the rear part of the strip. From this result, it was found that the lowest fixation temperature was as low as 95°C in both cases of capsule toner A and capsule toner C, so that these toners were excellent in the fixing property at low temperature.

Further, to evaluate the blocking resistance of the capsule toners A and C, the cohesiveness was observed by allowing the above described capsule toners to stand for 3 days at every degree from 50°C to 70°C. Then, using these toners, images were developed and evaluated as described above. A point at which roughness in a highlight region changed was defined as a blocking resistance temperature. According to the evaluation, it was found that the blocking resistance of the capsule toner C was 61°C and the blocking resistance of the capsule toner A was 58°C, so that the capsule toner C was excellent in the blocking resistance.

As described above, the low-temperature fixing property is realized by capsulating a toner with a PHA of low glass transition temperature, and further by coating a capsule toner coated with a PHA of low glass transition temperature, with a PHA of high glass transition temperature, both low temperature fixation and blocking resistance can be achieved at the same time.

### Example 13 Production of capsule toner (3)

A 3 liter four-neck separable flask was equipped with a reflux condenser, a thermometer, a nitrogen introducing tube, and a stirrer. Next, a mixture of 1200 parts of ion exchange water, 15 parts of polyvinyl alcohol, 0.1 part of dodecyl sodium sulfate, 75 parts of styrene monomer, 25 part of n-butyl acrylate, 5 parts of chromium di-tert-butylsalicylate complex salt, 5 parts of copper phthalocyanine, and 6 parts of 2,2-azobis(2,4-dimethylvaleronitrile) was charged into this flask, then the mixture was stirred by a high-speed stirrer, TK-homomixer, for 10 minutes at 10,000 rpm for granulation. Then, the revolutions per minute was decreased to 1,000 rpm and bubbling with nitrogen gas was sufficiently performed. Next, substituting the stirring blades of crescent-shape for the above homomixer, polymerization was performed for 16 hours in an oil bath at 80°C with mild stirring.

After the polymerization reaction was completed, the reaction vessel was cooled to room temperature, then the dispersion was washed by decantation for 5 times, filtered, washed with water, and dried to obtain core particles as blue powder. The PHA synthetase derived from pYN2-C1 recombinant strain was immobilized to the above described core particles by the method described in Example 11, to obtain an immobilized enzyme.

Ten parts of the above described immobilized enzyme were suspended in 480 parts of a 0.1M phosphate buffer (pH 7.0), then 8 parts of (R,S)-3-hydroxy-5-phenoxyvaleryl CoA (prepared by a method described in Eur. J. Biochem., 250, 432-439 (1997) after obtaining 3-hydroxy-5-phenoxyvaleric acid by hydrolyzing 3-hydroxy-5-phenoxyvaleric acid ester obtained by a Reformatsky reaction of 3-phenoxypropanal with ethyl bromoacetate), 2 parts of (R,S)-3-hydroxy-7,8-epoxyoctanoyl CoA (prepared by a method described in Eur. J. Biochem., 250, 432-439 (1997) after epoxidizing an unsaturated part of 3-hydroxy-7-octenoic acid with 3-chlorobenzoic acid, the 3-hydroxy-7-octenoic acid being synthesized by a method described in Int. J. Biol. Macromol., 12, 85-91 (1990)), and 1 part of bovine serum albumin (Sigma) were added to the suspension and moderately shaken for 2 hours at 30°C.

After the reaction was completed, the above described reaction solution was subjected to centrifugation (10,000×g, 4°C, for 10 min.) to collect capsule constructs and the capsule constructs were suspended in 100 parts of purified water. This operation was repeated 3 times to recover precipitate. From the precipitate, a blue capsule construct D was obtained through a filtration process and a drying process. Then 0.2 part of ground hydrophobic silica having 362 m²/g BET was mixed with 10 parts of capsule constructs D by a Henschel mixer to obtain a toner D.

Further, 10 parts of the above described capsule toner D was suspended in purified water and 5 parts of hexamethylenediamine were dissolved therein as a crosslinking agent. After confirming that the crosslinking agent was dissolved, water was removed by lyophilization and the reaction was performed for 12 hours at 70°C. After the reaction was completed, the above described reaction solution was subjected to centrifugation for 10 minutes at 10,000xg, and the precipitate was suspended in 1000 parts of a 0.1M phosphate buffer (pH 7.0) and again subjected to centrifugation. This operation was repeated 3 times to recover precipitate. From the precipitate, a blue capsule construct E was obtained after filtration and drying. Then 0.2 part of ground hydrophobic silica whose BET value was 362 m²/g was added to 10 parts of capsule constructs E and mixed by a Henschel mixer to obtain a toner E.

To evaluate the image developed with the above prepared toners, 6 parts of the toner and 94 parts of ferrite carrier of 35 µm in particle size and coated with a silicone resin were charged into a polyethylene bottle and mixed with each other, then the mixture was stirred by a tumbler mixer to prepare a two-component developer. And this developer was set in a modified color laser copying machine CLC-500 (Canon Inc.), and the initial image and the image after ten thousand sheet copying at 23°C and 60%RH were observed by SEM, then the image quality and the deterioration of developer were evaluated.

The evaluation of the image quality was performed by a multivalue recording with the pulse width modulation (PWM) of laser within one pixel and the reproducibility of a minimum spot was evaluated by a microscopic observation. In addition, the developer after copying a ten thousand times was observed by a scanning electron microscope.

Further, to evaluate the blocking resistance of the toner, agglomeration was observed after standing the toner for 3 days under different temperature conditions. Then, using each toner sample for the developer as above, the image evaluation was carried out.

In addition, to evaluate the fixing properties, a fixing test was performed by an external fixing device having the same arrangement as the CLC-500. In this fixing test, a strip of 2 cm wide by 10 cm long was made, and the unfixed image on the strip was fixed by passing the strip between rollers along a longitudinal direction of the strip while monitoring the temperature of the upper roller of the external fixing devise, then the fixing property was determined by checking whether the offset was seen or not at the rear part of the strip.

Meanwhile, the above described capsule construct was replaced with the core particle without capsulation to form toner F, and the same evaluation as described above was performed. The results are shown in Table 7.

**Table 7**

| | Image quality | Blocking resistance | Fixing property |
|---|---|---|---|
| Toner D | AA | A | AA |
| Toner E | AA | AA | AA |
| Toner F | A | A | B |

| | | | |
|---|---|---|---|
| Notes: Symbols in Table; AA: very good, A: good, B: somewhat inferior, C: no good (the above symbols AA, A, Baned C do not correspond to the symbols in Example 11) | | | |

### Example 14 Production of capsule toner (4)

A core particulate was produced in the same manner as in Example 13 and the PHA synthetase derived from pYN2-C1 recombinant strain was immobilized to obtain an immobilized enzyme. Then 10 parts of the immobilized enzyme were suspended in 480 parts of a 0.1M phosphate buffer (pH 7.0), then 8 parts of (R,S)-3-hydroxy-5-phenoxyvaleryl CoA (prepared as with Example 13), 2 parts of (R,S)-3-hydroxy-7,8-epoxyoctanoyl CoA (prepared as with Example 14), and one part of bovine serum albumin (Sigma) were added to the suspension, and moderately shaken for 2 hours at 30°C.

After the reaction was completed, the above described reaction solution was subjected to centrifugation (10,000xg, 4°C, for 10 min.) to recover capsule constructs and the capsule constructs were suspended in 100 parts of purified water. This operation was repeated 3 times to recover precipitate. From the precipitate, a blue capsule construct G was obtained after filtration and drying. Then 0.2 part of ground hydrophobic silica whose BET value was 362 m²/g was added to 10 parts of capsule constructs G and mixed by a Henschel mixer to obtain a toner G.

Further, 100 parts of terminal amino modified polysiloxane (modified silicone oil TSF4700, GE Toshiba Silicones) was added to 10 parts of the capsule construct G for reaction of two hours at 70°C. The reaction product was suspended in methanol and subjected to centrifugation (10,000xg, 4°C, for 20 min.) several times for washing and dried to give a blue capsule construct H having a graft chain of polysiloxane. Then 0.2 part of ground hydrophobic silica whose BET value was 362 m²/g was added to 10 parts of capsule construct H and mixed by a Henschel mixer to obtain a toner H.

With these toners, the image quality and the deterioration of developer were evaluated as with Example 14.

Meanwhile, instead of the above described capsule construct, the core particle without capsulation was used for a toner I, and the same evaluation as described above was performed. The results are shown in Table 8.

**Table 8**

| | Image quality | Blocking resistance | Fixing property |
|---|---|---|---|
| Toner G | AA | A | AA |
| Toner H | AA | AA | AA |
| Toner I | A | A | B |

| | | | |
|---|---|---|---|
| Note: Symbols in Table 8; AA: very good, A: good, B: somewhat inferior, C: no good (wherein the above described symbols do not correspond to symbols in Example 11) | | | |

### Alternative Examples (outside the invention as claimed)

A laminated construct comprised of a sheet of a base material coated with a polymer compound is used, for example, as a recording medium for inkjet recording system. In the inkjet recording system, micro-droplets of ink are applied to a recording medium such as paper to record images and characters on it by ejecting droplets on various operation principles. In such recording system, the ink contains a lot of liquid medium such as water and a mixture of water and organic solvents, so that the ink must be applied in a certain amount to obtain a high image density. In addition, since the ink droplets are continuously ejected to the recording medium, there may occur a beading phenomenon where the ejected ink droplets fuse and consequently dots will fuse leading to image distortion. Therefore, high ink-absorbing capacity and high ink-absorption speed are required for the inkjet recording medium.

For this purpose, it has been proposed a recording medium provided with an ink-receiving layer on the base material to increase ink absorption capacity. For example, Japanese Patent Application Laid-Open No. 55-146786 proposes a recording medium of which base material is coated with a water-soluble resin such as polyvinyl alcohol and polyvinyl pyrrolidone. In addition, Japanese Patent Application Laid-Open No. 5-221112 and others propose a recording medium employing a waterproof resin. Further, a recording medium employing an ionic resin as an ink receiving layer is proposed (Japanese Patent Application Laid-Open Nos. 11-78221 and 2000-190631, for example), to provide a recording medium excellent in wettability, water resistance, and dye-fixing properties, as well as the ink-absorption and drying properties and vivid image forming properties.

To form the ink absorbing layer on the base material, conventionally, coating methods have been widely used, for example, blade coating, air knife coating, roll coating, flash coating, gravure coating, kiss coating, die coating, extrusion coating, slide hopper coating, curtain coating, spray coating, etc.

As the base material of the laminated construct the following substances can be used but not limited thereto: Plastic films of polyethylene terephthalate (PET), diacetate, triacetate, cellophane, celluloid, polycarbonate, polyimide, polyvinyl chloride, polyvinylidene chloride, polyacrylate, polyethylene, polypropylene, or polyester; porous polymer membranes of polyvinyl chloride, polyvinyl alcohol, acetyl cellulose, polycarbonate, nylon, polypropylene, polyethylene, or Teflon; wooden boards; glass plates; fabrics of cotton, rayon, acrylic, silk, or polyester; papers such as wood-free paper, medium-quality paper, art paper, bond paper, recycled paper, baryta paper, cast-coated paper, corrugated fiberboard, and resin-coated paper. The above described base material may have a smooth or uneven surface, and also may be transparent, translucent or opaque. The laminated construct may be made by laminating two or more substances which are selected among the above listed substances.

In the above described process, it is alternatively possible to change the monomer unit composition of the coating PHA in a vertical direction with a flat construct, by changing the composition of 3-hydroxyacyl CoA (e.g., species and/or concentration) in the aqueous reaction solution with time.

Similarly as before, for example, the base material may be coated with one PHA layer of which composition is continuously changed to form a composition gradient in a vertical direction, or may be coated with a plurality of PHA layers of which compositions are different from one another.

Regarding washing, similarly as before, the method for washing is not particularly limited as long as the construct would not be affected undesirably in view of production purpose of the construct. If the construct is a construct in which a plate-like base material is coated with PHA, washing can be performed by soaking the construct in the above-described washing agent, for example. Further, the construct can be subjected to a drying process as required. Furthermore, the construct may be subjected to various secondary processing or chemical modifications before use.

As mentioned before, the present invention enables production of constructs of which production has been difficult by conventional organic synthetic chemistry. Similarly, it is alternatively also possible to obtain a construct having excellent properties that could not have been conferred to laminated constructs manufactured by conventional organic synthetic chemistry. For example, it enables utilization of new polymer compounds or it can provide new functions or construct, which have been difficult by the conventional organic synthetic chemistry. More specifically, by utilizing highly strict molecule-recognition ability or stereo selectivity specific to a biological catalyst derived from a living organism, it is possible to produce a new functional polymer compound not obtainable by the conventional synthetic chemistry, or a laminated construct coated with a polymer compound having extremely high chirality through a significantly simple process.

As described above, in general, various coating methods have been used to form an ink absorption layer on the base material of the conventional recording medium. However, it is possible to produce a recording medium without using such a method. That is, a base material to which an enzyme has been immobilized is allowed to react with, for example, 3-hydroxypimelyl CoA represented by the following chemical formula [21].

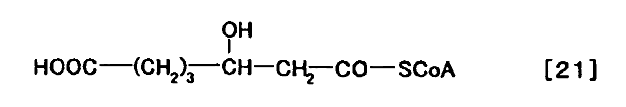

As a result, a recording medium having PHA represented by the following chemical formula [22] as an ink receiving layer is produced, where the PHA has an anionic functional group, i.e., carboxyl group, on the side group.

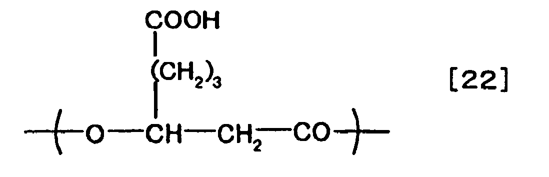

Hence, it becomes possible to manufacture a novel functional recording medium as described above which has been difficult to be manufactured by the conventional techniques. There has been no report on making a recording medium by such techniques.

### Alternative Example 1: Preparation of laminated construct

A porous glass sheet of 10 mm × 10 mm × 1 mm was dipped into 1% glutaraldehyde for one hour and washed with purified water, then the glass sheet was dipped into a PHA synthetase solution (10U/ml) derived from the recombinant strain pYN2-C1 for 30 minutes at 30°C in order to immobilize the above described enzyme to the glass sheet. Unreacted PHA synthetase was removed by washing with PBS, and consequently an immobilized enzyme was obtained.

The above described immobilized enzyme was dipped into 0.1M phosphate buffer (pH 7.0) containing 30 mM (R)-3-hydroxyoctanoyl CoA (prepared by a method described in Eur. J. Biochem., 250, 432-439 (1997)) and 0.1% bovine serum albumin (Sigma), then moderately shaken for two hours at 30°C. After completing the reaction, the immobilized enzyme was washed with 0.1M phosphate buffer (pH 7.0) to remove unreacted substances or the like.

The glass sheet after being subjected to the reaction was stained with a 1% solution of Nile blue A in water and observed by a fluorescence microscope (330 to 380 nm excitation filter, 420 nm long pass absorption filter, Nikon Corp.). As a result, fluorescence was observed on the surface of the glass sheet to show that the glass sheet was coated with a layer of PHA forming a laminated construct.

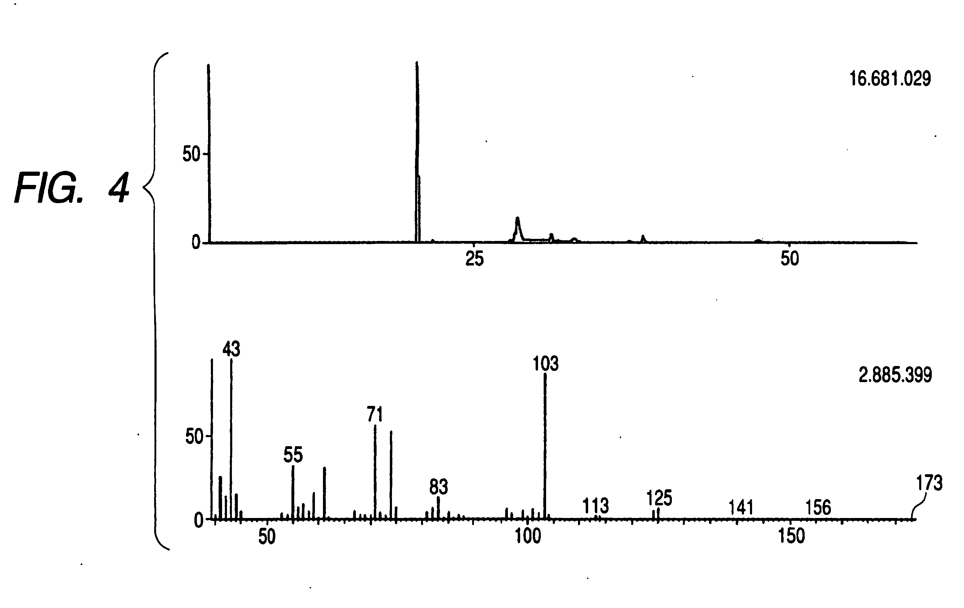

Next, the above described laminated construct was dried in a vacuum, and dipped into chloroform with stirring for 20 hours at 60°C to extract PHA coating. After the extract was filtered through a membrane filter of 0.45 µm pore size, and concentrated under a reduced pressure by a rotary evaporator, methanolysis was performed according to the conventional method and analysis was performed by a gas chromatography-mass spectroscope (GC-MS, Shimadzu QP-5050, an EI method), and the methyl-esterified PHA monomer units were identified. As a result of the identification, it was confirmed that the above described PHA was a PHA made with 3-hydroxyoctanoic acid monomer unit as shown in FIG. 4.

### Alternative Example 2:

### Preparation of recording medium

A PET film (100Q80D, TORAY Inc.) was dipped into a 1% glutaraldehyde solution for one hour and washed by purified water, then the film was dipped into a PHA synthetase solution (10U/ml) derived from pYN2-C1 recombinant strain for 30 minutes at 30°C to immobilized the enzyme to the film. Unreacted PHA synthetase was removed by washing with PBS, then an immobilized enzyme was obtained.

The above described PET film was dipped into a 0.1M phosphate buffer (pH 7.0) including 30mM (R)-3-hydroxypimelyl CoA (prepared by a method described in J. Bacteriol., 182, 2735-2760 (2000)) and 0.1% bovine serum albumin (Sigma), and moderately shaken for 30 minutes at 30°C. After the reaction was completed, the film was washed with a 0.1M phosphate buffer (pH 7.0) to remove unreacted substances, and air-dried. Consequently, a desired recording medium having an ionic polymer of poly-(R)-3-hydroxypimelic acid as an ink receiving layer was obtained.

As a comparative example, a PET film was made by the similar treatment as described above, except that PHA synthetase was not used.

Using these recording media respectively, a black image and a colored image were printed thereon by an inkjet printer (BJC-4301, Canon Inc.). Then, whether the feathering or beading of the ink were present or not on the recording medium was checked. As a result, such feathering or beading could hardly be seen and excellent images were obtained with the recording medium, while heavy feathering or beading were observed with the comparative recording medium.

### Alternative Example 3:

### Preparation of recording medium

As described in Alternative Example 2, a PET film (100Q80D, TORAY Inc.) was dipped into a 1% glutaraldehyde solution for one hour and washed with purified water, then the film was dipped into a PHA synthetase solution (10U/ml) of pYN2-C1 recombinant strain for 30 minutes at 30°C to immobilized the enzyme to the film. Unreacted PHA synthetase was washed away with PBS, then an immobilized enzyme was obtained.

The PET film to which this enzyme was immobilized was dipped into 100 parts of a 0.1M phosphate buffer (pH 7.0) including 30mM (R)-3-hydroxyoctanoyl CoA (prepared by a method described in Eur. J. Biochem., 250, 432-439 (1997)) and 0.1% bovine serum albumin (Sigma). Then, 0.1M phosphate buffer (pH 7.0) including 30mM (R)-3-hydroxypimelyl CoA (prepared by a method described in J. Bacteriol., 182, 2735-2760 (2000)) and 0.1% bovine serum albumin (Sigma) was added to the above reaction solution by the use of a micro-tube pump (TOKYO RIKAKIKAI Co., MP-3N) at a rate of 25 parts per minute while moderately shaken at 30°C.

After shaking for 30 minutes, the film was washed with a 0.1M phosphate buffer (pH 7.0) to remove unreacted substances or the like, and air-dried. Consequently, a recording medium was prepared.

The amount of the polymer formed on the surface of the recording medium was measured by a time-of-flight secondary ion mass spectroscope (TOF-SIMS IV, CAMECA). The obtained mass spectrum showed that the surface of the recording medium was made up by a copolymer of 3-hydroxypimelic acid and 3-hydroxyoctanoic acid (the molar ratio was 17:1). In addition, mass spectra were taken by the TOF-SIMS as described above while scraping the surface of the glass sheet little by little by ion sputtering. As a result, the percentage of 3-hydroxypimelic acid in the copolymer gradually decreased in an inward direction while the percentage of 3-hydroxyoctanoic acid gradually increased. As a result, it was shown that the recording medium of this example was an intended recording medium having the outer surface of polyhydroxypimelate having hydrophilic functional groups, and under the polyhydroxypimelate layer a layer of a copolymer of 3-hydroxypimelic acid having a hydrophilic functional group and 3-hydroxyoctanoic acid having a hydrophobic functional group where the percentage of the 3-hydroxyoctanoic acid gradually increased in a direction toward the lower part of this layer.

Using this recording medium, a black image and a colored image were printed thereon by an inkjet printer (BJC-4301, Canon Inc.) as described in Alternative Examples 2. Then, whether the blots or beading of the ink were present or not on the recording medium was checked, and this result was compared to that of the recording medium prepared in Alternative Example 2. As a result, it was found that the recording medium of this example had less blots in the ink receiving layer compared to the recording medium of Alternative Example 2. The reason of this can be considered as follows: the PHA coating of the recording medium of this example gradually becomes hydrophobic in a direction toward the lower part of this layer, so that the diffusion of the absorbed ink at the surface is gradually suppressed in the layer.

## Claims

1. An electrophotographic encapsulated toner particle comprising a core particle as base material coated with a shell of polyhydroxyalkanoate,
wherein the polyhydroxyalkanoate comprises a 3-hydroxyalkanoic acid unit other than 3-hydroxypropionic acid unit, 3-hydroxy-n-butyric acid unit, and 3-hydroxy-n-valeric acid unit, and
wherein said polyhydroxyalkanoate is a polyhydroxyalkanoate comprising at least one selected from the group consisting of monomer units represented by a chemical formula [1] to a chemical formula [10]: 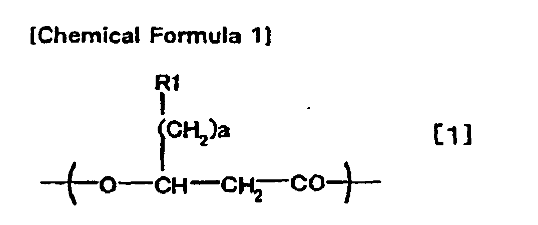 wherein R1 and a are selected from the group of combinations consisting of:
R1 is hydrogen atom (H) and a is any of integers from 3 to 10;
R1 is a halogen atom and a is any of integers from 1 to 10;
R1 is a chromophore and a is any of integers from 1 to 10;
R1 is a carboxyl group or a salt thereof and a is any of integers from 1 to 10; and
R1 is 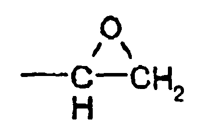
and a is any of Integers from 1 to 7; 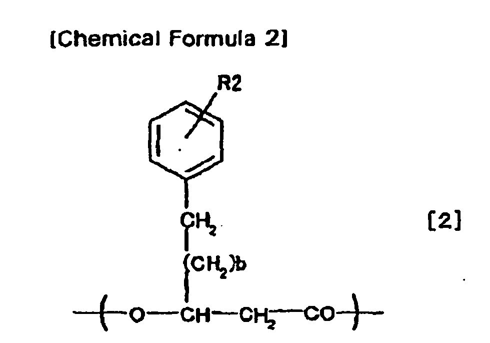
wherein b represents any of integers from 0 to 7 and R2 is selected from the group consisting of hydrogen atom (H), halogen atoms, -CN, -NO₂, -CF₃, -C₂F₅, and -C₃F₇); 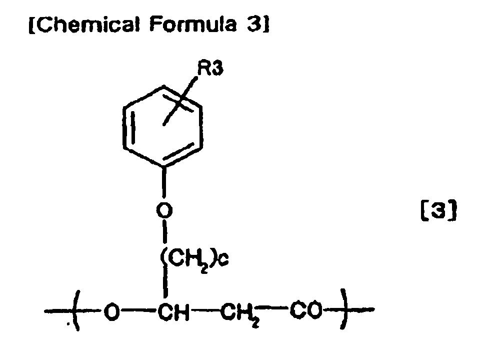 wherein c represents any of integers from 1 to 8 and R3 is selected from the group consisting of hydrogen atom (H), halogen atoms, -CN, -NO₂, -CF₃, -C₂F₅, and -C₃F₇; 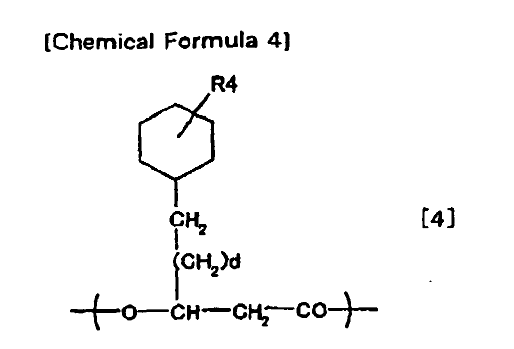 wherein d represents any of integers from 0 to 7 and R4 is selected from the group consisting of hydrogen atom (H), halogen atoms, -CN, -NO₂, -CF₃, -C₂F₅, and -C₃F₇; 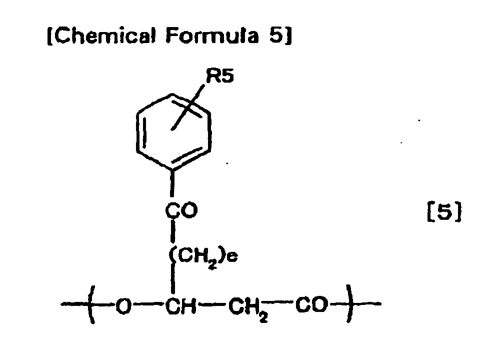 wherein e represents any of integers from 1 to 8 and R5 is selected from the group consisting of hydrogen atom (H), halogen atoms, -CN, -NO₂, -CF₃, -C₂F₅, -C₃F₇, -CH₃, -C₂H₅, and -C₂H₇; 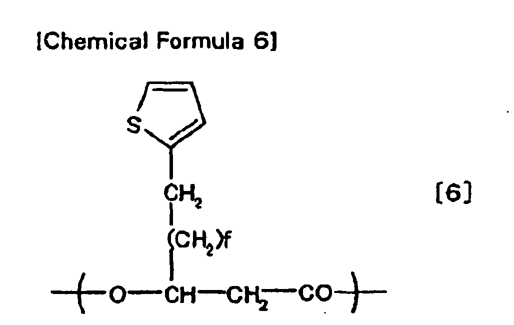 wherein f represents any of integers from 0 to 7; 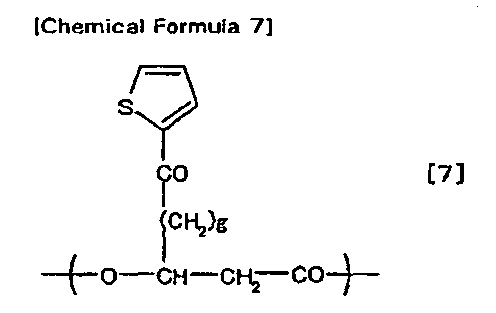 wherein g represents any of integers from 1 to 8; 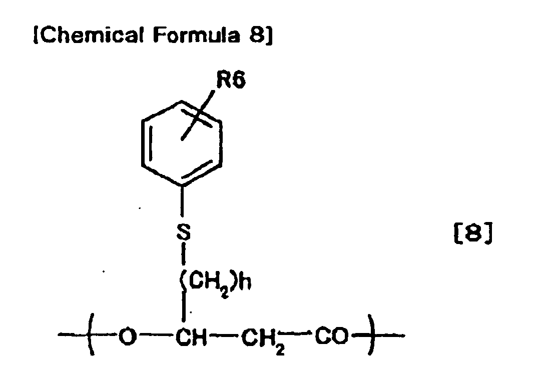 wherein h represents any of integers from 1 to 7 and R6 is selected from the group consisting of hydrogen atom (H), halogen atoms, -CN, -NO₂, -COOR', -SO₂R", -CH₃, -C₂H₅, -C₃H₇, -CH(CH₃)₂, and -C(CH₃)₃, wherein R' is selected from the group consisting of hydrogen atom (H), Na, K, -CH₃, and -C₂H₅, and R" is selected from the group consisting of -OH, -ONa, -OK, halogen atoms, -OCH₃, and -OC₂H₅; 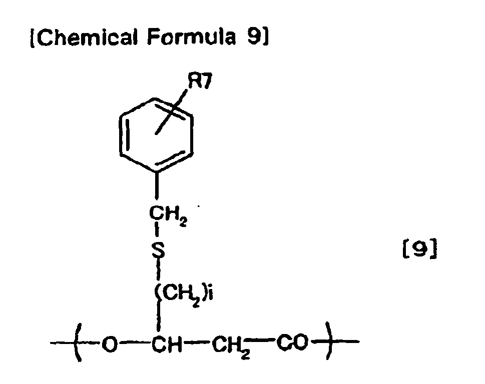 wherein i represents any of integers from 1 to 7 and R7 is selected from the group consisting of hydrogen atom (H), halogen atoms, -CN, -NO₂, -COOR', -SO₂R", wherein R' is selected from the group consisting of hydrogen atom (H), Na, K, -CH₃, and -C₂H₅, and R" is selected from the group consisting of -OH, -ONa, -OK, halogen atoms, -OCH₃, and -OC₂H₅; and 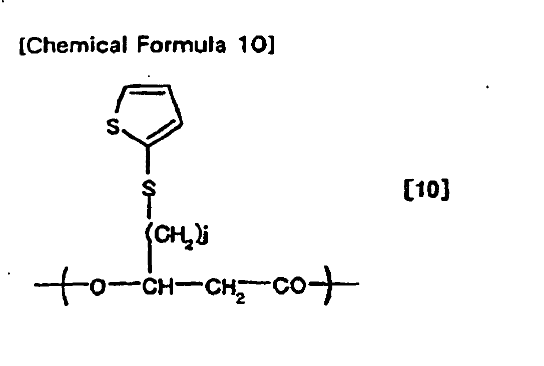 wherein j represents any of integers from 1 to 9.

2. The toner particle according to claim 1, wherein at least part of said polyhydroxyalkanoate is chemically modified.

3. The toner particle according to claim 2, wherein said chemical modification is a graft chain.

4. The toner particle according to claim 3, wherein said graft chain is introduced by chemically modifying a monomer unit having an epoxy group of the polyhydroxyalkanoate.

5. The toner particle according to claim 3, wherein said graft chain is made with a compound having an amino group.

6. The toner particle according to claim 5, wherein said compound having an amino group is a terminal amino-modified compound.

7. The toner particle according to claim 6, wherein said terminal amino-modified compound is selected from the group consisting of polyvinylamine, polyethyleneimine, and terminal amino-modified polysiloxane.

8. The toner particle according to claim 2, wherein at least a part of said polyhydroxyalkanoate is crosslinked.

9. The toner particle according to claim 8, wherein said polyhydroxyalkanoate is crosslinked between monomer units having an epoxy group.

10. The toner particle according to claim 1, wherein said base material contains a colorant.

11. The toner particle according to claim 10, wherein said colorant contains a pigment.

12. The toner particle according to claim 10, wherein said colorant contains a dye.

13. The toner particle according to claim 1, wherein said base material is a pigment.

14. The toner particle according to claim 1, wherein said polyhydroxyalkanoate is made with a composition of monomer units varying in an outward direction of said particle.

15. The toner particle according to claim 1, wherein the particle size of the base material is in the range of 0.1 µm to 1.0 mm.

16. The toner particle according to any one of claims 1 to 15, wherein a molecular weight of said polyhydroxyalkanoate is 1,000 to 10,000,000.

17. The toner particle according to claim 16, wherein a molecular weight of said polyhydroxyalkanoate is 3,000 to 1,000,000.

18. The toner particle according to any one of claims 1 to 17, wherein a polyhydroxyalkanoate synthetase is immobilized to said base material.

19. A method for making an encapsulated particle which comprises a core particle as base material coated with a shell of polyhydroxyalkanoate having a 3-hydroxyalkanoic acid unit, comprising the steps of:
immobilizing a medium chain length, i.e. 3 to 12 carbon atoms, polyhydroxyalkanoate synthetase to the base material, and
reacting 3-hydroxyacyl coenzymes A with the synthetase to synthesize a polyhydroxyalkanoate and to coat said base material with a shell of the polyhydroxyalkanoate,
wherein said polyhydroxyalkanoate comprises at least a monomer unit selected from the group consisting of monomer units represented by chemical formulas [1] to [10], and said monomer units are derived from corresponding 3-hydroxyacyl coenzyme as represented by chemical formulas [11] to [20] in this order: 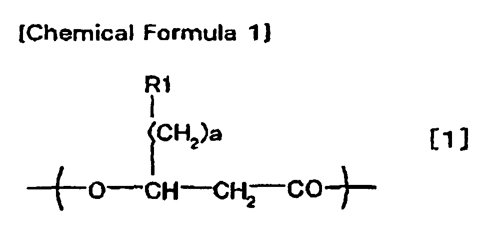
wherein R1 and a are selected from the group of combinations consisting of:
R1 is hydrogen atom (H) and a is any of integers from 3 to 10;
R1 is a halogen atom and a is any of integers from 1 to 10;
R1 is a chromophore and a is any of integers from 1 to 10;
R1 is a carboxyl group or a salt thereof and a is any of integers from 1 to 10; and
R1 is 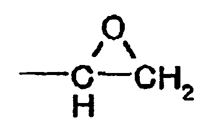
and a is any of integers from 1 to 7; 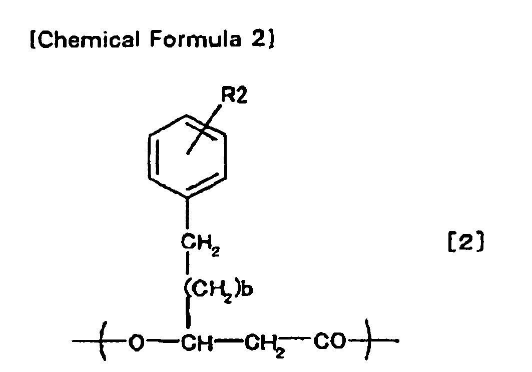
wherein b represents any of integers from 0 to 7 and R2 is selected from the group consisting of hydrogen atom (H), halogen atoms, -CN, -NO₂, -CF₃, -C₂F₅, and -C₃F₇; 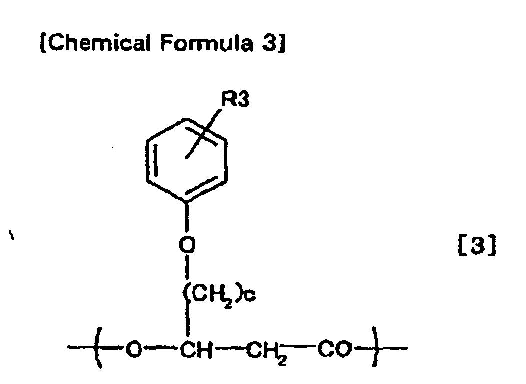 wherein c represents any of integers from 1 to 8 and R3 is selected from the group consisting of hydrogen atom (H), halogen atoms, -CN, -NO₂, -CF₃, -C₂F₅, and -C₃F₇; 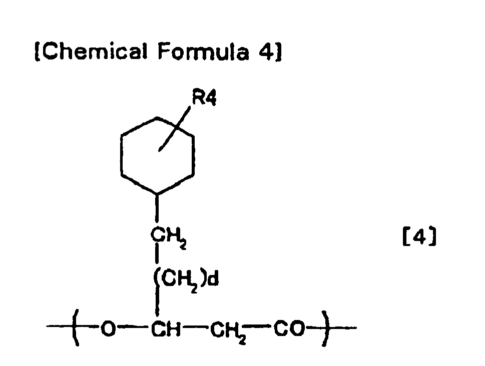 wherein d represents any of integers from 0 to 7 and R4 is selected from the group consisting of hydrogen atom (H), halogen atoms, -CN, -NO₂, -CF₃, -C₂F₅, and -C₃F₇; 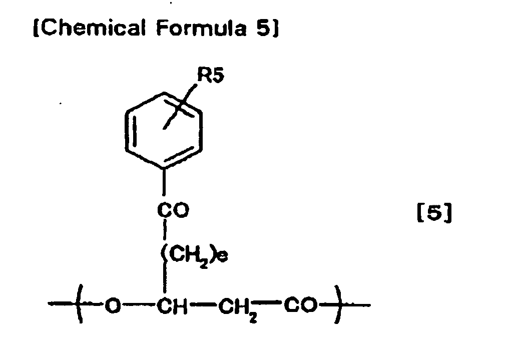 wherein e represents any of integers from 1 to 8 and R6 is selected from the group consisting of hydrogen atom (H), halogen atoms, -CN, -NO₂, -CF₃, -C₂F₅, -C₃F₇, -CH₃, -C₂H₅, and -C₃H₇); 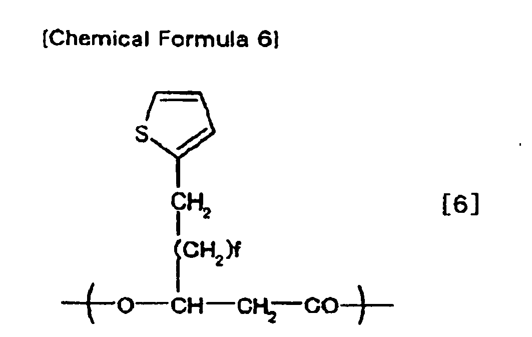 wherein f represents any of integers from 0 to 7; 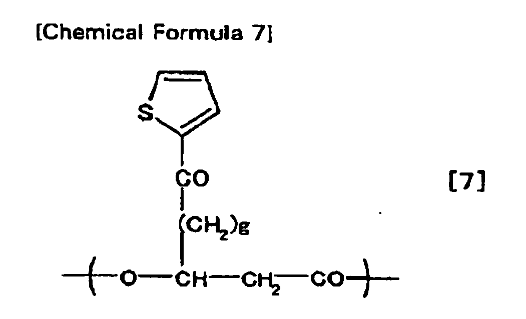 wherein g represents any of integers from 1 to 8; 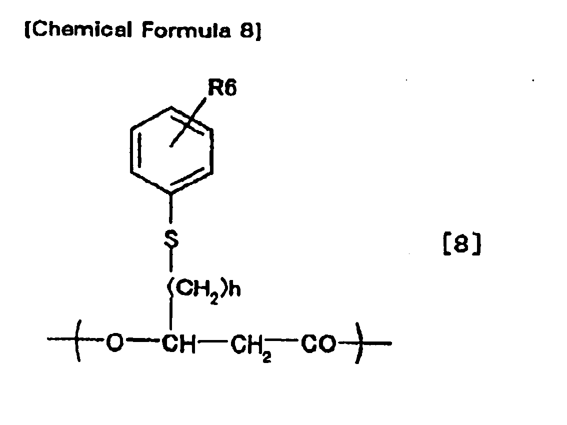 wherein h represents any of integers from 1 to 7 and R6 is selected from the group consisting of hydrogen atom (H), halogen atoms, -CN, -NO₂, -COOR', -SO₂R", -CH₃, -C₂H₅, -C₃H₇, -CH(CH₃)₂, and -C(CH₃)₃, wherein R' is selected from the group consisting of hydrogen atom (H), Na, K, -CH₃, and -C₂H₅, and R" is selected from the group consisting of -OH, -ONa, -OK, halogen atoms, -OCH₃, and -OC₂H₆; 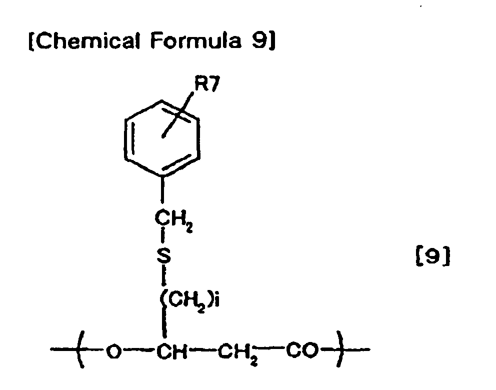 wherein i represents any of integers from 1 to 7 and R7 is selected from the group consisting of hydrogen atom (H), halogen atoms, -CN, -NO₂, -COOR', -SO₂R", wherein R' is selected from the group consisting of hydrogen atom (H), Na, K, -CH₃, and -C₂H₅, and R" is selected from the group consisting of -OH, -ONa, -OK, halogen atoms, -OCH₃, and -OC₂H₆; and 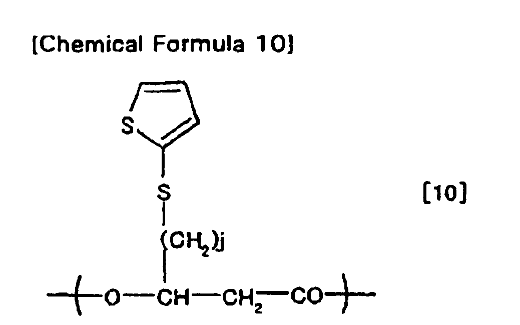 wherein j represents any of integers from 1 to 9, 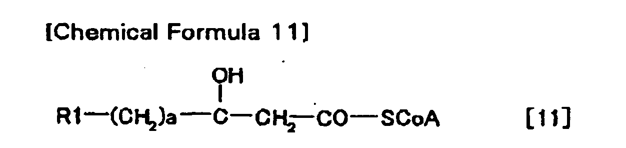 wherein -SCoA represents coenzyme A bound to an alkanoic acid, and R1 and a are the same as Chemical formula [1]; 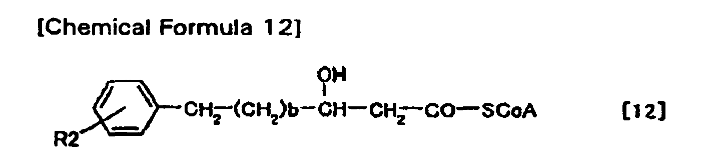 wherein -SCoA represents coenzyme A bound to alkanoic acid, R2 and b are the same as Chemical formula [2]; 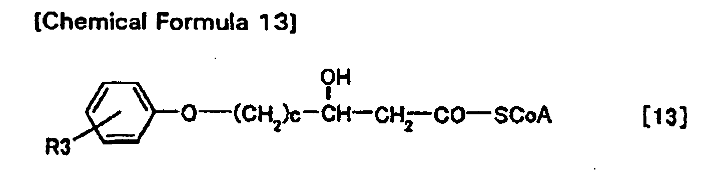 wherein -SCoA represents coenzyme A bound to an alkanoic acid, R3 and c are as defined with chemical formula [3]; 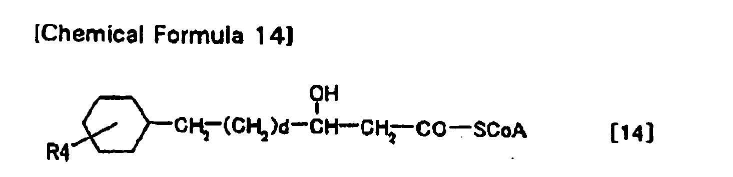 wherein -SCoA represents coenzyme A bound to an alkanoic acid, and R4 and d are the same as with the monomer unit represented by Chemical formula [4]; 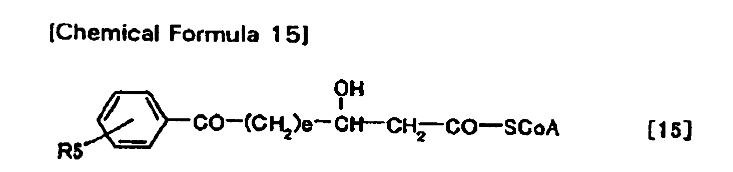 wherein -SCoA represents coenzyme A bound to alkanoic acid, and R5 and e are the same as with the monomer unit represented by Chemical formula [5]; 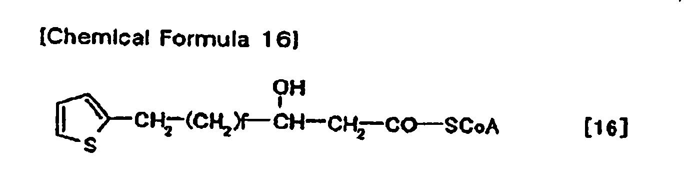 wherein -SCoA represents coenzyme A bound to an alkanoic acid, and f is the same as with the monomer unit represented by Chemical formula [6]; 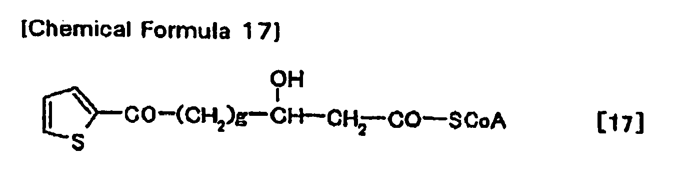 wherein -SCoA represents coenzyme A bound to an alkanoic acid, g is the same as with the monomer unit represented by Chemical formula [7]); 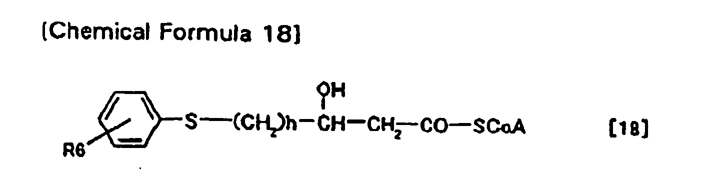 wherein -SCoA represents coenzyme A bound to an alkanoic acid, and R6 and h are the same as with the monomer unit represented by Chemical formula [8]; 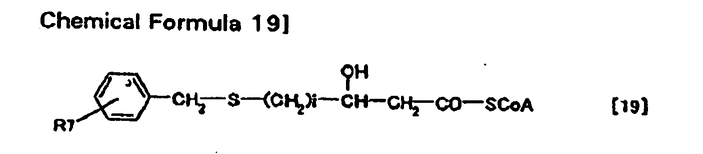 wherein -SCoA represents coenzyme A bound to an alkanoic acid, and R7 and i are the same as with the monomer unit represented by Chemical formula [9]; and 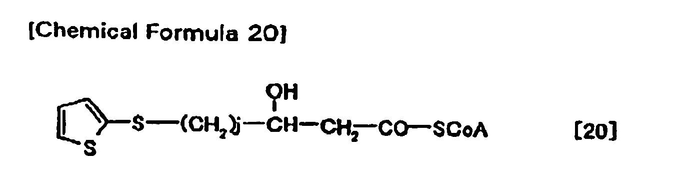 wherein -SCoA represents coenzyme A bound to an alkanoic acid, and j is the same as with the monomer unit represented by Chemical formula [10].

20. The method according to claim 19, further comprising a step of chemically modifying at least a part of said polyhydroxyalkanoate coating the base material.

21. The method according to claim 20, wherein said step of chemically modifying is a step of adding a graft chain to at least a part of said polyhydroxyalkanoate.

22. The method according to claim 21, wherein said step of adding the graft chain is a step of reacting at least a part of said polyhydroxyalkanoate with a compound having a reactive functional group at a terminus thereof.

23. The method according to claim 20, wherein said step of chemically modifying is a step of crosslinking at least a part of said hydroxyalkanoate.

24. The method according to claim 23, wherein said crosslinking step is a step of reacting at least a part of said polyhydroxyalkanoate with a crosslinking agent.

25. The method according to claim 24, wherein said crosslinking agent is selected from the group consisting of a diamine compound, succinic anhydride, and 2-methyl-4-methylimidazole.

26. The method according to claim 25, wherein said diamine compound is hexamethylenediamine.

27. The method according to claim 23, wherein said crosslinking step is a step of irradiating said polyhydroxyalkanoate with an electron beam.

28. The method according to claim 19, further comprising a step of changing the 3-hydroxyacyl coenzymes A in composition with time to change said polyhydroxyalkanoate in composition of monomer units along an inside-to-outside direction of said shell.

29. The method according to claim 19, wherein said polyhydroxyalkanoate synthetase is produced by using a microorganism having a production capability of the synthetase.

30. The method according to claim 19, wherein said polyhydroxyalkanoate synthetase is produced by a transformant into which a gene participating in the production capability of the synthetase has been introduced.

31. The method according to claim 30, wherein said gene is obtained from a microorganism having a production capability of a polyhydroxyalkanoate synthetase.

32. The method according to claim 29, wherein the microorganism is *Pseudomonas* sp.

33. The method according to claim 32, wherein the microorganism of *Pseudomonas* sp. is selected from the group consisting of *Pseudomonas putida* P91, FERM BP-7373, *Pseudomonas cichorii* H45, FERM BP-7374. *Pseudomonas cichorii* YN2, FERM BP-7375, and *Pseudomonas jessenii* P161, FERM BP-7376.

34. The method according to claim 29, wherein the microorganism is *Burkholderia* sp.

35. The method according to claim 34, wherein said microorganism of *Burkholderia* sp. is selected from the group consisting of *Burkholderia* sp. OK3, FERM P-17370 and *Burkholderia* sp. OK4, FERM P-17371.

36. The method according to claim 30, wherein the transformant having a production capability of said polyhydroxyalkanoate synthetase is *Escherichia coli.*

37. An electrophotographic toner, comprising encapsulated toner particles according to any one of claims 1 to 18.

38. A method for making an encapsulated toner particle, comprising the steps of:
preparing a particulate base material; and
using the method according to any one of claims 19 to 36 to coat said base material with said shell of the polyhydroxyalkanoate.

## Patentansprüche

1. Verkapseltes Tonerpartikel für die Elektrophotographie,
umfassend ein Kernpartikel als Grundmaterial, das mit einer Hülle aus Polyhydroxyalkanoat überzogen ist,
wobei das Polyhydroxyalkanoat eine 3-Hydroxyalkansäure-Einheit umfasst, die von einer 3-Hydroxypropionsäure-Einheit, 3-Hydroxy-n-buttersäure-Einheit und 3-Hydroxy-n-valeriansäure-Einheit verschieden ist, und
wobei das Polyhydroxyalkanoat ein solches ist, welches zumindest eine Monomereinheit, die aus der Gruppe von Monomereinheiten mit den chemischen Formeln [1] bis [10] ausgewählt ist, umfasst: 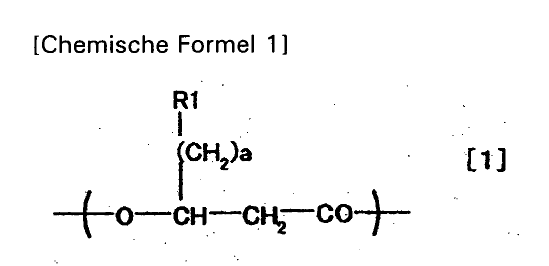 worin R1 und a ausgewählt sind aus der Gruppe folgender Kombinationen, bestehend aus:
R1 ist ein Wasserstoffatom (H) und a ist jede ganze Zahl von 3 bis 10;
R1 ist ein Halogenatom und a ist jede ganze Zahl von 1 bis 10;
R1 ist ein Chromophor und a ist jede ganze Zahl von 1 bis 10;
R1 ist eine Carboxylgruppe oder ein Salz davon und a ist jede ganze Zahl von 1 bis 10; und
R1 ist 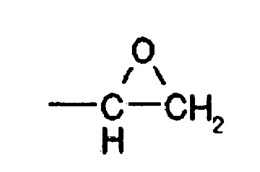
und a ist jede ganze Zahl von 1 bis 7; 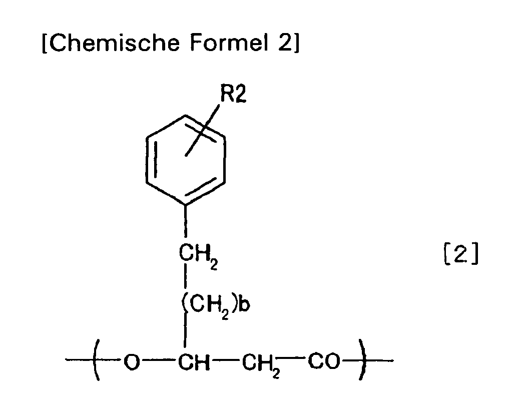 worin b für jede ganze Zahl von 0 bis 7 steht und R2 ausgewählt ist aus der Gruppe, bestehend aus Wasserstoffatom (H), Halogenatomen, -CN, -NO₂, -CF₃, -C₂F₅ und -C₃F₇; 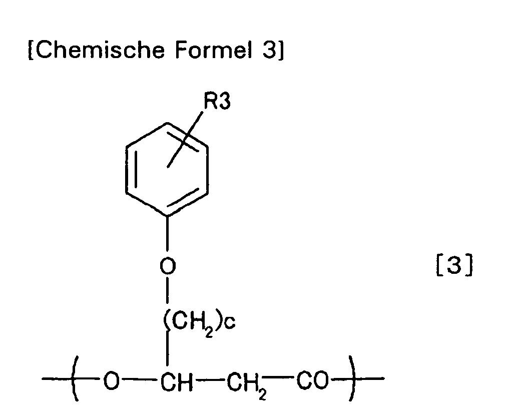 worin c für jede ganze Zahl von 1 bis 8 steht und R3 ausgewählt ist aus der Gruppe, bestehend aus Wasserstoffatom (H), Halogenatomen, -CN, -NO₂, -CF₃, -C₂F₅ und -C₃F₇; 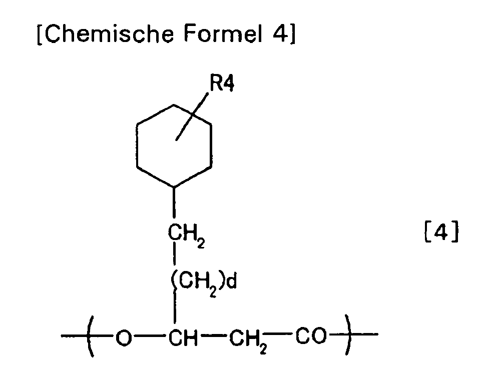 worin d für jede ganze Zahl von 0 bis 7 steht und R4 ausgewählt ist aus der Gruppe, bestehend aus Wasserstoffatom (H), Halogenatomen, -CN, -NO₂, -CF₃, -C₂F₅ und -C₃F₇; 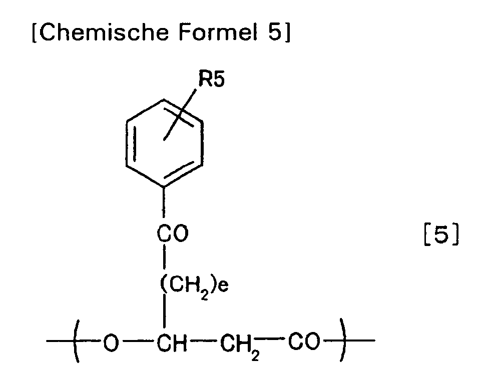 worin e für jede ganze Zahl von 1 bis 8 steht und R5 ausgewählt ist aus der Gruppe, bestehend aus Wasserstoffatom (H), Halogenatomen, -CN, -NO₂, -CF₃, -C₂F₅, -C₃F₇, -CH₃, -C₂H₅ und -C₃H₇; 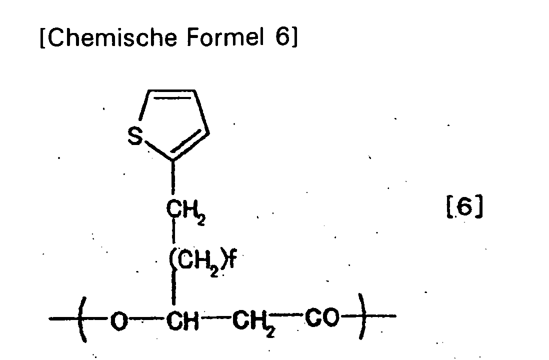 worin f für jede ganze Zahl von 0 bis 7 steht; 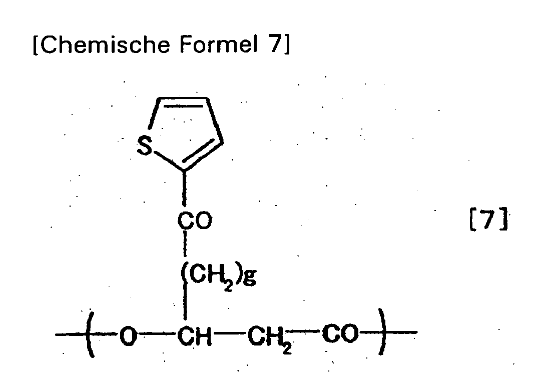 worin g für jede ganze Zahl von 1 bis 8 steht; 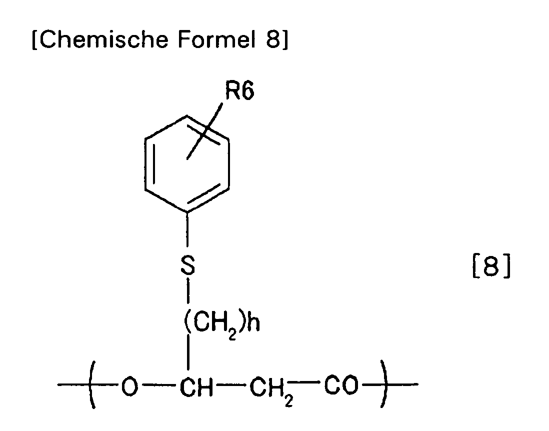 worin h für jede ganze Zahl von 1 bis 7 steht und R6 ausgewählt ist aus der Gruppe, bestehend aus Wasserstoffatom (H), Halogenatomen, -CN, -NO₂, -COOR', -SO₂R", -CH₃, -C₂H₅, -C₃H₇, -CH(CH₃)₂ und -C(CH₃)₃, wobei R' ausgewählt ist aus der Gruppe, bestehend aus Wasserstoffatom (H), Na, K, -CH₃ und -C₂H₅, und R" ausgewählt ist aus der Gruppe, bestehend aus -OH, -ONa, -OK, Halogenatomen, -OCH₃ und -OC₂H₅; 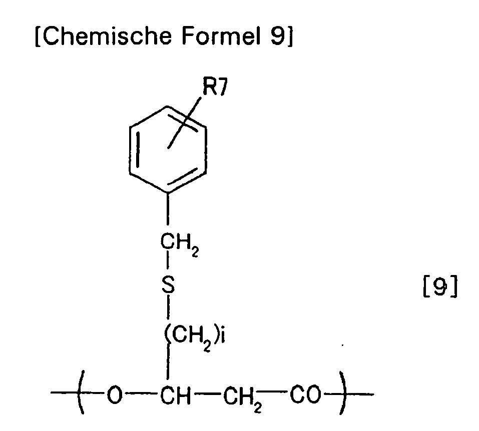 worin i für jede ganze Zahl von 1 bis 7 steht und R7 ausgewählt ist aus der Gruppe, bestehend aus Wasserstoffatom (H), Halogenatomen, -CN, -NO₂, -COOR', -SO₂R", wobei R' ausgewählt ist aus der Gruppe, bestehend aus Wasserstoffatom (H), Na, K, -CH₃ und -C₂H₅, und R" ausgewählt ist aus der Gruppe, bestehend aus -OH, -ONa, -OK, Halogenatomen, -OCH₃ und -OC₂H₅; und 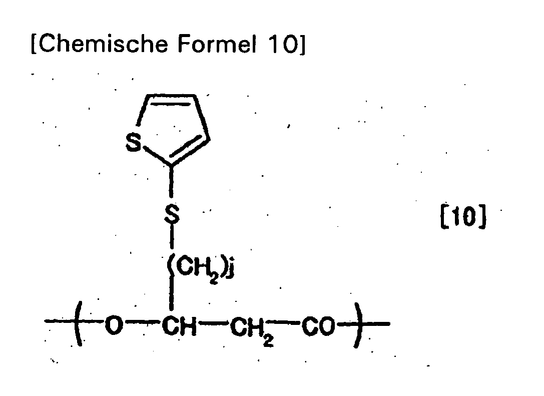 worin j für jede ganze Zahl von 1 bis 9 steht.

2. Tonerpartikel nach Anspruch 1, wobei
zumindest ein Teil des Polyhydroxyalkanoats chemisch modifiziert ist.

3. Tonerpartikel nach Anspruch 2, wobei
die chemische Modifizierung eine Pfropfkette ist.

4. Tonerpartikel nach Anspruch 3, wobei
die Pfropfkette durch chemisches Modifizieren einer Monomereinheit mit einer Epoxygruppe des Polyhydroxyalkanoats eingeführt wird.

5. Tonerpartikel nach Anspruch 3, wobei
die Pfropfkette mit einer Verbindung mit einer Aminogruppe erzeugt wird.

6. Tonerpartikel nach Anspruch 5, wobei
die Verbindung mit einer Aminogruppe eine mit einer endständigen Aminogruppe modifizierte Verbindung ist.

7. Tonerpartikel nach Anspruch 6, wobei
die mit einer endständigen Aminogruppe modifizierte Verbindung ausgewählt ist aus der Gruppe, bestehend aus Polyvinylamin, Polyethylenimin und mit einer endständigen Aminogruppe modifiziertem Polysiloxan.

8. Tonerpartikel nach Anspruch 2, wobei
zumindest ein Teil des Polyhydroxyalkanoats vernetzt ist.

9. Tonerpartikel nach Anspruch 8, wobei
das Polyhydroxyalkanoat vernetzt ist zwischen Monomereinheiten, die eine Epoxygruppe aufweisen.

10. Tonerpartikel nach Anspruch 1, wobei
das Grundmaterial ein Färbemittel enthält.

11. Tonerpartikel nach Anspruch 10, wobei
das Färbemittel ein Pigment enthält.

12. Tonerpartikel nach Anspruch 10, wobei
das Färbemittel einen Farbstoff enthält.

13. Tonerpartikel nach Anspruch 1, wobei
das Grundmaterial ein Pigment ist.

14. Tonerpartikel nach Anspruch 1, wobei
das Polyhydroxyalkanoat hergestellt ist mit einer Zusammensetzung von Monomereinheiten, die sich in einer Auswärtsrichtung des Partikels ändert.

15. Tonerpartikel nach Anspruch 1, wobei
die Grundmaterial-Partikelgröße im Bereich von 0,1 µm bis 1,0 mm liegt.

16. Tonerpartikel nach einem der Ansprüche 1 bis 15, wobei
das Molekulargewicht des Polyhydroxyalkanoats 1.000 bis 10.000.000 beträgt.

17. Tonerpartikel nach Anspruch 16, wobei
das Molekulargewicht des Polyhydroxyalkanoats 3.000 bis 1.000.000 beträgt.

18. Tonerpartikel nach einem der Ansprüche 1 bis 17, wobei
eine Polyhydroxyalkanoat-Synthetase am Grundmaterial immobilisiert ist.

19. Verfahren zum Herstellen eines verkapselten Partikels, umfassend ein Kernpartikel als Grundmaterial, das überzogen ist mit einer, eine 3-Hydroxyalkansäure-Einheit aufweisenden Hülle aus Polyhydroxyalkanoat, umfassend die Schritte:
Immobilisieren einer Polyhydroxyalkanoat-Synthetase mit mittlerer Kettenlänge, d.h. 3 bis 12 Kohlenstoffatome, am Grundmaterial und
Umsetzen von 3-Hydroxyacyl-Coenzymen A mit der Synthetase, so dass ein Polyhydroxyalkanoat synthetisiert wird und das Grundmaterial mit einer Polyhydroxyalkanoat-Hülle überzogen wird,
wobei das Polyhydroxyalkanoat zumindest eine, aus der Gruppe von Monomereinheiten ausgewählte Monomereinheit umfasst, die mit den chemischen Formeln [1] bis [10] angegeben sind, und wobei die Monomereinheiten erhalten werden vom entsprechenden 3-Hydroxyacyl-Coenzym, das in dieser Reihenfolge mit den chemischen Formeln [11] bis [20] angegeben wird: 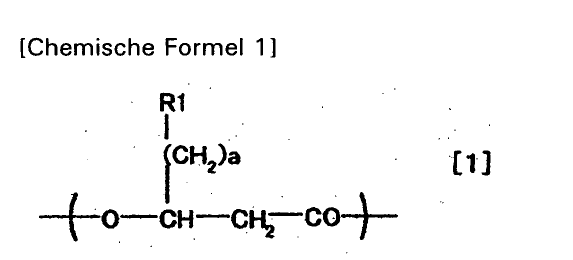 worin R1 und a aus den folgenden Kombinationen ausgewählt sind:
R1 ist ein Wasserstoffatom (H) und a ist jede ganze Zahl von 3 bis 10;
R1 ist ein Halogenatom und a ist jede ganze Zahl von 1 bis 10;
R1 ist ein Chromophor und a ist jede ganze Zahl von 1 bis 10;
R1 ist eine Carboxylgruppe oder ein Salz davon und a ist jede ganze Zahl von 1 bis 10; und
R1 ist 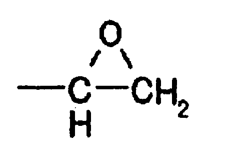
und a ist jede ganze Zahl von 1 bis 7; 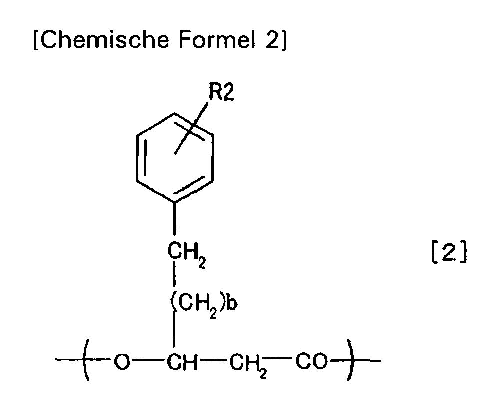 worin b für jede ganze Zahl von 0 bis 7 steht und R2 ausgewählt ist aus der Gruppe, bestehend aus Wasserstoffatom (H), Halogenatomen, -CN, -NO₂, -CF₃, -C₂F₅ und -C₃F₇; 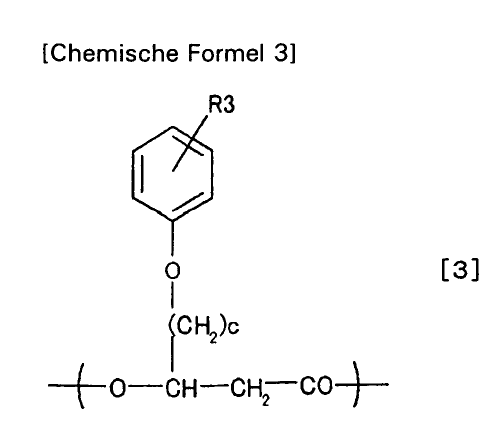 worin c für jede ganze Zahl von 1 bis 8 steht und R3 ausgewählt ist aus der Gruppe, bestehend aus Wasserstoffatom (H), Halogenatomen, -CN, -NO₂, -CF₃, -C₂F₅ und -C₃F₇; 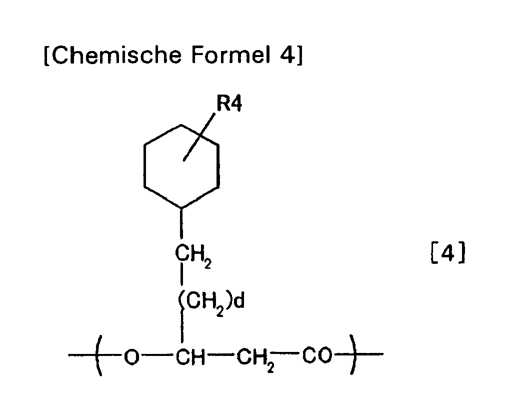 worin d für jede ganze Zahl von 0 bis 7 steht und R4 ausgewählt ist aus der Gruppe, bestehend aus Wasserstoffatom (H), Halogenatomen, -CN, -NO₂, -CF₃, -C₂F₅ und -C₃F₇; 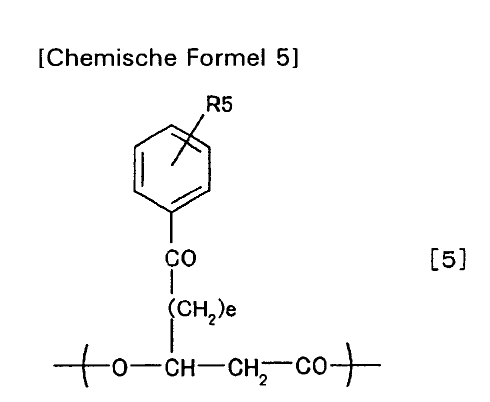
worin e für jede ganze Zahl von 1 bis 8 steht und R5 ausgewählt ist aus der Gruppe, bestehend aus Wasserstoffatom (H), Halogenatomen, -CN, -NO₂, -CF₃, -C₂F₅, -C₃F₇, -CH₃, -C₂H₅ und -C₃H₇; 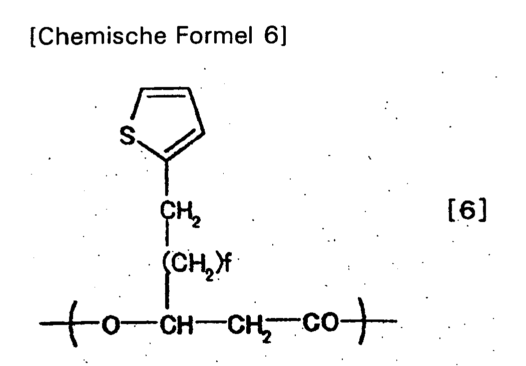 worin f für jede ganze Zahl von 0 bis 7 steht; 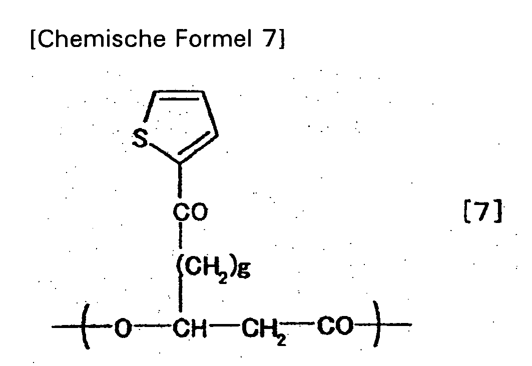 worin g für jede ganze Zahl von 1 bis 8 steht; 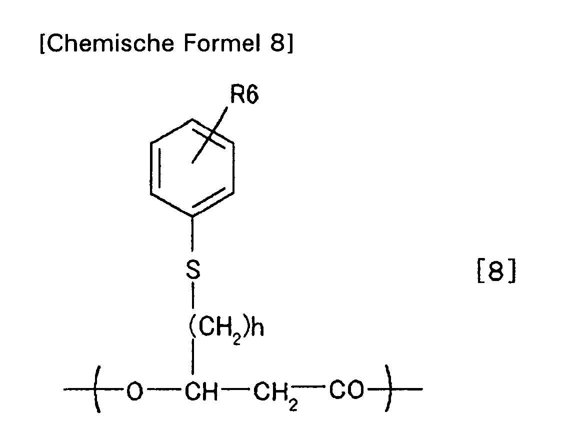 worin h für jede ganze Zahl von 1 bis 7 steht und R6 ausgewählt ist aus der Gruppe, bestehend aus Wasserstoffatom (H), Halogenatomen, -CN, -NO₂, -COOR', -SO₂R", -CH₃, -C₂H₅, -C₃H₇, -CH(CH₃)₂ und -C(CH₃)₃, wobei R' ausgewählt ist aus der Gruppe, bestehend aus Wasserstoffatom (H), Na, K, -CH₃ und -C₂H₅, und R" ausgewählt ist aus der Gruppe, bestehend aus -OH, -ONa, -OK, Halogenatomen, -OCH₃ und -OC₂H₅; 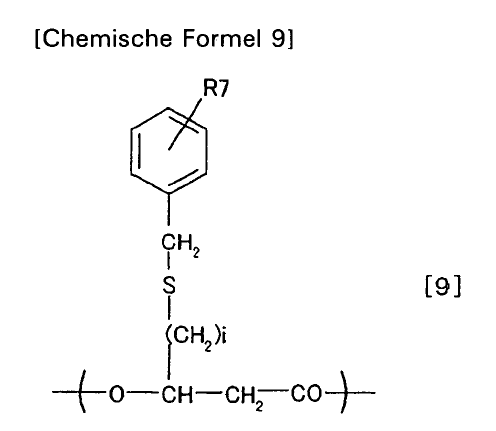 worin i für jede ganze Zahl von 1 bis 7 steht und R7 ausgewählt ist aus der Gruppe, bestehend aus Wasserstoffatom (H), Halogenatomen, -CN, -NO₂, -COOR', -SO₂R", wobei R' ausgewählt ist aus der Gruppe, bestehend aus Wasserstoffatom (H), Na, K, -CH₃ und -C₂H₅, und R" ausgewählt ist aus der Gruppe, bestehend aus -OH, -ONa, -OK, Halogenatomen, -OCH₃ und -OC₂H₅; und 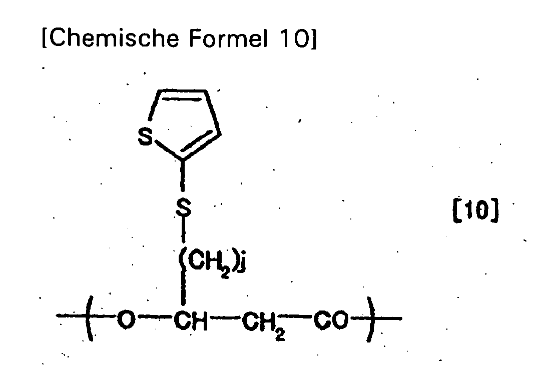 worin j für jede ganze Zahl von 1 bis 9 steht, 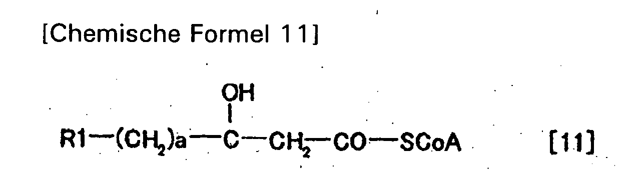 worin -SCoA für Coenzym A steht, das an einer Alkansäure gebunden ist, und R1 und a wie in der chemischen Formel [1] sind; 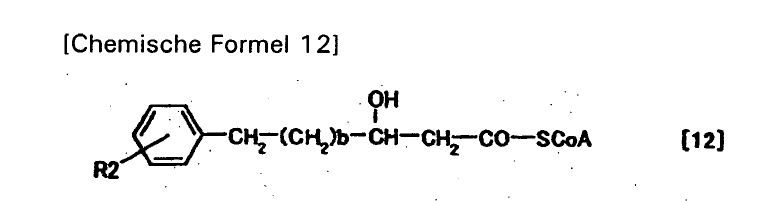 worin -SCoA für Coenzym A steht, das an Alkansäure gebunden ist, R2 und b wie in der chemischen Formel [2] sind; 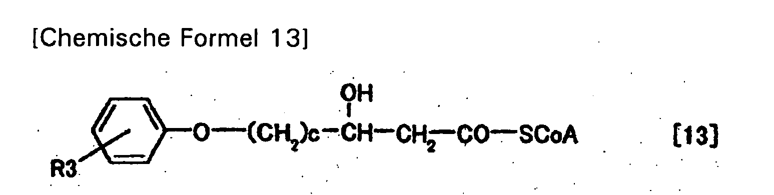 worin -SCoA für Coenzym A steht, das an Alkansäure gebunden ist, R3 und c wie in der chemischen Formel [3] sind; 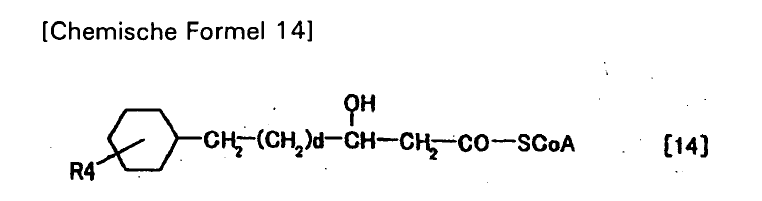 wobei -SCoA für Coenzym A steht, das an einer Alkansäure gebunden ist, und R4 und d wie bei der Monomereinheit der chemischen Formel [4] sind; 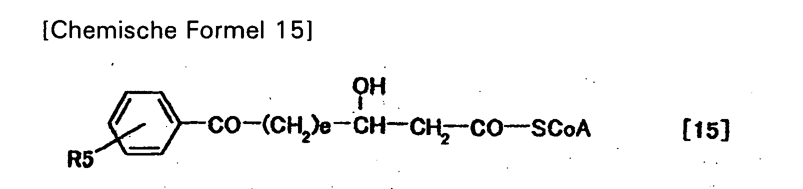 wobei -SCoA für Coenzym A steht, das an Alkansäure gebunden ist, und R5 und e wie bei der Monomereinheit der chemischen Formel [5] sind; 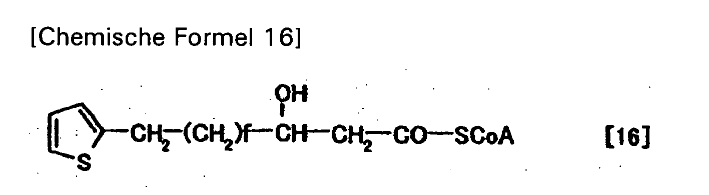 wobei -SCoA für Coenzym A steht, das an einer Alkansäure gebunden ist, und f wie bei der Monomereinheit der chemischen Formel [6] ist; 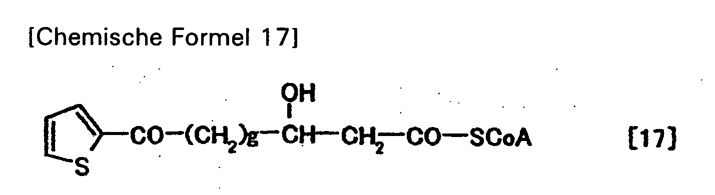 wobei -SCoA für Coenzym A steht, das an einer Alkansäure gebunden ist, und g wie bei der Monomereinheit der chemischen Formel [7] ist; 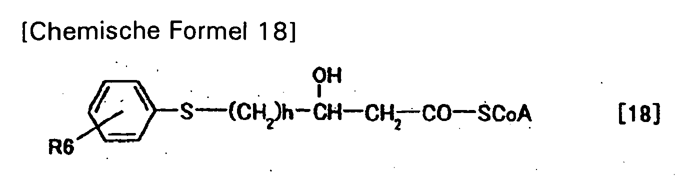 wobei -SCoA für Coenzym A steht, das an einer Alkansäure gebunden ist, und R6 und h wie bei der Monomereinheit der chemischen Formel [8] sind; 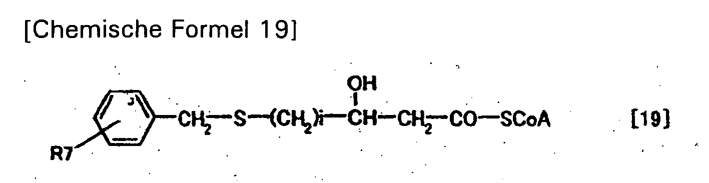 wobei -SCoA für Coenzym A steht, das an einer Alkansäure gebunden ist, und R7 und i wie bei der Monomereinheit der chemischen Formel [9] sind; und 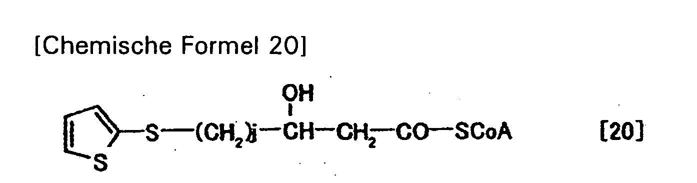 wobei -SCoA für Coenzym A steht, das an einer Alkansäure gebunden ist, und j wie bei der Monomereinheit der chemischen Formel [10] ist.

20. Verfahren nach Anspruch 19, ferner umfassend:
einen Schritt zum chemischen Modifizieren von zumindest einem Teil des das Grundmaterial überziehenden Polyhydroxyalkanoats.

21. Verfahren nach Anspruch 20, wobei
der Schritt zum chemischen Modifizieren ein solcher ist, bei dem an zumindest einem Teil des Polyhydroxyalkanoats eine Pfropfkette angefügt wird.

22. Verfahren nach Anspruch 21, wobei
der Schritt zum Anfügen der Pfropfkette ein solcher ist, bei dem zumindest ein Teil des Polyhydroxyalkanoats umgesetzt wird mit einer Verbindung, die an ihrem Ende eine reaktive funktionelle Gruppe aufweist.

23. Verfahren nach Anspruch 20, wobei
der Schritt zum chemischen Modifizieren ein solcher ist, bei dem zumindest ein Teil des Polyhydroxyalkanoats vernetzt wird.

24. Verfahren nach Anspruch 23, wobei
der Vernetzungsschritt ein solcher ist, bei dem zumindest ein Teil des Polyhydroxyalkanoats mit einem Vernetzungsmittel umgesetzt wird.

25. Verfahren nach Anspruch 24, wobei
das Vernetzungsmittel ausgewählt ist aus der Gruppe, bestehend aus einer Diaminverbindung, Succinsäuereanhydrid und 2-Methyl-4-methylimidazol.

26. Verfahren nach Anspruch 25, wobei
die Diaminverbindung Hexamethylendiamin ist.

27. Verfahren nach Anspruch 23, wobei
der Vernetzungsschritt ein solcher ist, bei dem das Polyhydroxyalkanoat mit einem Elektronenstrahlungsbündel bestrahlt wird.

28. Verfahren nach Anspruch 19, ferner umfassend:
einen Schritt, bei dem die Zusammensetzung der 3-Hydroxyacyl-Coenzyme A im Verlauf der Zeit geändert wird, so dass die Zusammensetzung der Monomereinheiten des Polyhydroxyalkanoats entlang einer Richtung der Hülle von innen nach außen verändert wird.

29. Verfahren nach Anspruch 19, wobei
die Polyhydroxyalkanoat-Synthetase produziert wird unter Verwendung eines Mikroorganismus, der die Fähigkeit zur Produktion der Synthetase hat.

30. Verfahren nach Anspruch 19, wobei
die Polyhydroxyalkanoat-Synthetase produziert wird von einer Transformante, in die ein Gen eingeführt worden ist, welches an der Fähigkeit zur Produktion der Synthetase beteiligt ist.

31. Verfahren nach Anspruch 30, wobei
das Gen erhalten wird von einem Mikroorganismus, der die Fähigkeit zur Produktion einer Polyhydroxyalkanoat-Synthetase aufweist.

32. Verfahren nach Anspruch 29, wobei
der Mikroorganismus Pseudomonas sp. ist.

33. Verfahren nach Anspruch 32, wobei
der Mikroorganismus Pseudomonas sp. ausgewählt ist aus der Gruppe, bestehend aus Pseudomonas putida P91, FERM BP-7373, Pseudomonas cichorii H45, FERM BP-7374, Pseudomonas cichorii YN2, FERM BP-7375 und Pseudomonas jessenii P161, FERM BP-7376.

34. Verfahren nach Anspruch 29, wobei
der Mikroorganismus Burkholderia sp. ist.

35. Verfahren nach Anspruch 34, wobei
der Mikroorganismus Burkholderia sp. ausgewählt ist aus der Gruppe, bestehend aus Burkholderia sp. OK3, FERM P-17370 und Burkholderia sp. OK4, FERM P-17371.

36. Verfahren nach Anspruch 30, wobei
die Transformante mit der Fähigkeit zur Produktion der Polyhydroxyalkanoat-Synthetase vorliegt als Escherichia coli.

37. Toner für die Elektrophotographie, umfassend:
verkapselte Tonerpartikel nach einem der Ansprüche 1 bis 18.

38. Verfahren zum Herstellen eines verkapselten Tonerpartikels, umfassend die Schritte:
Herstellen eines partikelförmigen Grundmaterials; und
Anwendung des Verfahrens nach einem der Ansprüche 19 bis 36, derart dass das Grundmaterial mit der Polyhydroxyalkanoat-Hülle überzogen wird.

## Revendications

1. Particule de toner électrophotographique encapsulé, comprenant une particule centrale comme matière de base revêtue d'une enveloppe d'un polyhydroxyalcanoate,
dans laquelle le polyhydroxyalcanoate comprend un motif acide 3-hydroxyalcanoïque autre qu'un motif acide 3-hydroxy-propionique, un motif acide 3-hydroxy-n-butyrique et un motif acide 3-hydroxy-n-valérique, et
dans laquelle ledit polyhydroxyalcanoate est un polyhydroxyalcanoate comprenant au moins un motif choisi dans le groupe consistant en les motifs monomères représentés par les formules chimiques [1] à [10]: 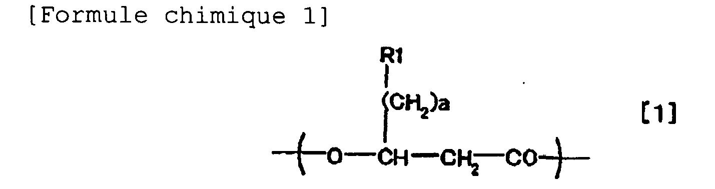 dans laquelle R1 et a sont choisis dans le groupe de combinaisons consistant en:
R1 représente un atome d'hydrogène (H) et a représente n'importe lequel des nombres entiers de 3 à 10 ;
R1 représente un atome d'halogène et a représente n'importe lequel des nombres entiers de 1 à 10 ;
R1 représente un chromophore et a représente n'importe lequel des nombres entiers de 1 à 10 ;
R1 représente un groupe carboxyle ou un de ses sels et a représente n'importe lequel des nombres entiers de 1 à 10; et
R1 représente un groupe 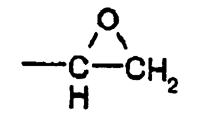
et a représente n'importe lequel des nombres entiers de 1 à 7 ; 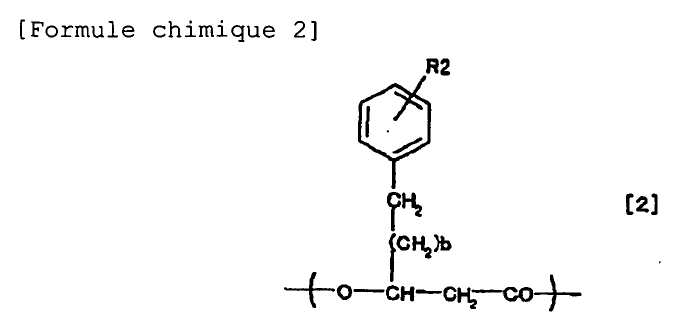
dans laquelle b représente n'importe lequel des nombres entiers de 0 à 7 et R2 est choisi dans le groupe consistant en un atome d'hydrogène (H), des atomes d'halogène, des groupes -CN, -NO₂, -CF₃, -C₂F₅ et -C₃F₇; 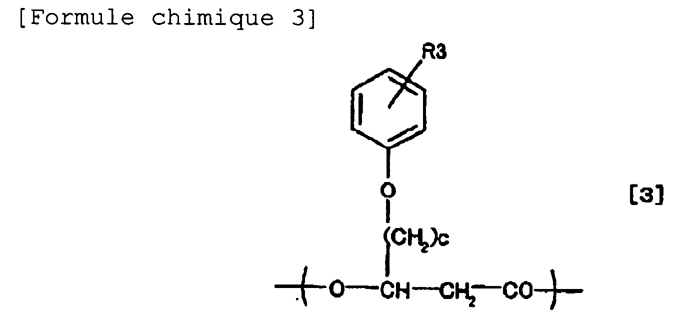 dans laquelle c représente n'importe lequel des nombres entiers de 1 à 8 et R3 est choisi dans le groupe consistant en un atome d'hydrogène (H), des atomes d'halogène, des groupes -CN, -NO₂, -CF₃, -C₂F₅ et -C₃F₇; 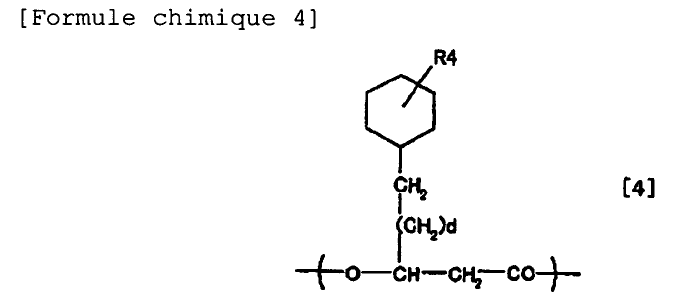 dans laquelle d représente n'importe lequel des nombres entiers de 0 à 7 et R4 est choisi dans le groupe consistant en un atome d'hydrogène (H), des atomes d'halogène, des groupes -CN, -NO₂, -CF₃, -C₂F₅ et -C₃F₇; 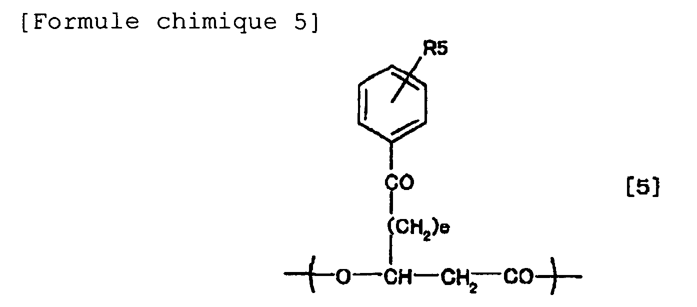 dans laquelle e représente n'importe lequel des nombres entiers de 1 à 8 et R5 est choisi dans le groupe consistant en un atome d'hydrogène (H), des atomes d'halogène, des groupes -CN, -NO₂, -CF₃, -C₂F₅, -C₃F₇, -CH₃, -C₂H₅ et -C₃H₇; 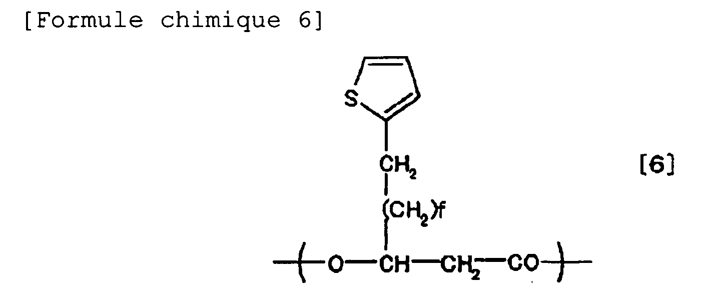 dans laquelle f représente n'importe lequel des nombres entiers de 0 à 7 ; 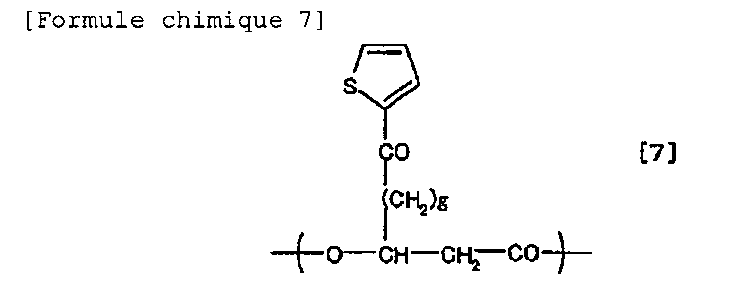 dans laquelle g représente n'importe lequel des nombres entiers de 1 à 8 ; 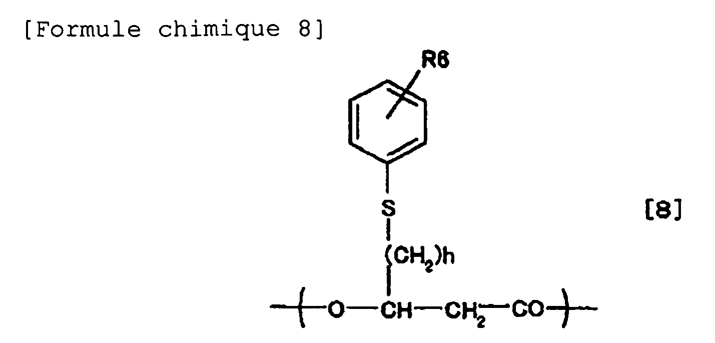 dans laquelle h représente n'importe lequel des nombres entiers de 1 à 7 et R6 est choisi dans le groupe consistant en un atome d'hydrogène (H), des atomes d'halogène, des groupes -CN, -NO₂, -COOR', -SO₂R", -CH₃, -C₂H₅, -C₃H₇, -CH(CH₃)₂ et -C(CH₃)₃, dans lesquels R' est choisi dans le groupe consistant en un atome d'hydrogène (H), Na, K, des groupes -CH₃, et -C₂H₅, et R" est choisi dans le groupe consistant en des groupes -OH, -ONa, -OK, des atomes d'halogène, des groupes -OCH₃ et-OC₂H₅; 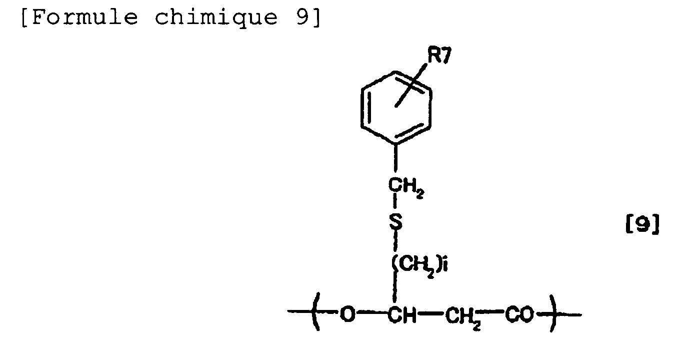 dans laquelle i représente n'importe lequel des nombres entiers de 0 à 7 et R7 est choisi dans le groupe consistant en un atome d'hydrogène (H), des atomes d'halogène, des groupes -CN, -NO₂, -COOR' et -SO₂R", dans lesquels R' est choisi dans le groupe consistant en un atome d'hydrogène (H), Na, K, des groupes -CH₃, et -C₂H₅, et R" est choisi dans le groupe consistant en des groupes -OH, -ONa, -OK, des atomes d'halogène, des groupes -OCH₃ et-OC₂H₅; et 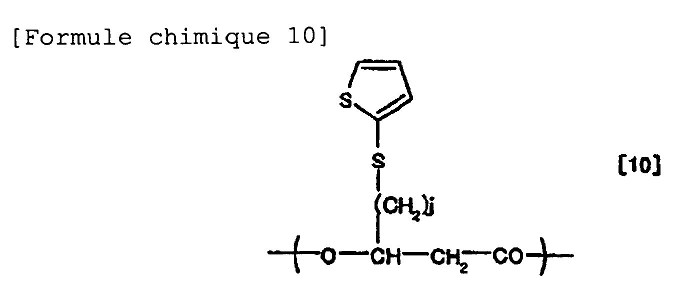 dans laquelle j représente n'importe lequel des nombres entiers de 1 à 9.

2. Particule de toner suivant la revendication 1, dans laquelle au moins une partie dudit polyhydroxyalcanoate est modifiée chimiquement.

3. Particule de toner suivant la revendication 2, dans laquelle ladite modification chimique est une chaîne greffée.

4. Particule de toner suivant la revendication 3, dans laquelle ladite chaîne greffée est introduite par modification chimique d'un motif monomère ayant un groupe époxy du polyhydroxyalcanoate.

5. Particule de toner suivant la revendication 3, dans laquelle ladite chaîne greffée est produite avec un composé ayant un groupe amino.

6. Particule de toner suivant la revendication 5, dans laquelle ledit composé ayant un groupe amino est un composé à modification amino terminale.

7. Particule de toner suivant la revendication 6, dans laquelle ledit composé à modification amino terminale est choisi dans le groupe consistant en la polyvinylamine, la polyéthylèneimine et un polysiloxane à modification amino terminale.

8. Particule de toner suivant la revendication 2, dans laquelle au moins une partie dudit polyhydroxyalcanoate est réticulée.

9. Particule de toner suivant la revendication 8, dans laquelle ledit polyhydroxyalcanoate est réticulé entre des motifs monomères ayant un groupe époxy.

10. Particule de toner suivant la revendication 1, dans laquelle ladite matière de base contient une matière colorante.

11. Particule de toner suivant la revendication 10, dans laquelle ladite matière colorante contient un pigment.

12. Particule de toner suivant la revendication 10, dans laquelle ladite matière colorante contient un colorant.

13. Particule de toner suivant la revendication 1, dans laquelle ladite matière de base est un pigment.

14. Particule de toner suivant la revendication 1, dans laquelle ledit polyhydroxyalcanoate est produit avec une composition de motifs monomères variant vers l'extérieur de ladite particule.

15. Particule de toner suivant la revendication 1, dans laquelle le diamètre de particule de la matière de base est compris dans l'intervalle de 0,1 µm à 1,0 mm.

16. Particule de toner suivant l'une quelconque des revendications 1 à 15, dans laquelle le poids moléculaire dudit polyhydroxyalcanoate est de 1000 à 10 000 000.

17. Particule de toner suivant la revendication 16, dans laquelle le poids moléculaire dudit polyhydroxyalcanoate est de 3000 à 1 000 000.

18. Particule de toner suivant l'une quelconque des revendications 1 à 17, dans laquelle une polyhydroxyalcanoate-synthétase est immobilisée au niveau de ladite matière de base.

19. Procédé pour la préparation d'une particule encapsulée qui comprend une particule centrale comme matière de base revêtue d'une enveloppe d'un polyhydroxyalcanoate ayant un motif acide 3-hydroxyalcanoïque, comprenant les étapes:
d'immobilisation d'une polyhydroxyalcanoate-synthétase à longueur de chaîne moyenne, c'est-à-dire de 3 à 12 atomes, au niveau de la matière de base, et
de réaction de 3-hydroxyacyl-coenzyme-A avec la synthétase pour synthétiser un polyhydroxyalcanoate et pour revêtir ladite matière de base avec une enveloppe du polyhydroxyalcanoate,
dans lequel ledit polyhydroxyalcanoate comprend au moins un motif monomère choisi dans le groupe consistant en en les motifs monomères représentés par les formules chimiques [1] à [10], et lesdits motifs monomères sont dérivés du 3-hydroxyacyl-enzyme correspondant, comme représenté par les formules chimiques [11] à [20] dans cet ordre: 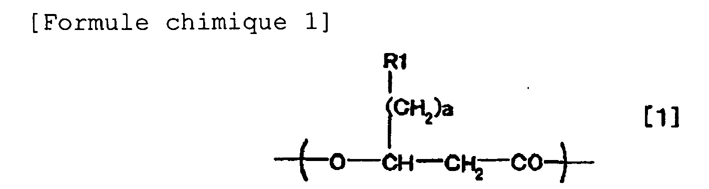 dans laquelle R1 et a sont choisis dans le groupe de combinaisons consistant en:
R1 représente un atome d'hydrogène (H) et a représente n'importe lequel des nombres entiers de 3 à 10 ;
R1 représente un atome d'halogène et a représente n'importe lequel des nombres entiers de 1 à 10 ;
R1 représente un chromophore et a représente n'importe lequel des nombres entiers de 1 à 10 ;
R1 représente un groupe carboxyle ou un de ses sels et a représente n'importe lequel des nombres entiers de 1 à 10; et
R1 représente un groupe 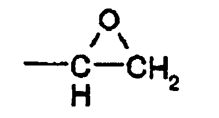
et a représente n'importe lequel des nombres entiers de 1 à 7 ; 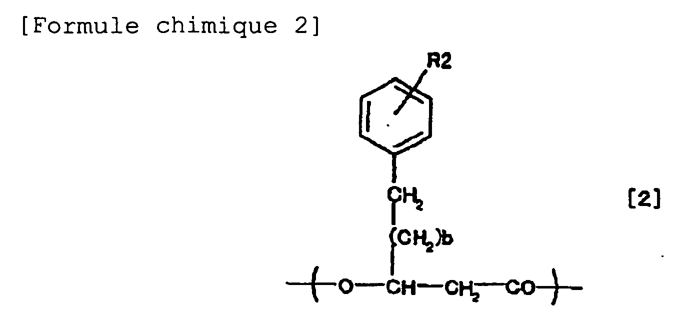
dans laquelle b représente n'importe lequel des nombres entiers de 0 à 7 et R2 est choisi dans le groupe consistant en un atome d'hydrogène (H), des atomes d'halogène, des groupes -CN, -NO₂, -CF₃, -C₂F₅ et -C₃F₇; 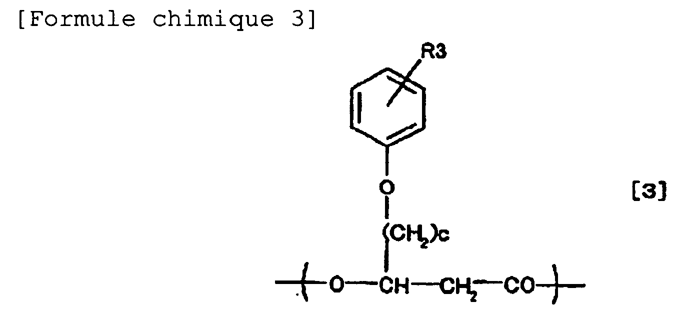 dans laquelle c représente n'importe lequel des nombres entiers de 1 à 8 et R3 est choisi dans le groupe consistant en un atome d'hydrogène (H), des atomes d'halogène, des groupes -CN, -NO₂, -CF₃, -C₂F₅ et -C₃F₇; 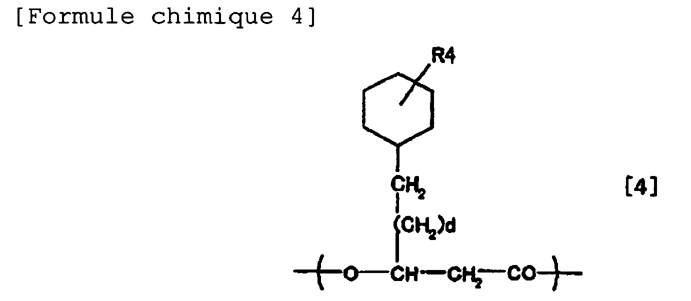 dans laquelle d représente n'importe lequel des nombres entiers de 0 à 7 et R4 est choisi dans le groupe consistant en un atome d'hydrogène (H), des atomes d'halogène, des groupes -CN, -NO₂, -CF₃, -C₂F₅ et -C₃F₇; 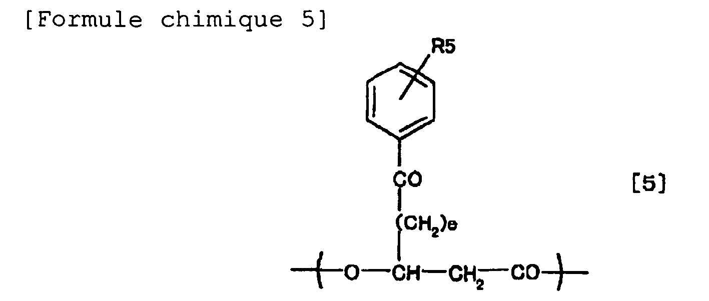 dans laquelle e représente n'importe lequel des nombres entiers de 1 à 8 et R5 est choisi dans le groupe consistant en un atome d'hydrogène (H), des atomes d'halogène, des groupes -CN, -NO₂, -CF₃, -C₂F₅, -C₃F₇, -CH₃, -C₂H₅ et -C₃H₇; 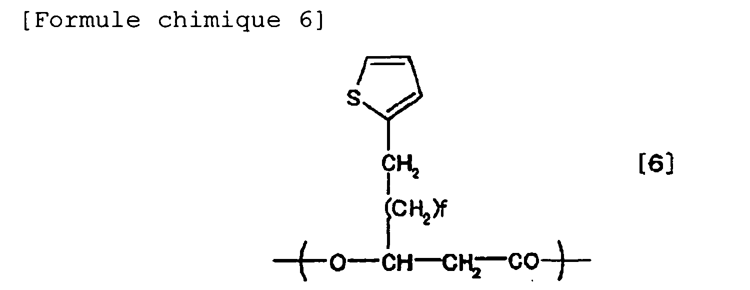 dans laquelle f représente n'importe lequel des nombres entiers de 0 à 7 ; 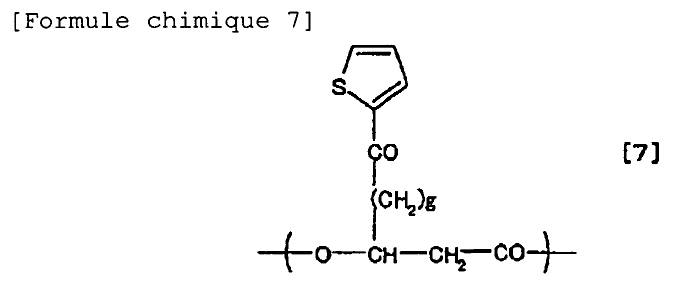 dans laquelle g représente n'importe lequel des nombres entiers de 1 à 8 ; 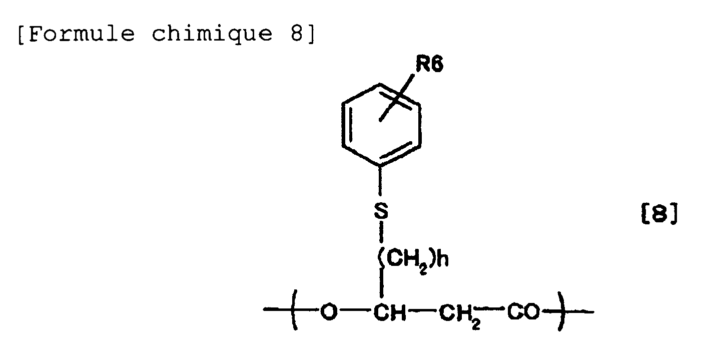 dans laquelle h représente n'importe lequel des nombres entiers de 1 à 7 et R6 est choisi dans le groupe consistant en un atome d'hydrogène (H), des atomes d'halogène, des groupes -CN, -NO₂, -COOR', -SO₂R", -CH₃, -C₂H₅, -C₃H₇, -CH(CH₃)₂ et -C(CH₃)₃, dans lesquels R' est choisi dans le groupe consistant en un atome d'hydrogène (H), Na, K, des groupes -CH₃, et -C₂H₅, et R" est choisi dans le groupe consistant en des groupes -OH, -ONa, -OK, des atomes d'halogène, des groupes -OCH₃ et -OC₂H₆; 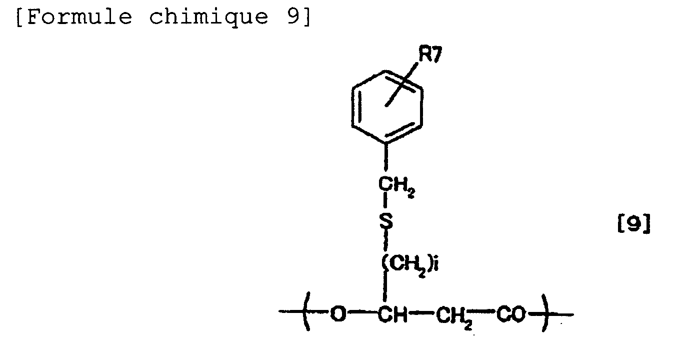 dans laquelle i représente n'importe lequel des nombres entiers de 0 à 7 et R7 est choisi dans le groupe consistant en un atome d'hydrogène (H), des atomes d'halogène, des groupes -CN, -NO₂, -COOR' et -SO₂R", dans lesquels R' est choisi dans le groupe consistant en un atome d'hydrogène (H), Na, K, des groupes -CH₃, et -C₂H₅, et R" est choisi dans le groupe consistant en des groupes -OH, -ONa, -OK, des atomes d'halogène, des groupes -OCH₃ et -OC₂H₅; et 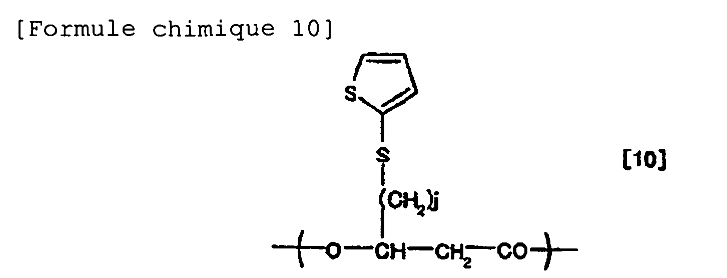 dans laquelle j représente n'importe lequel des nombres entiers de 1 à 9, 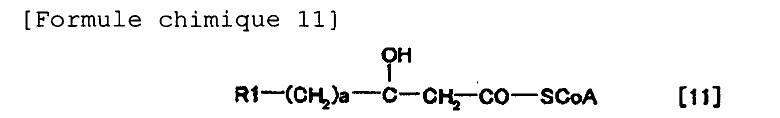 dans laquelle -SCoA représente le coenzyme A lié à un acide alcanoïque, et R1 et a sont identiques à ceux indiqués pour la formule chimique [1] ; 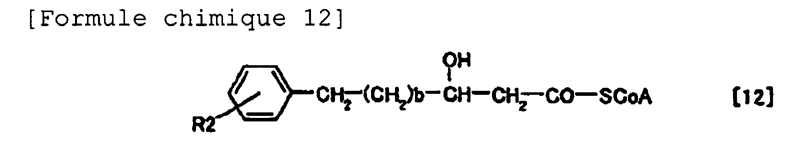 dans laquelle -SCoA représente le coenzyme A lié à un acide alcanoïque, et R2 et b sont identiques à ceux indiqués pour la formule chimique [2] ; 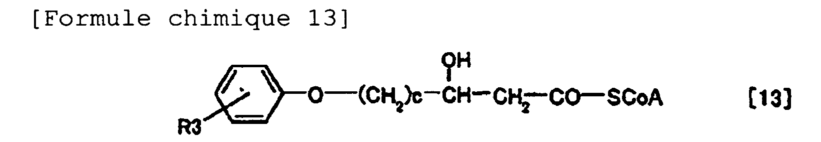 dans laquelle -SCoA représente le coenzyme A lié à un acide alcanoïque, et R3 et c sont tels que définis pour la formule chimique [3] ; 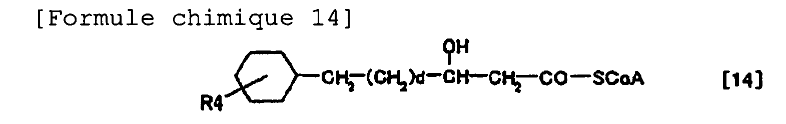 dans laquelle -SCoA représente le coenzyme A lié à un acide alcanoïque, et R4 et d sont identiques à ceux indiqués pour le motif monomère représenté par la formule chimique [4] ; 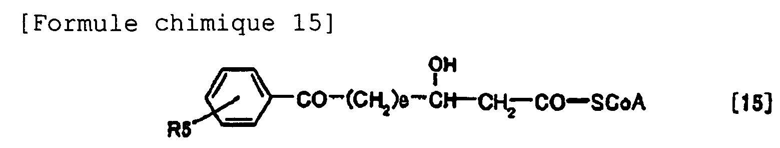 dans laquelle -SCoA représente le coenzyme A lié à un acide alcanoïque, et R5 et e sont identiques à ceux indiqués pour le motif monomère représenté par la formule chimique [5] ; 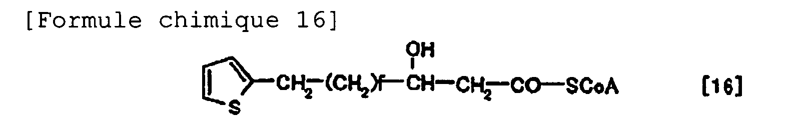 dans laquelle -SCoA représente le coenzyme A lié à un acide alcanoïque, et f est identique à celui indiqué pour le motif monomère représenté par la formule chimique [6] ; 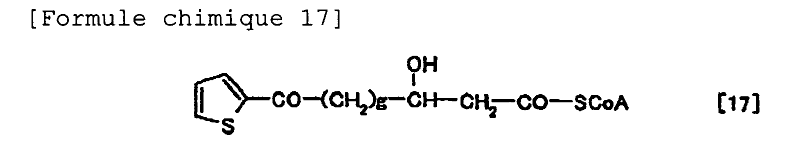 dans laquelle -SCoA représente le coenzyme A lié à un acide alcanoïque, g est identique à celui indiqué pour le motif monomère représenté par la formule chimique [7] ; 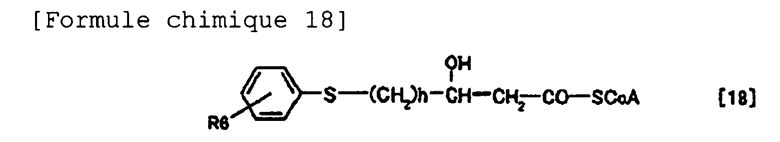 dans laquelle -SCoA représente le coenzyme A lié à un acide alcanoïque, et R6 et h sont identiques à ceux indiqués pour le motif monomère représenté par la formule chimique [8] ; 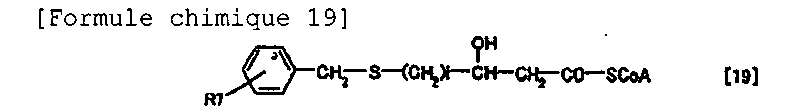 dans laquelle -SCoA représente le coenzyme A lié à un acide alcanoïque, et R7 et i sont identiques à ceux indiqués pour le motif monomère représenté par la formule chimique [9] ; et 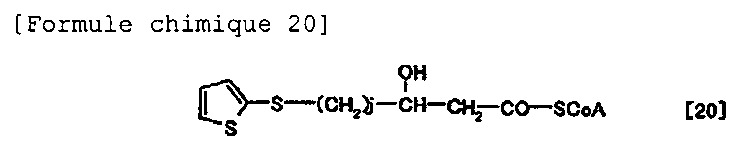 dans laquelle -SCoA représente le coenzyme A lié à un acide alcanoïque, et j est identique à celui indiqué pour le motif monomère représenté par la formule chimique [10].

20. Procédé suivant la revendication 19, comprenant en outre une étape de modification chimique d'au moins une partie dudit polyhydroxyalcanoate revêtant la matière de base.

21. Procédé suivant la revendication 20, dans lequel ladite étape de modification chimique est une étape d'addition d'une chaîne greffée à au moins une partie dudit polyhydroxyalcanoate.

22. Procédé suivant la revendication 21, dans lequel ladite étape d'addition de la chaîne greffée est une étape de réaction d'au moins une partie dudit polyhydroxyalcanoate avec un composé ayant un groupe fonctionnel réactif à une de ses extrémités.

23. Procédé suivant la revendication 20, dans lequel ladite étape de modification chimique est une étape de réticulation d'au moins une partie dudit hydroxyalcanoate.

24. Procédé suivant la revendication 23, dans lequel ladite étape de réticulation est une étape de réaction d'au moins une partie dudit polyhydroxyalcanoate avec un agent de réticulation.

25. Procédé suivant la revendication 24, dans lequel ledit agent de réticulation est choisi dans le groupe consistant en une diamine, l'anhydride succinique et le 2-méthyl-4-méthylimidazole.

26. Procédé suivant la revendication 25, dans lequel ladite diamine est l'hexaméthylènediamine.

27. Procédé suivant la revendication 23, dans lequel ladite étape de réticulation est une étape d'irradiation dudit polyhydroxyalcanoate avec un faisceau d'électrons.

28. Procédé suivant la revendication 19, comprenant en outre une étape de modification des 3-hydroxyacyl-coenzyme-A en ce qui concerne la composition en fonction du temps pour modifier ledit polyhydroxyalcanoate en ce qui concerne la composition en motifs monomères de l'intérieur à l'extérieur de ladite enveloppe.

29. Procédé suivant la revendication 19, dans lequel ladite polyhydroxyalcanoate-synthétase est produite en utilisant un microorganisme ayant une capacité de production de la synthétase.

30. Procédé suivant la revendication 19, dans lequel ladite polyhydroxyalcanoate-synthétase est produite par un transformant dans lequel un gène participant à la capacité de production de la synthétase a été introduit.

31. Procédé suivant la revendication 30, dans lequel ledit gène est obtenu à partir d'un microorganisme ayant une capacité de production d'une polyhydroxyalcanoate-synthétase.

32. Procédé suivant la revendication 29, dans lequel le microorganisme est *Pseudomonas* sp.

33. Procédé suivant la revendication 32, dans lequel le microorganisme *Pseudomonas sp* est choisi dans le groupe consistant en *Pseudomonas putida* P91, FERM BP-7373, *Pseudomonas Cichorii* H45, FERM PB-7374, *Pseudomonas cichorii* YN2, FERM BP-7375 et *Pseudomonas jessenii* P161, FERM BP-7376.

34. Procédé suivant la revendication 29, dans lequel le microorganisme est *Burkholderia* sp.

35. Procédé suivant la revendication 34, dans lequel le microorganisme est *Burkholderia* sp est choisi dans le groupe consistant *Burkholderia* sp. OK3, FERM P-170 et *Burkholderia* sp. OK4, FERM P-17371.

36. Procédé suivant la revendication 30, dans lequel le transformant ayant une capacité de production de ladite polyhydroxyalcanoate-synthétase est *Escherichia coli.*

37. Toner électrophotographique, comprenant des particules de toner encapsulé suivant l'une quelconque des revendications 1 à 18.

38. Procédé pour la production d'une particule de toner encapsulé, comprenant les étapes:
de préparation d'une matière de base en particules; et
d'utilisation du procédé suivant l'une quelconque des revendications 19 à 36 pour revêtir ladite matière de base avec ladite enveloppe du polyhydroxyalcanoate.


### SEQUENCE LISTING

<110> CANON KABUSHIKI KAISHA
<120> Construct and Method for Making It
<130> CFO16374
<140>
   <141>
<150> JP P2001-131694
   <151> 2001-04-27
<150> JP P2001-208704
   <151> 2001-07-10
<210> 1
   <211> 1501
   <212> DNA
   <213> Pseudomonas jessenii 161 strain.
<400> 1
   tgaacgctgg cggcaggcct aacacatgca agtcgagcgg atgacgggag cttgctcctg    60
   aattcagcgg cggacgggtg agtaatgcct aggaatctgc ctggtagtgg gggacaacgt   120
   ctcgaaaggg acgctaatac cgcatacgtc ctacgggaga aagcagggga ccttcgggcc   180
   ttgcgctatc agatgagcct aggtcggatt agctagttgg tgaggtaatg gctcaccaag   240
   gcgacgatcc gtaactggtc tgagaggatg atcagtcaca ctggaactga gacacggtcc   300
   agactcctac gggaggcagc agtggggaat attggacaat gggcgaaagc ctgatccagc   360
   catgccgcgt gtgtgaagaa ggtcttcgga ttgtaaagca ctttaagttg ggaggaaggg   420
   cattaaccta atacgttagt gttttgacgt taccgacaga ataagcaccg gctaactctg   480
   tgccagcagc cgcggtaata cagagggtgc aagcgttaat cggaattact gggcgtaaag   540
   cgcgcgtagg tggtttgtta agttggatgt gaaagccccg ggctcaacct gggaactgca   600
   ttcaaaactg acaagctaga gtatggtaga gggtggtgga atttcctgtg tagcggtgaa   660
   atgcgtagat ataggaagga acaccagtgg cgaaggcgac cacctggact gatactgaca   720
   ctgaggtgcg aaagcgtggg gagcaaacag gattagatac cctggtagtc cacgccgtaa   780
   acgatgtcaa ctagccgttg ggagccttga gctcttagtg gcgcagctaa cgcattaagt   840
   tgaccgcctg ggagtacggc cgcaaggtta aaactcaaat gaattgacgg gggcccgcac   900
   aagcggtgga gcatgtggtt taattcgaag caacgcgaag aaccttacca ggccttgaca   960
   tccaatgaac tttccagaga tggatgggtg ccttcgggaa cattgagaca ggtgctgcat  1020
   ggctgtcgtc agctcgtgtc gtgagatgtt gggttaagtc ccgtaacgag cgcaaccctt  1080
   gtccttagtt accagcacgt aatggtgggc actctaagga gactgccggt gacaaaccgg  1140
   aggaaggtgg ggatgacgtc aagtcatcat ggcccttacg gcctgggcta cacacgtgct  1200
   acaatggtcg gtacagaggg ttgccaagcc gcgaggtgga gctaatccca caaaaccgat  1260
   cgtagtccgg atcgcagtct gcaactcgac tgcgtgaagt cggaatcgct agtaatcgcg  1320
   aatcagaatg tcgcggtgaa tacgttcccg ggccttgtac acaccgcccg tcacaccatg  1380
   ggagtgggtt gcaccagaag tagctagtct aaccttcggg aggacggtta ccacggtgtg  1440
   attcatgact ggggtgaagt cgtaccaagg tagccgtagg ggaacctgcg gctggatcac  1500
   c                                                                  1501
<210> 2
   <211> 20
   <212> DNA
   <213> Artificial Sequence
<400> 2
   tgctggaact gatccagtac 20
<210> 3
   <211> 23
   <212> DNA
   <213> Artificial Sequence
<400> 3
   gggttgagga tgctctggat gtg 23
<210> 4
   <211> 1680
   <212> DNA
   <213> Pseudomonas cichorii YN2 ; FERM P-17411
<400> 4
   atgagtaaca agagtaacga tgagttgaag tatcaagcct ctgaaaacac   50
   cttggggctt aatcctgtcg ttgggctgcg tggaaaggat ctactggctt  100
   ctgctcgaat ggtgcttagg caggccatca agcaaccggt gcacagcgtc  150
   aaacatgtcg cgcactttgg tcttgaactc aagaacgtac tgctgggtaa  200
   atccgggctg caaccgacca gcgatgaccg tcgcttcgcc gatccggcct  250
   ggagccagaa cccgctctat aaacgttatt tgcaaaccta cctggcgtgg  300
   cgcaaggaac tccacgactg gatcgatgaa agtaacctcg cccccaagga  350
   tgtggcgcgt gggcacttcg tgatcaacct catgaccgaa gccatggcgc   400
   cgaccaacac cgcggccaac ccggcggcag tcaaacgctt tttcgaaacc   450
   ggtggcaaaa gcctgctcga cggcctctcg cacctggcca aggatctggt   500
   acacaacggc ggcatgccga gccaggtcaa catgggtgca ttcgaggtcg   550
   gcaagagcct gggcgtgacc gaaggcgcgg tggtgtttcg caacgatgtg   600
   ctggaactga tccagtacaa gccgaccacc gagcaggtat acgaacgccc   650
   gctgctggtg gtgccgccgc agatcaacaa gttctacgtt ttcgacctga   700
   gcccggacaa gagcctggcg cggttctgcc tgcgcaacaa cgtgcaaacg   750
   ttcatcgtca gctggcgaaa tcccaccaag gaacagcgag agtggggcct   800
   gtcgacctac atcgaagccc tcaaggaagc ggttgatgtc gttaccgcga   850
   tcaccggcag caaagacgtg aacatgctcg gcgcctgctc cggcggcatc   900
   acttgcaccg cgctgctggg ccattacgcg gcgattggcg aaaacaaggt   950
   caacgccctg accttgctgg tgagcgtgct tgataccacc ctcgacagcg  1000
   atgttgccct gttcgtcaat gaacagaccc ttgaagccgc caagcgccac  1050
   tcgtaccagg ccggcgtact ggaaggccgc gacatggcga aggtcttcgc  1100
   ctggatgcgc cccaacgatc tgatctggaa ctactgggtc aacaattacc  1150
   tgctaggcaa cgaaccgccg gtgttcgaca tcctgttctg gaacaacgac  1200
   accacacggt tgcccgcggc gttccacggc gacctgatcg aactgttcaa  1250
   aaataaccca ctgattcgcc cgaatgcact ggaagtgtgc ggcaccccca  1300
   tcgacctcaa gcaggtgacg gccgacatct tttccctggc cggcaccaac  1350
   gaccacatca ccccgtggaa gtcctgctac aagtcggcgc aactgtttgg  1400
   cggcaacgtt gaattcgtgc tgtcgagcag cgggcatatc cagagcatcc  1450
   tgaacccgcc gggcaatccg aaatcgcgct acatgaccag caccgaagtg  1500
   gcggaaaatg ccgatgaatg gcaagcgaat gccaccaagc ataccgattc  1550
   ctggtggctg cactggcagg cctggcaggc ccaacgctcg ggcgagctga  1600
   aaaagtcccc gacaaaactg ggcagcaagg cgtatccggc aggtgaagcg  1650
   gcgccaggca cgtacgtgca cgaacggtaa                        1680
<210> 5
   <211> 1683
   <212> DNA
   <213> Pseudomonas cichorii YN2 ; FERM P-17411
<400> 5
   atgcgcgata aacctgcgag ggagtcacta cccacccccg ccaagttcat 50
   caacgcacaa agtgcgatta ccggcctgcg tggccgggat ctggtttcga 100
   ctttgcgcag tgtcgccgcc catggcctgc gccaccccgt gcacaccgcg 150
   cgacacgcct tgaaactggg tggtcaactg ggacgcgtgt tgctgggcga 200
   caccctgcat cccaccaacc cgcaagaccg tcgcttcgac gatccggcgt 250
   ggagtctcaa tcccttttat cgtcgcagcc tgcaggcgta cctgagctgg 300
   cagaagcagg tcaagagctg gatcgacgaa agcaacatga gcccggatga 350
   ccgcgcccgt gcgcacttcg cgttcgccct gctcaacgat gccgtgtcgc 400
   cgtccaacag cctgctcaat ccgctggcga tcaaggaaat cttcaactcc 450
   ggcggcaaca gcctggtgcg cgggatcggc catctggtcg atgacctctt 500
   gcacaacgat ggcttgcccc ggcaagtcac caggcatgca ttcgaggttg 550
   gcaagaccgt cgccaccacc accggcgccg tggtgtttcg caacgagctg 600
   ctggagctga tccaatacaa gccgatgagc gaaaagcagt attccaaacc 650
   gctgctggtg gtgccgccac agatcaacaa gtactacatt tttgacctca 700
   gcccccataa cagcttcgtc cagttcgcgc tcaagaacgg cctgcaaacc 750
   ttcgtcatca gctggcgcaa tccggatgta cgtcaccgcg aatggggcct 800
   gtcgacctac gtcgaagcgg tggaagaagc catgaatgtc tgccgggcaa 850
   tcaccggcgc gcgcgaggtc aacctgatgg gcgcctgcgc tggcgggctg 900
   accattgctg ccctgcaggg ccacttgcaa gccaagcgac agctgcgccg 950
   cgtctccagc gcgacgtacc tggtgagcct gctcgacagc caactggaca 1000
   gcccggccac actcttcgcc gacgaacaga ccctggaggc ggccaagcgc 1050
   cgctcctacc agaaaggtgt gctggaaggc cgcgacatgg ccaaggtttt 1100
   cgcctggatg cgccccaacg atttgatctg gagctacttc gtcaacaatt 1150
   acctgatggg caaggagccg ccggcgttcg acattctcta ctggaacaat 1200
   gacaacacac gcctgccggc cgccctgcat ggtgacttgc tggacttctt 1250
   caagcacaac ccgctgagcc atccgggtgg cctggaagtg tgcggcaccc 1300
   cgatcgactt gcaaaaggtc accgtcgaca gtttcagcgt ggccggcatc 1350
   aacgatcaca tcacgccgtg ggacgcggtg tatcgctcaa ccctgttgct 1400
   cggtggcgag cgtcgctttg tcctggccaa cagcggtcat gtgcagagca 1450
   ttctcaaccc gccgaacaat ccgaaagcca actacctcga aggtgcaaaa 1500
   ctaagcagcg accccagggc ctggtactac gacgccaagc ccgtcgacgg 1550
   tagctggtgg acgcaatggc tgggctggat tcaggagcgc tcgggcgcgc 1600
   aaaaagaaac ccacatggcc ctcggcaatc agaattatcc accgatggag 1650
   gcggcgcccg ggacttacgt gcgcgtgcgc tga 1683
<210> 6
   <211> 29
   <212> DNA
   <213> Artificial Sequence
<220>
   <223> Primer for PCR multiplication
<400> 6
   ggaccaagct tctcgtctca gggcaatgg 29
<210> 7
   <211> 29
   <212> DNA
   <213> Artificial Sequence
<220>
   <223> Primer for PCR multiplication
<400> 7
   cgagcaagct tgctcctaca ggtgaaggc 29
<210> 8
   <211> 29
   <212> DNA
   <213> Artificial Sequence
<220>
   <223> Primer for PCR multiplication
<400> 8
   gtattaagct tgaagacgaa ggagtgttg 29
<210> 9
   <211> 30
   <212> DNA
   <213> Artificial Sequence
<220>
   <223> Primer for PCR multiplication
<400> 9
   catccaagct tcttatgatc gggtcatgcc 30
<210> 10
   <211> 30
   <212> DNA
   <213> Artificial Sequence
<220>
   <223> Primer for PCR multiplication
<400> 10
   cgggatccag taacaagagt aacgatgagt 30
<210> 11
   <211> 30
   <212> DNA
   <213> Artificial Sequence
<220>
   <223> Primer for PCR multiplication
<400> 11
   cgatctcgag ttaccgttcg tgcacgtacg 30
<210> 12
   <21i> 30
   <212> DNA
   <213> Artificial Sequence
<220>
   <223> Primer for PCR multiplication
<400> 12
   cgggatcccg cgataaacct gcgagggagt 30
<210> 13
   <211> 30
   <212> DNA
   <213> Artificial Sequence
<220>
   <223> Primer for PCR multiplication
<400> 13
   cgatctcgag gcgcacgcgc acgtaagtcc 30